# EUROPEAN PATENT APPLICATION

(11) **EP 2 107 116 A2**
(43) Date of publication of application: **07.10.2009**
(21) Application number: 09001498.6
(22) Date of filing: 09.06.2003
(51) Int. Cl.: C12N 15/10

(54) **Methods for improving a binding characteristic of a molecule**

(30) Priority: 12.06.2002 US 388386 P
(62) Divisional of application: 03760249.7
(71) Applicant: GENENCOR INTERNATIONAL, INC., Palo Alto, California 94304 (US)
(72) Inventor: Schellenberger, Volker, Palo Alto, CA 94303 (US); Murray, Christopher J., Soquel, CA 95073 (US)
(74) Representative: Kiddle, Simon John

(57) **Abstract**

The present invention relates to methods for improving a binding characteristic of a molecule, e.g., a peptide, for a binding target, in which the molecule is covalently linked to a detachable moiety, e.g., an enzyme, or an active portion or derivative thereof. The present invention also relates to molecules produced by the methods of the present invention.

## Description

### 1. FIELD OF THE INVENTION

The present invention relates to methods for improving a binding characteristic of a molecule, *e.g.*, a peptide, for a binding target, in which the molecule is joined to a detectable moiety, *e.g.*, an enzyme, or the active portion thereof. The present invention also relates to molecules produced by the methods of the present invention.

### 2. BACKGROUND OF THE INVENTION

Molecules that bind a particular target are useful in a number of applications, including diagnostic and therapeutic methods, affinity purification methods, methods of delivery to specific locations, *etc.* Of particular interest are proteins and peptides, including antibodies, that bind particular and specific targets, for example, nucleic acids or proteins. Molecules that have particular binding abilities can be generated in a number of ways. One method is by immunizing an animal with a target molecule and subsequently isolating antibodies, or fragments thereof, that bind the target. Another method is by using phage display or other display methods to isolate binding molecules, including proteins. *See, e.g.,* Chen et al., 2001, Nat. Biotechnol. 19:537-42.

In many cases, the binding properties of the isolated molecules obtained by such methods are not ideal for their ultimate application. Several methods have been described to improve the binding properties, which mostly involve generating variants of the starting sequence and identifying variants with improved binding properties. *See, e.g.,* Yang et al., 1995, J. Mol. Biol. 254:392-403; Schier et al., 1996, J. Mol. Biol. 263:551-67; and Beiboer et al., 2000, J. Mol. Biol. 296:833-49, which are related to phage display-based methods. However, such methods are time consuming and require several rounds of "panning". Further, it is known that such methods can result in the enrichment of binding molecules that show reduced binding affinity for the selected target. Thus, typically, tens of thousands of potential molecules must be screened to isolate those with improved binding ability, and this screening process typically requires the use of helper reagents, such as anti-phage antibodies and antibody-enzyme conjugates, that limit the sensitivity and precision of subsequent screens.

Another frequently used method for screening is the ELISA method. In this method, a binding target is attached to a surface (*e.g*., a well in a microtiter dish). The target attached to the surface is incubated with a candidate binding molecule in a first binding reaction. The first binding reaction is washed to remove unbound candidate molecules. A helper reagent (e.g., an antibody-enzyme conjugate) is then added for a second binding reaction. The helper reagent binds the candidate molecules bound to the target. After a second wash to remove unbound helper reagent, a substrate is added. The substrate is converted into a detectable form by helper reagent bound to candidate binding molecules bound to target.

The ELISA methodology has several drawbacks, principally due to the requirement of a second binding reaction. During the second binding reaction, the helper reagent can interact non-specifically with the target or reaction vessel thus leading to a high background signal, which limits the ability to detect weakly bound molecules.

Other assay formats are also used to measure or detect binding interactions, including radioimmune and biotinylation-based binding assays. Similarly, these assays also require helper reagents and suffer from the same limitations.

Thus, there remains in the art a need for more sensitive and efficient methods for identifying molecules with improved binding characteristics.

Citation of a reference in this or any section of the specification shall not be construed as an admission that such reference is prior art to the present invention.

### 3. SUMMARY OF THE INVENTION

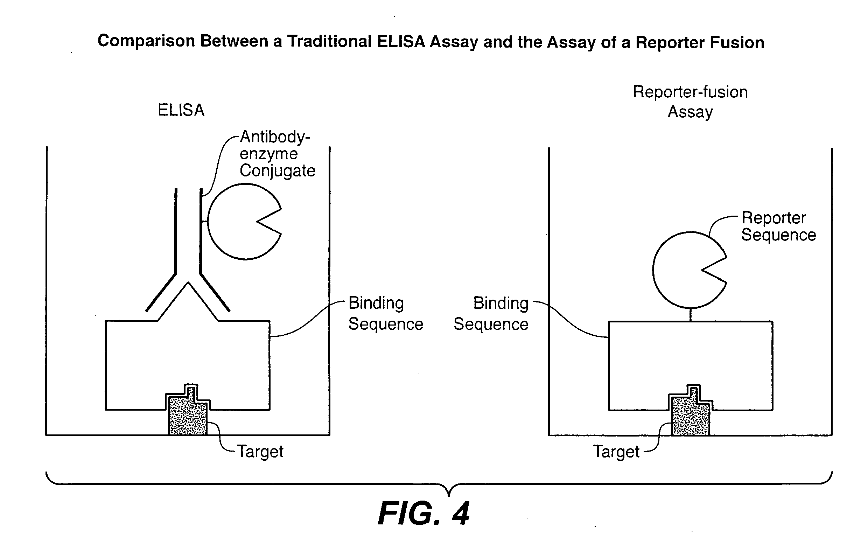

The present invention relates to compositions and methods for improving a binding characteristic of a binding molecule, e.g., a peptide binding sequence, in which the binding molecule is joined to a reporter molecule, e.g., an enzyme, or the active portion or catalytic domain thereof. The reporter molecule, and thus a binding molecule linked to it, can be detected without a second binding reaction, e.g., of the type used in standard ELISA assays, as illustrated in Figure 4.

In one aspect, the present invention provides methods of improving a binding characteristic, e.g., affinity, selectivity, release rate or turnover rate, of a prototype binding molecule for a target, comprising: contacting the target with a reporter fusion under conditions that allow the reporter fusion to bind to the target, wherein the reporter fusion comprises a reporter molecule and a variant binding molecule derived from the prototype binding molecule that binds to the target, and selecting the reporter fusion if it binds the target with an improved binding characteristic relative to that of the prototype binding domain for the target.

In another aspect, the invention provides methods for improving a binding characteristic of a binding molecule for a target, comprising: contacting the target with a library comprising a multiplicity of reporter fusions, under conditions that allow a reporter fusion to bind the target, wherein said reporter fusions comprise a reporter molecule and a variant binding molecule derived from a prototype binding molecule that binds the target, and selecting a reporter fusion that binds the target and has a binding characteristic for the target that is improved relative to the binding characteristic of the prototype binding molecule for the target.

In another aspect, the invention provides methods for improving a binding characteristic of a binding molecule for a target, comprising: contacting a target with a library comprising a multiplicity of reporter fusions, under conditions that allow a reporter fusion to bind the target, wherein said reporter fusions comprise a reporter molecule and a variant binding molecule derived from a prototype binding molecule that binds the target, selecting a reporter fusion bound to the target and having a binding characteristic that is improved relative to the binding characteristic of the prototype binding molecule, and removing the reporter molecule from the selected reporter fusion.

In one embodiment, the selecting step comprises incubating the reporter fusion bound to the target under conditions that cause a reporter fusion with an undesirable binding characteristic to dissociate from the target. In another embodiment, the selecting step comprises incubating the reporter fusion bound to the target with multiple rounds of conditions that cause a reporter fusion with an undesirable binding characteristic to dissociate from the target, wherein each subsequent round of conditions causes dissociation of a reporter fusion with a better binding characteristic for the target than the previous round. In another embodiment, the amount of bound reporter fusion is measured between one or more rounds of dissociation. In another embodiment, the selecting step comprises selecting a reporter fusion if it binds to the target under a first condition better than it binds to the target under a second condition. In another embodiment, the condition is pH, with the first condition being a pH lower than the second condition. In another such embodiment, the first condition is a pH higher than the second condition. In another embodiment, the condition is temperature, with the first condition being a lower temperature than the second condition. In another embodiment, the first condition is a temperature higher than the second condition. In another embodiment, the selecting step comprises incubating the reporter fusion bound to the target in the presence of proteases or under conditions that degrade or destabilize the reporter fusion. In another embodiment, conditions may include, but are not limited to, heat, pH or subjugation to solutes that affect stability.

In another embodiment, the method further comprises repeating the contacting and selecting steps, wherein the variant binding molecule selected in a previous selection step is the prototype binding molecule of a subsequent contacting step.

In another embodiment, the reporter molecule is a reporter sequence. In another embodiment, the reporter sequence is an enzyme or a functional fragment or derivative of an enzyme. In another embodiment, the enzyme is α β-lactamase, β-galactosidase, phosphatase, peroxidase, reductase, esterase, hydrolase, isomerase or protease.

In another embodiment, the reporter fusion is selected if it has a binding affinity that is greater than the binding affinity of the prototype binding molecule. In another embodiment, the reporter fusion is selected if it has a binding affinity that is less than the binding affinity for the target of the prototype binding molecule. In another embodiment, the reporter fusion is selected if it has a binding selectivity that is greater than the binding selectivity of the prototype binding molecule. In another embodiment, the reporter fusion is selected if it has a binding selectivity that is less than the binding selectivity of the prototype binding molecule. In another embodiment, the reporter fusion is selected if it has a release rate that is greater than the release rate of the prototype binding molecule. In another embodiment, the reporter fusion is selected if it has a release rate that is less than the release rate of the prototype binding molecule. In another embodiment, the reporter fusion is selected if it has a turnover rate that is greater than the turnover rate of the prototype binding molecule. In another embodiment, the reporter fusion is selected if it has a turnover rate that is less than the turnover rate of the prototype binding molecule.

In another embodiment, the prototype binding molecule binds the target with a K_{d} of about 100 µM or less, 10 µM or less, 1 µM or less, 100 nM or less, about 90 nM or less, about 80 nM or less, about 70 nM or less, about 60 nM or less, about 50 nM or less, about 40 nM or less, about 30 nM or less, about 20 nM or less, about 10 nM or less, about 5 nM or less, about 1 nM or less or about 0.1 nM or less. In yet another embodiment, the selected variant sequence binds the target with a K_{d} of about 100 µM or less, 10 µM or less, 1 µM or less, 100 nM or less, about 90 nM or less, about 80 nM or less, about 70 nM or less, about 60 nM or less, about 50 nM or less, about 40 nM or less, about 30 nM or less, about 20 nM or less, about 10 nM or less, about 5 nM or less, about 1 nM or less or about 0.1 nM or less. In another embodiment, the selected variant sequence binds the target with a K_{d} of about 100 µM or more, 10 µM or more, 1 µM or more, 100 nM or more, about 90 nM or more, about 80 nM or more, about 70 nM or more, about 60 nM or more, about 50 nM or more, about 40 nM or more, about 30 nM or more, about 20 nM or more, about 10 nM or more, about 5 nM or more, about 1 nM or more or about 0.1 nM or more.

In another embodiment, the variant binding molecule has been covalently modified relative to the prototype binding molecule.

In another embodiment, the binding molecule is a binding sequence. In another embodiment, the variant binding sequence has an amino acid sequence that is at least 50, 55, 60, 65, 70, 75, 80, 85, 90, 95 , 98 or 99% identical to the amino acid sequence of the prototype binding sequence. In another embodiment, the variant binding sequence has been post-translationally modified relative to the prototype binding sequence.

In another aspect, the present invention provides a binding molecule produced or identified by the methods of the present invention.

The present invention can be more fully explained by reference to the following drawings, detailed description and illustrative examples.

### 4. BRIEF DESCRIPTION OF THE DRAWINGS

Figure 1 illustrates an embodiment of the present invention.
Figure 2 illustrates a reporter fusion and a target of the invention.
Figure 3 illustrates an embodiment of the invention by which variant binding sequences are selected on the basis of having a particular dissociation constant that corresponds to tighter binding and a slow dissociation of the binding sequence and the target.
Figure 4 illustrates certain differences between a method of the present invention and a standard ELISA assay.
Figure 5 illustrates an embodiment of the invention for selecting pH-dependent binding sequences.
Figure 6 illustrates an embodiment of the invention for screening reporter fusions on a surface.
Figure 7 illustrates an amino acid sequence of β-lactamase.
Figure 8 presents a schematic diagram of plasmid pADEPT06. P lac = lac promoter, Pel B leader sequence = signal seq, L49VH = Heavy chain, L49VL = Light chain, 218 linker = linker region between heavy and light chains, β-lactamase = β-lactamase gene, L49sFv-bl = scFv-BLA fusion, CAT = chloramphenicol resistance gene.
Figure 9 shows results of a secondary screening of 21 mutants in quadruplicates. The x-axis shows variant designation and the y-axis shows the performance index. A ratio of bound activity at T₁ vs. To was calculated for each mutant, and the performance index was calculated by dividing the ratio of mutant over parent, as shown in Table 3.

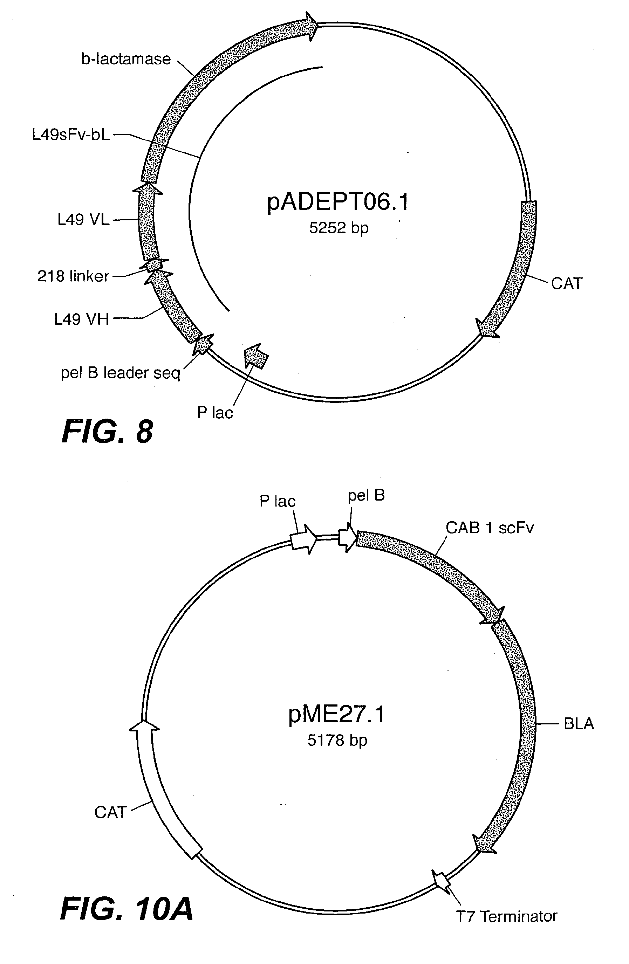

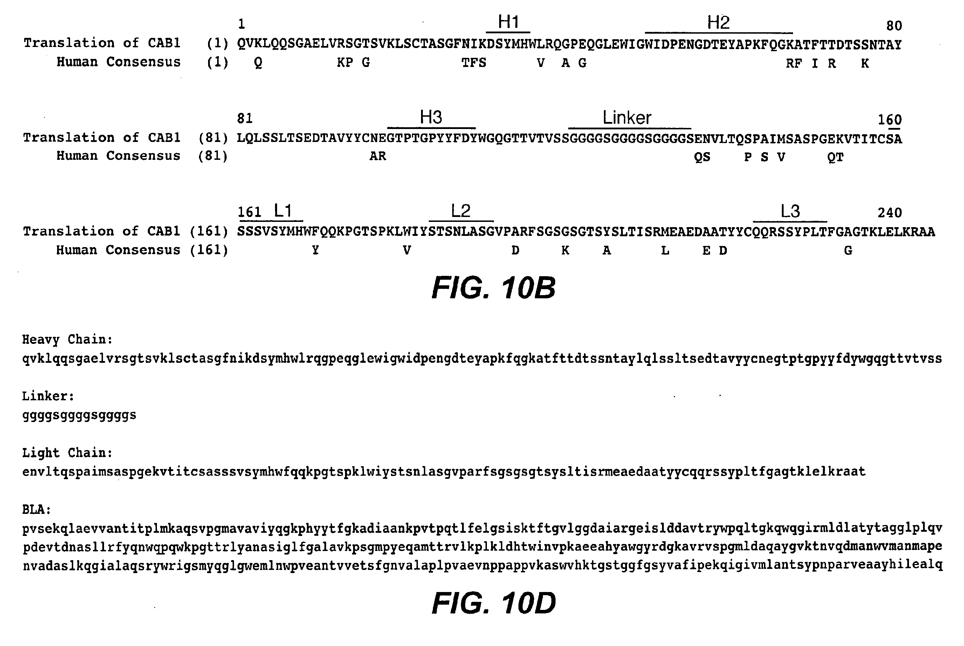

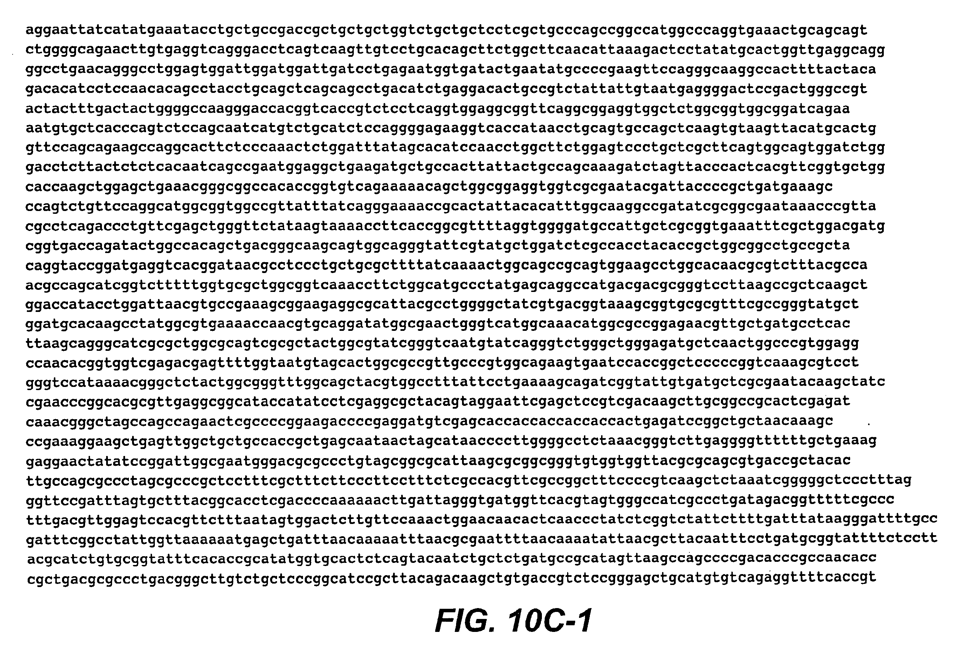

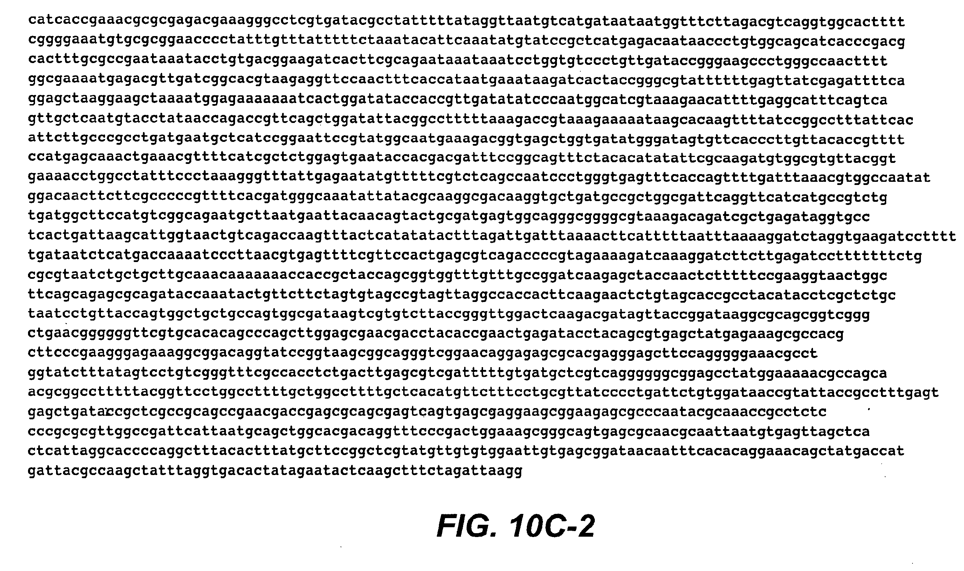

Figure 10 present details related to plasmid pME27.1 Figure 10A presents a schematic diagram of plasmid pME27.1. P lac = lac promoter, Pel B leader sequence = signal seq, CAB1scFv=single chain antibody, BLA= β-lactamase gene, CAT = chloramphenicol resistance gene, T7 terminator=terminator. Figure 10B presents shows the sequence of CAB1-scFv, the CDRs and mutations chosen for combinatorial mutagenesis. Figure 10C presents and nucleotide sequence of pME27.1 Figure 10D shows the amino acid sequence of CAB 1 which shows, for example, the sequence of the heavy chain, the sequence of the linker, the sequence of the light chain and the sequence of BLA.
Figure 11 shows binding assays and SDS page results. Specifically, Figure 11A shows the binding of variants from library NA05; Figure 11B displays and SDS PAGE of stable CAB1-BLA variants of the NA05 library; Figure 11C shows binding of various isolates from NA06 to CEA.
Figure 12 shows a comparison of vH and vL sequences of CAB1-scFv with a published frequency analysis of human antibodies. Specifically, Figure 12A shows the observed frequencies of the five most abundant amino acids in alignment of human sequence in the heavy chain; Figure 12B shows the observed frequencies of the five most abundant amino acids in alignment of human sequence in the light chain.
Figure 13 shows screening results of NA08 library. The x-axis shows binding at pH 7.4, and the Y-axis shows binding at pH 6.5. Clones that were chosen are represented by a square.
Figure 14 shows positions that were chosen for combinatorial mutagenesis.
Figure 15 shows pH-dependent binding of NA08 variants to immobilized carcinoembryonic antigen.
Figure 16 shows a chromatogram for 18 hour old extract of ME27.1
Figure 17 shows an SDS-PAGE for 18 hour extract of ME27.1
Figure 18 shows a chromatogram for 26 hour old extract of ME27.1
Figure 19 shows an SDS-PAGE for 26 hour extract of ME27.1
Figure 20 shows a chromatogram for 26 hour extract for ME 27.1 (4-5 days)
Figure 21 shows an SDS-PAGE for 26 hour old extract. Conditions: 4-12% Tris-Bis/MES/Reducing conditions.
Figure 22 shows CAB1 purification using anion exchange and PBA.

### 5. DETAILED DESCRIPTION OF THE INVENTION

A "binding molecule," unless otherwise stated, includes both "prototype binding molecules" and "variant binding molecules," for example, a binding sequence.

A "prototype binding molecule" is a molecule that has a measurable binding affinity for a target of interest.

A "variant binding molecule" is a molecule that is similar to, but different from, a prototype binding molecule. The difference can be, for example, any difference in structure, including, *e.g*., the addition, deletion or substitution of one or more atoms, amino acid residues or functional groups.

A "binding sequence," unless otherwise stated, includes both "prototype binding sequences" and "variant binding sequences."

A "prototype binding sequence" is a peptide, polypeptide or protein sequence that has a measurable binding affinity for a target of interest.

A "variant binding sequence" is a peptide, polypeptide or protein sequence that is similar to, but different from, a prototype binding sequence. The difference can be, for example, any difference in sequence, including, *e.g*., addition, substitution, and/or deletion of one or more amino acids. The difference also can be or include any form of covalent modification, *e.g.,* a post-translational modification.

A "target" is anything, or any combination of things, to which a peptide, polypeptide or protein can bind.

A "reporter molecule" is a molecule that can be detected independent of its binding to a detectable molecule, for example, a labeled antibody or other reporter molecule-binding molecule. A reporter sequence is a type of reporter molecule.

A "detectable molecule" is a macromolecule that may bind to a molecule and may be used to detected a reporter molecule; a detectable molecule is not a small molecule substrate.

A "reporter sequence" is a reporter molecule that comprises a peptide, polypeptide or protein sequence that can be detected independent of its binding to a detectable molecule, for example, a labeled antibody or other reporter-sequence binding molecule. The reporter sequence can be, for example, an enzyme, a catalytically active fragment or derivative of a protein, or a labeled peptide, polypeptide or protein, *e.g*., a fluorescently labeled or a radioactively labeled peptide, polypeptide or protein.

A "reporter fusion" is a molecule having a binding molecule and a reporter molecule that are bound to each other, *e.g*., covalently bound to each other. The reporter fusion can optionally comprise other elements, for example, one or more linker molecules joining one or more parts of the reporter fusion, e.g., a reporter molecule and a binding molecule. Examples of reporter fusions include chimeric polypeptides and targeted enzymes as described in U.S. Pat. App. Ser. No.s 10/022,073 and 10/022,097, both filed December 13, 2001, and incorporated herein by reference in their entireties.

A "binding characteristic" is a measure of the interaction of two molecules. Examples of binding characteristics include affinity, selectivity, release rate, turnover rate, stability of a molecule necessary for binding and purification of a molecule which has additional or other binding characteristics. Turnover may refer to *in virto* or *in vivo* internalization and/or degradation by a cell or tissue of any or all of the molecules engaged in the binding interaction that renders the molecules unavailable for binding. For example, a target and/or binding molecule may be internalized by a cell and degraded intracellularly. Binding molecules and targets may also be degraded by cell-surface proteases. Alternatively, following internalization, any or all of the binding molecules may be exocytosed to the cell surface and be accessible and available for binding interactions.

"Selectivity" describes the ability of a binding molecule to discriminate between different targets. A binding molecule is said to have high selectivity if it binds with significantly higher affinity to its intended target than to most other surfaces or molecules.

Unless otherwise noted, the term "protein" is used interchangeably here with the terms "peptide" and "polypeptide," and refers to a molecule comprising two or more amino acid residues joined by a peptide bond.

The terms "cell", "cell line", and "cell culture" can be used interchangeably and all such designations include progeny. Thus, the words "transformants" or "transformed cells" include the primary transformed cell and cultures derived from that cell without regard to the number of transfers. All progeny may not be precisely identical in DNA content, due to deliberate or inadvertent mutations. Mutant progeny that have the same functionality as screened for in the originally transformed cell are included in the definition of transformants. The cells can be prokaryotic or eukaryotic.

The term "control sequences" refers to DNA sequences necessary for the expression of an operably linked coding sequence in a particular host organism. The control sequences that are suitable for procaryotes, for example, include a promoter, optionally an operator sequence, a ribosome binding site, positive retroregulatory elements (see, *e.g*., U.S. Pat. No. 4,666,848, incorporated herein by reference), and possibly other sequences. Eucaryotic cells are known to utilize promoters, polyadenylation signals, and enhancers.

The term "expression clone" refers to DNA sequences containing a desired coding sequence and control sequences in operable linkage, so that hosts transformed with these sequences are capable of producing the encoded proteins. The term "expression system" refers to a host transformed with an expression clone. To effect transformation, the expression clone may be included on a vector; however, the relevant DNA may also be integrated into the host chromosome.

The term "gene" refers to a DNA sequence that comprises control and coding sequences necessary for the production of a protein, polypeptide or precursor.

The term "operably linked" refers to the positioning of the coding sequence such that control sequences will function to drive expression of the protein encoded by the coding sequence. Thus, a coding sequence "operably linked" to control sequences refers to a configuration wherein the coding sequences can be expressed under the direction of a control sequence.

The term "oligonucleotide" as used herein is defined as a molecule comprised of two or more deoxyribonucleotides or ribonucleotides. The exact size will depend on many factors, which in turn depends on the ultimate function or use of the oligonucleotide. Oligonucleotides can be prepared by any suitable method, including, for example, cloning and restriction of appropriate sequences and direct chemical synthesis by a method such as the phosphotriester method of Narang et al., 1979, Meth. Enzymol. 68:90-99; the phosphodiester method of Brown et al., 1979, Meth. Enzymol. 68:109-151; the diethylphosphoramidite method of Beaucage et al., 1981, Tetrahedron Lett. 22:1859-1862; and the solid support method of U.S. Pat. No. 4,458,066, each incorporated herein by reference. A review of synthesis methods is provided in Goodchild, 1990, Bioconjugate Chemistry 1(3):165-187, incorporated herein by reference.

The term "primer" as used herein refers to an oligonucleotide which is capable of acting as a point of initiation of synthesis when placed under conditions in which primer extension is initiated. Synthesis of a primer extension product that is complementary to a nucleic acid strand is initiated in the presence of the requisite four different nucleoside triphosphates and a DNA polymerase in an appropriate buffer at a suitable temperature. A "buffer" includes cofactors (such as divalent metal ions) and salt (to provide the appropriate ionic strength), adjusted to the desired pH.

A primer that hybridizes to the non-coding strand of a gene sequence (equivalently, is a subsequence of the coding strand) is referred to herein as an "upstream" or "forward" primer. A primer that hybridizes to the coding strand of a gene sequence is referred to herein as an "downstream" or "reverse" primer.

The terms "restriction endonucleases" and "restriction enzymes" refer to enzymes, typically bacterial in origin, which cut double-stranded DNA at or near a specific nucleotide sequence.

Families of amino acid residues having similar side chains have been defined in the art. These families include amino acids with basic side chains (*e.g*., lysine, arginine, histidine), acidic side chains (*e.g*., aspartic acid, glutamic acid), uncharged polar side chains (*e.g*., asparagine, glutamine, serine, threonine, tyrosine), nonpolar side chains (*e.g*., alanine, valine, leucine, isoleucine, proline, phenylalanine, methionine, tryptophan, cysteine, glycine), beta-branched side chains (*e.g*., threonine, valine, isoleucine) and aromatic side chains (e.g., tyrosine, phenylalanine, tryptophan, histidine). Standard three-letter or one-letter amino acid abbreviations are used herein.

The peptides, polypeptides and proteins of the invention can comprise one or more non-classical amino acids. Non-classical amino acids include but are not limited to the D-isomers of the common amino acids, α-amino isobutyric acid, 4-aminobutyric acid (4-Abu), 2-aminobutyric acid (2- Abu), 6-amino hexanoic acid (Ahx), 2-amino isobutyric acid (2-Aib), 3-amino propionoic acid, ornithine, norleucine, norvaline, hydroxyproline, sarcosine, citrulline, cysteic acid, t-butylglycine, t-butylalanine, phenylglycine, cyclohexylalanine, β-alanine, fluoro-amino acids, designer amino acids such as β-methyl amino acids, Cα-methyl amino acids, Nα-methyl amino acids, and amino acid analogs in general.

As used herein, a "point mutation" in an amino acid sequence refers to either a single amino acid substitution, a single amino acid insertion or single amino acid deletion. A point mutation preferably is introduced into an amino acid sequence by a suitable codon change in the encoding DNA. Individual amino acids in a sequence are represented herein as AN, wherein A is the standard one letter symbol for the amino acid in the sequence, and N is the position in the sequence. Mutations within an amino acid sequence are represented herein as A₁ NA₂, wherein A₁ is the standard one letter symbol for the amino acid in the unmutated protein sequence, A₂ is the standard one letter symbol for the amino acid in the mutated protein sequence, and N is the position in the amino acid sequence. For example, a G46D mutation represents a change from glycine to aspartic acid at amino acid position 46. The amino acid positions are numbered based on the full-length sequence of the protein from which the region encompassing the mutation is derived. Representations of nucleotides and point mutations in DNA sequences are analogous.

As used herein, a "chimeric" protein refers to a protein whose amino acid sequence represents a fusion product of subsequences of the amino acid sequences from at least two distinct proteins. A chimeric protein preferably is not produced by direct manipulation of amino acid sequences, but, rather, is expressed from a "chimeric" gene that encodes the chimeric amino acid sequence.

The term "host immune response" refers to a response of a host organism's immune system to contact with an immunogenic substance. Specific aspects of a host immune response can include, *e.g*.; increased antibody production, T cell activation, monocyte activation or granulocyte activation. Each of these aspects can be detected and/or measured using standard *in vivo* or *in vitro* methods.

The term "Ab" or "antibody" refers to polyclonal and monoclonal antibodies, an entire immunoglobulin or antibody or any functional fragment of an immunoglobulin molecule that binds to the target antigen. Examples of such functional entities include complete antibody molecules, antibody fragments, such as Fv, single chain.Fv, complementarity determining regions (CDRs), V_{L} (light chain variable region), V_{H} (heavy chain variable region), and any combination of those or any other functional portion of an immunoglobulin peptide capable of binding to target antigen.

The term "% sequence homology" is used interchangeably herein with the terms "% homology," "% sequence identity" and "% identity" and refers to the level of amino acid sequence identity between two or more peptide sequences, when aligned using a sequence alignment program. For example, as used herein, 80% homology means the same thing as 80% sequence identity determined by a defined algorithm, and accordingly a homologue of a given sequence has greater than 80% sequence identity over a length of the given sequence. Exemplary levels of sequence identity include, but are not limited to, 60, 70, 80, 85, 90, 95, 98 or 99% or more sequence identity to a given sequence.

Exemplary computer programs which can be used to determine identity between two sequences include, but are not limited to, the suite of BLAST programs, *e.g*., BLASTN, BLASTX, and TBLASTX, BLASTP and TBLASTN, known to one of skill in the art and publicly available on the Internet at http://www.ncbi.nlm.nih.gov/BLAST/". *See also* Altschul et al., 1990, J. Mol. Biol. 215: 403-10 (with special reference to the published default setting, *i.e*., parameters w=4, t=17) and Altschul et al., 1997, Nucleic Acids Res., 25:3389-3402. Sequence searches are typically carried out using the BLASTP program when evaluating a given amino acid sequence relative to amino acid sequences in the GenBank Protein Sequences and other public databases. The BLASTX program is preferred for searching nucleic acid sequences that have been translated in all reading frames against amino acid sequences in the GenBank Protein Sequences and other public databases. Both BLASTP and BLASTX are run using default parameters of an open gap penalty of 11.0, and an extended gap penalty of 1.0, and utilize the BLOSUM-62 matrix. *See* Altschul, *et* a/., 1997.

A preferred alignment of selected sequences in order to determine "% identity" between two or more sequences, is performed using for example, the CLUSTAL-W program in MacVector version 6.5, operated with default parameters, including an open gap penalty of 10.0, an extended gap penalty of 0.1, and a BLOSUM 30 similarity matrix.

"Hit density" is the fraction of useful clones in a library.

"Hapaxomer" is a restriction endonuclease that generates unique ends. *See* Berger, S. L. Anal Biochem 222:1 (1994).

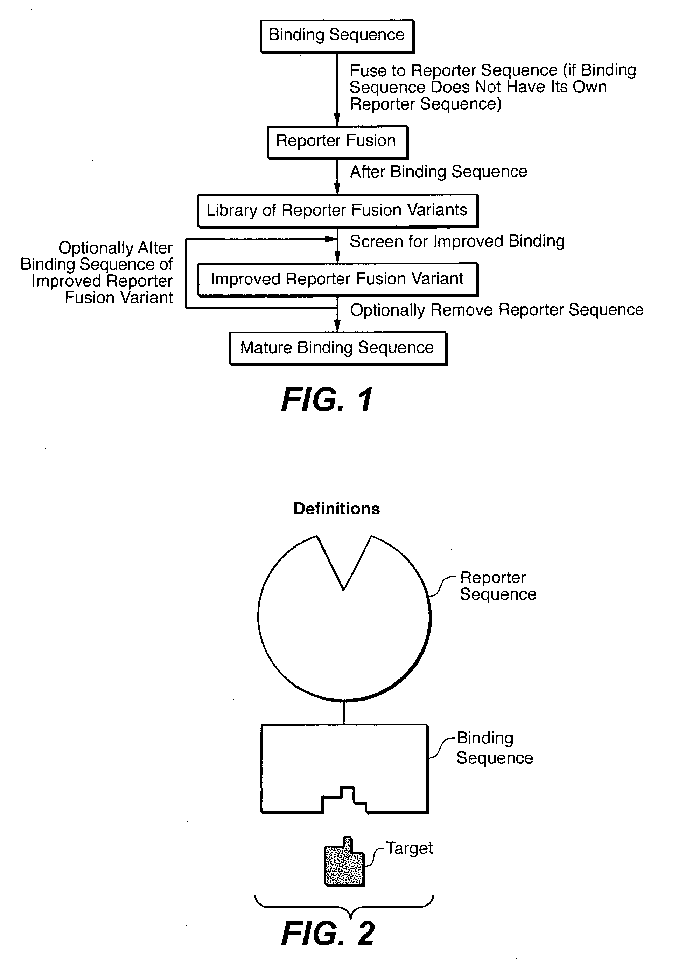

The present invention relates to methods and compositions for identifying variants of a binding molecule that have improved binding properties. The methods use reporter fusions comprising variant binding molecules and reporter molecules. Variants with improved binding properties are identified using the reporter molecule. The process can be repeated multiple times. Ultimately, the binding molecule can be produced without its reporter, either alone or as part of a larger molecule, *e.g*., a binding sequence that is part of a larger polypeptide. One embodiment of the method is illustrated in Figure 1.

### Reporter Fusions

A reporter fusion comprises a binding molecule operably linked to a reporter molecule. The binding molecule and the reporter molecule are operably linked if the binding molecule can bind the target and the reporter molecule can be detected. In one embodiment, the reporter fusion comprises a plurality of binding molecules. In another embodiment, the reporter fusion comprises a plurality of reporter molecules. In another embodiment, the reporter fusion comprises a plurality of binding molecules and a plurality of reporter molecules.

The binding molecule and the reporter molecule can be joined together using any means for doing so provided that the binding molecule is able to bind the target and the reporter molecule is detectable. In one embodiment, the binding molecule and the reporter molecule are covalently attached, for example, covalently attached to each other directly (*e.g*., through a peptide bond or a disulfide bond), or covalently attached to each other via a linker. Examples of linkers include peptides and peptide analogs (*e.g*., peptide nucleic acids), nucleic acids and nucleic acid analogs, and chemical cross linkers such as p-azidobenzoyl hydrazide, N-(4-(p-azidosalicylamido)butyl)-3-(2'-pyridylthio)-propionamide, 1-(p-azidosalicylamido0-4-(iodoacetamido)Butane, 4-(p-azidosalicylamido)butylamine, 4,4'-diazidodiphenyl-ethane, 4,4'-diazidodiphenyl-ether, dithio bis phenyl azide, bis(b-(4-azidosalicylaminoethyl)disulfide, sulfosuccinimidyl-6(4'-azido-2'-nitrophenylamino)hexanoate, sulfosuccinimidyl-4(p-azidophenyl)butyrate, sulfosuccinimidyl-(4-azidosalicylamido)hexanoate, N-hydroxysuccinimidyl-4-azido benzoate, N-hydroxysulfosuccinimidyl-4-azido benzoate, sulfosuccinimidyl-2-(p-azidosalicylamido)-ethyl-1,3-dithiopropionate, p-azidophenyl glyoxal, monohydrate, N-(4-(p-azidosalicylamido)butyl)-3-(2'-pyridylthio)-propionamide, and 1-(p-azidosalicylamido0-4-(iodoacetamido)butane. In a more particularly defined embodiment, the binding molecule is a binding sequence, the reporter molecule is a reporter sequence, and the reporter fusion is a fusion protein. The fusion protein can be synthesized chemically, by direct manipulation and joining of peptides, or translated *in vivo* or *in vitro* from an appropriate nucleic acid template, as described below. Examples of reporter fusions that are fusion proteins are provided in, for example, Yamabhai et al., 1997, Anal. Biochem. 247:143-51; Schlehuber et al., 2001, Biophys. Chem. 96:213-28; Griep et al., 1999, Prot. Express. Purif. 16:63-69; Morino et al., 2001, J. Immunol. Meth. 257:175-84; Wright *et al.,* 2001, 253:223-32, incorporated herein by reference in their entireties.

In one aspect, the present invention provides a library comprising a multiplicity of reporter fusions. Various reporter fusions in the library comprise a reporter sequence and a different variant binding molecule. The variant sequences are similar to, but different from, a prototype binding molecule. In one embodiment, the variant binding molecules are generated from a reporter fusion comprising the reporter molecule and the prototype binding molecule. In another embodiment, the reporter fusion comprises a polypeptide comprising a reporter sequence and a binding sequence. In a more particularly defined embodiment, variant binding sequences are generated by mutating a nucleic acid encoding the reporter fusion. The mutagenesis can target all or part of the prototype binding sequence, and can alter the prototype binding sequence in any way, including, for example, adding, deleting or substituting one or more amino acids. If more than one change is made, they can be made contiguously or in different parts of the prototype binding sequence.

In another embodiment, the reporter fusion comprises the binding sequence and/or the reporter sequence as an integral component. This approach is useful for making diagnostic reagents or targeted enzymes that have therapeutic (*e.g*., TEPT) or other applications. *See, e.g.,* U.S. Pat. App. Ser. No.s 10/022,073 and 10/022,097, both filed December 13, 2001, incorporated therein by reference in their entireties.

In another embodiment, the reporter fusion is made by grafting one or more binding sequences into a reporter sequence, or by grafting one or more reporter sequences into a binding sequence, e.g., as described in copending U.S. Pat. App. Ser. No.s 10/022,073 and 10/022,097, both filed December 13, 2001, or in copending U.S. Pat. App. Ser. No. 60,279,609 and United States Serial Number 10,170,387 (attorney docket no. 9342-041 and 40-999), incorporated herein by reference in their entireties.

### Binding Molecules

A prototype binding molecule comprises a molecule that has a measurable binding affinity for a target of interest. The prototype binding molecule can be any type of molecule, for example, a small organic molecule, a biological molecule (*e.g*., a peptide, a polypeptide, a protein, a nucleic acid, an oligonucleotide, a polynucleotide, a sugar, a metabolite, a lipid, a vitamin, a co-factor, a nucleotide or an amino acid), a polymer, a drug, or an inorganic molecule. In one embodiment, the prototype binding molecule is a prototype binding sequence. A prototype binding sequence comprises a peptide, either alone or covalently attached to one or more other molecules, that binds to a target. The peptide can have any amino acid sequence and can have one or more covalent modifications. In one embodiment, the prototype binding sequence is an antibody, antibody fragment, or derivative. In another embodiment, the prototype binding sequence is not an antibody, antibody fragment, or derivative.

A variant binding molecule is similar to a prototype binding molecule that binds a target but differs from it in one or more aspects. The difference can be any difference that affects a binding property of the binding molecule. The difference can be, for example, one or more insertions, deletions and/or substitutions, or combinations thereof, of atoms, amino acids or functional groups. In one embodiment, the binding molecule is a binding sequence, and the difference is a difference in the amino acid sequence of the prototype binding sequence. The variant binding sequence can be, for example, at least 50, 55, 60, 65, 70, 75, 80, 85, 90, 95 , 98 or 99% identical to the prototype binding sequence. The amino acid sequence of the variant binding sequence can differ from the amino acid sequence of the prototype binding sequence by the presence or absence of one or more non-classical amino acids or chemical amino acid analogs. Non-classical amino acids include but are not limited to the D-isomers of the common amino acids, α-amino isobutyric acid, 4-aminobutyric acid (4-Abu), 2-aminobutyric acid (2- Abu), 6-amino hexanoic acid (Ahx), 2-amino isobutyric acid (2-Aib), 3-amino propionoic acid, ornithine, norleucine, norvaline, hydroxyproline, sarcosine, citrulline, cysteic acid, t-butylglycine, t-butylalanine, phenylglycine, cyclohexylalanine, β-alanine, fluoro-amino acids, designer amino acids such as β-methyl amino acids, Cα-methyl amino acids, Nα-methyl amino acids, and amino acid analogs in general.

In another embodiment, the variant binding sequences has or lacks a covalent modification relative to the prototype binding sequence, for example, glycosylation, methylation, acetylation, phosphorylation, amidation, derivatization by protecting/blocking groups, proteolytic cleavage, etc., as well as any of other numerous chemical modifications, including but not limited to specific chemical cleavage by cyanogen bromide, trypsin, chymotrypsin, papain, V8 protease, NaBH₄, acetylation, formylation, oxidation, reduction, metabolic synthesis in the presence of tunicamycin, *etc.*

These variant sequences can be rationally designed or can be generated by random or semi-random insertions, deletions or substitutions.

The binding molecules of the invention can bind a target with any affinity, e.g., with a K_{d} of about 100 µM or less, 10 µM or less, 1 µM or less, 100 nM or less, about 90 nM or less, about 80 nM or less, about 70 nM or less, about 60 nM or less, about 50 nM or less, about 40 nM or less, about 30 nM or less, about 20 nM or less, about 10 nM or less, about 5 nM or less, about 1 nM or less or about 0.1 nM or less. In one embodiment, the affinity, or another binding characteristic, of a binding molecule for a target is dependent on the conditions under which the binding is conducted. Examples of conditions affecting binding include pH, temperature, light, oxygen tension, salt concentration, the presence or absence of binding co-factors, and other conditions found in copending U.S. Pat. App. Ser. No. 60/279,609 (attorney docket no. 9342-042-999), filed concurrently with the present application, incorporated herein by reference in its entirety.

In one embodiment, the binding molecule is or is part of a targeted enzyme, *e.g.,* as described in copending U.S. Pat. App. Ser. No.s 10/022,073 and 10/022,097, both filed December 13, 2001, incorporated herein by reference in their entireties.

In another embodiment, the binding molecule is or is part of a milieu-dependent binding molecule, e.g., a milieu-dependent targeted agent as described in copending U.S. Pat. App. Ser. No. 60/388,387 (attorney docket no. 9342-042-999), filed concurrently with the present application, incorporated herein by reference in its entirety.

In another embodiment, the binding molecule is or is part of a multifunctional polypeptide, e.g., as described in copending U.S. Pat. App. Ser. No. 10/170,387 (attorney docket no. 9342-043-999), filed concurrently with the present application, incorporated herein by reference in its entirety.

### Reporter molecules

A reporter molecule can be any molecule that can be detected without the necessity of being bound by a detectable molecule, e.g., a labeled antibody or other type of peptide or molecule that is labeled and binds to the reporter molecule. The reporter molecule additionally can have one or more desirable traits, for example, sensitive detection, selection of clones that produce a reporter fusion comprising the reporter molecule and a binding molecule of interest, stabilization of the reporter fusion or the binding molecule, protease-resistance of the reporter fusion or the binding sequence, easy purification, good expression or secretion of product into culture medium. Examples of reporter molecules include radiolabeled substances, fluorescent molecules, light-emitting molecules and molecules catalyzing or otherwise participating in a detectable chemical reaction, e.g., a colorimetric reaction.

In one embodiment, the reporter molecule is a reporter sequence. In another embodiment, the reporter sequence is an enzyme. The enzyme can be any enzyme, or fragment or derivative of an enzyme, that can catalyze the transformation of a substrate into a detectable reaction product. Examples of enzymes that can be used as reporter sequences include β-lactamases, β-galactosidases, phosphatases, peroxidases, reductases, esterases, hydrolases, isomerases and proteases.

In one embodiment, the reporter sequence is the enzyme β-lactamase (BLA). The enzyme is highly active towards the specific substrate nitrocefin which allows the detection of bound reporter fusions at very low concentrations. One can synthesize substrates with higher sensitivity by using fluorogenic leaving groups. These substrates can be designed in analogy to BLA-activated prodrugs. *See, e.g.,* Hudyma et al., 1993, Bioorg Med Chem Lett 3:323-28. BLA can be expressed in high concentration in *E.coli.* In one embodiment, the present invention provides expression vectors that release substantial amounts of BLA into the culture medium which greatly simplifies screening. BLA confers antibiotic resistance to its host. This can be exploited to quickly evaluate the success of a cloning experiment. One can attach a binding sequence to the N-terminus or C-terminus of BLA. If the mutagenesis of the binding sequence leads to sequences that are not correctly translated or that interfere with the cell physiology then such undesirable mutants can in be rapidly identified or eliminated by selection with an appropriate antibiotic like cefotaxime or carbenicillin.

BLA and it s fusion products can be easily purified by affinity chromatography using immobilized phenylboronic acid or similar inhibitors. *See, e.g.,* Cartwright et al., 1984, Biochem J 221:505-12.

Using prodrugs that have been developed for cancer treatment it is possible to select for cells that do not express BLA activity. This can be used to identify mutants where the BLA gene has been inactivated.

In another embodiment, the BLA has a specific activity greater than about 0.01 U/pmol against nitrocefin using the assay described in United States Patent Application Serial Number 10/022,097, filed December 13, 2001, incorporated herein by reference in its entirety. In another embodiment, the specific activity is greater than about 0.1 U/pmol. In another embodiment, the specific activity is greater than about 1 U/pmol.

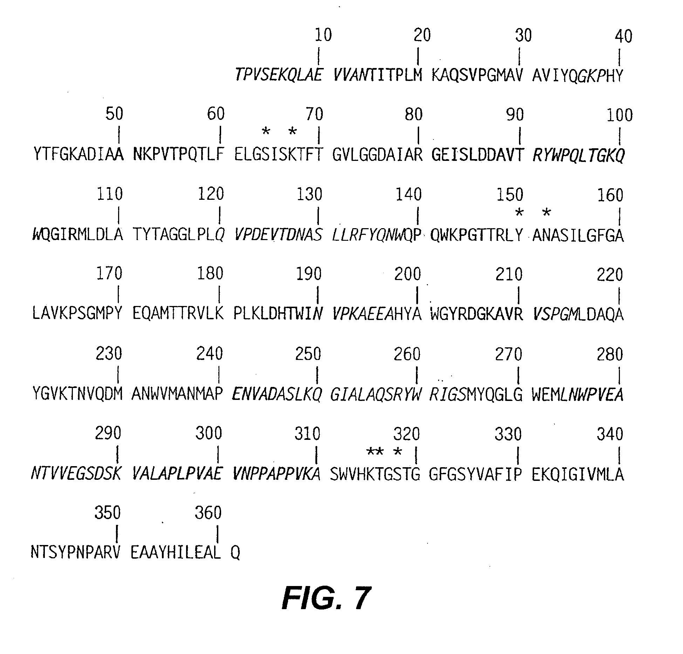

BLA enzymes are widely distributed in both gram-negative and gram-positive bacteria. BLA sequences are well known. A representative example of a BLA sequence is depicted in Figure 7. BLA enzymes vary in specificity, but have in common that they hydrolyze β-lactams, producing substituted β-amino acids. Thus, they confer resistance to antibiotics containing β-lactams. Because BLA enzymes are not endogenous to mammals, they are subject to minimal interference from inhibitors, enzyme substrates, or endogenous enzyme systems and therefore are particularly well-suited for therapeutic administration. BLA enzymes are further well-suited to the therapeutic methods of the present invention because of their small size (BLA from *E. cloacae* is a monomer of 43 kD; BLA from *E*. *coli* is a monomer of 30 kD) and because they have a high specific activity against their substrates and have optimal activity at 37° C. *See* Melton et al., Enzyme-Prodrug Strategies for Cancer Therapy, Kluwer Academic/Plenum Publishers, New York (1999).

The β-lactamases have been divided into four classes based on their sequences. *See* Thomson et al., 2000, Microbes and Infection 2:1225-35. The serine β-lactamases are subdivided into three classes: A (penicillinases), C (cephalosporinases) and D (oxacillnases). Class B β-lactamases are the zinc-containing or metallo β-lactamases. Any class of BLA can be utilized to generate reporter sequence of the invention.

In one embodiment, the present invention provides a BLA reporter sequence that comprises the sequence YXN at its substrate recognition site (throughout, "X" refers to any amino acid residue). In another embodiment, the BLA reporter sequence comprises the sequence RLYANASI at its active site. In another embodiment, the BLA reporter sequence comprises a sequence at its active site that differs from the sequence RLYANASI by one, two or three amino acid residues. The differences can be, for example, the substitution of conservative amino acid residues, insertions, deletions and non-conservative amino acid substitutions.

In another embodiment, the present invention provides a BLA reporter sequence that comprises the sequence KTXS at its substrate recognition site. In another embodiment, the BLA reporter sequence comprises the sequence VHKTGSTG at its active site. In another embodiment, the BLA reporter sequence comprises a sequence at its active site that differs from the sequence VHKTGSTG by one, two or three amino acid residues. The differences can be, for example, the substitution of conservative amino acid residues, insertions, deletions and non-conservative amino acid substitutions.

In another embodiment, the present invention provides a BLA reporter sequence that comprises the sequences YXN and KTXS at its substrate recognition site. In another embodiment, the BLA reporter sequence comprises the sequences VHKTGSTG and RLYANASI at its active site. In another embodiment, the BLA reporter sequence comprises sequences at its active site that differ from the sequences RLYANASI and VHKTGSTG by one, two or three amino acid residues. The differences can be, for example, the substitution of conservative amino acid residues, insertions, deletions and non-conservative amino acid substitutions.

In one embodiment, the BLA reporter sequnce comprises the amino acid sequence of Figure 7. In another embodiment, the BLA reporter sequence is at least 50%, 60%, 70%, 80%, 90%, 95%, 98 or 99% or more identical to the sequence depicted in Figure 7.

In another embodiment, a nucleic acid encoding the BLA reporter sequence hybridizes to a nucleic acid complementary to a nucleic acid encoding the amino acid sequence of Figure 7 under highly stringent conditions. The highly stringent conditions can be, for example, hybridization to filter-bound DNA in 0.5 M NaHPO₄, 7% sodium dodecyl sulfate (SDS), 1 mM EDTA at 65° C, and washing in 0.1xSSC/0.1 % SDS at 68° C (Ausubel et al., eds., 1989, Current Protocols in Molecular Biology, Vol. I, Green Publishing Associates, Inc., and John Wiley & Sons, Inc., New York, at p. 2.10.3). Other highly stringent conditions can be found in, for example, Current Protocols in Molecular Biology, at pages 2.10.1-16 and Molecular Cloning: A Laboratory Manual, 2d ed., Sambrook et al. (eds.), Cold Spring Harbor Laboratory Press, 1989, pages 9.47-57. In another embodiment, a nucleic acid encoding the BLA reporter sequence hybridizes to a nucleic acid complementary to a nucleic acid encoding the amino acid sequence of Figure 7 under moderately stringent conditions. The moderately stringent conditions can be, for example, washing in 0.2xSSC/0.1% SDS at 42° C (Ausubel et al., 1989, *supra*). Other moderately stringent conditions can be found in, for example, Current Protocols in Molecular Biology, Vol. I, Ausubel et al. (eds.), Green Publishing Associates, Inc., and John Wiley & Sons, Inc., 1989, pages 2.10.1-16 and Molecular Cloning: A Laboratory Manual, 2d ed., Sambrook et al. (eds.), Cold Spring Harbor Laboratory Press, 1989, pages 9.47-57.

Fluorescent reporters like green fluorescent protein (GFP) or red fluorescent protein (RFP) also can be used.

In one embodiment, the reporter fusion comprises a plurality of reporter molecules, e.g., a plurality of reporter sequences. In a particular embodiment, a reporter fusion comprises a reporter sequence at its N-terminus and at its C-terminus. A reporter fusion comprising a plurality of reporter sequences is particularly useful if the goal is to screen for protease-resistant variants of a binding sequence. In one embodiment, the reporter fusion comprises a BLA reporter sequence and a fluorescent reporter sequence, e.g., GFP or RFP. The BLA reporter can be used for antibiotic selection and purification and the GFP reporter for detection (e.g., using FACS).

### Targets

The targets of the present invention can be any substance or composition to which a molecule can be made to bind.

In one aspect, the target is a surface. In one embodiment, the surface is a biological surface. In another embodiment, the biological surface is a surface of an organ. In another embodiment, the biological surface is a surface of a tissue. In another embodiment, the biological surface is a surface of a cell. In another embodiment, the biological surface is a surface of a diseased organ, tissue or cell. In another embodiment, the biological surface is a surface of a normal or healthy organ, tissue or cell. In another embodiment, the surface is a macromolecule in the interstitial space of a tissue. In another embodiment, the biological surface is the surface of a virus or pathogen. In another embodiment, the surface is a non-biological surface. In another embodiment, the non-biological surface is a surface of a medical device. In another embodiment, the medical device is a therapeutic device. In another embodiment, the therapeutic device is an implanted therapeutic device. In another embodiment, the medical device is a diagnostic device. In another embodiment, the diagnostic device is a well or tray.

Sources of cells or tissues include human, animal, bacterial, fungal, viral and plant. Tissues are complex targets and refer to a single cell type, a collection of cell types or an aggregate of cells generally of a particular kind. Tissue may be intact or modified. General classes of tissue in humans include but are not limited to epithelial tissue, connective tissue, nerve tissue, and muscle tissue.

In another aspect, the target is a cancer-related target. The cancer-related target can be any target that a composition of the invention binds to as part of the diagnosis, detection or treatment of a cancer or cancer-associated condition in a subject, for example, a cancerous cell, tissue or organ, a molecule associated with a cancerous cell, tissue or organ, or a molecule, cell, tissue or organ that is associated with a cancerous cell, tissue or organ (*e.g*., a tumor-bound diagnostic or therapeutic molecule administered to a subject or to a biopsy taken from a subject, or a healthy tissue, such as vasculature, that is associated with cancerous tissue). Examples of cancer-related targets are provided in U.S. Pat. No. 6,261,535, which is incorporated herein by reference in its entirety.

The cancer-related target can be related to any cancer or cancer-associated condition. Examples of types of cancers include carcinomas, sarcomas, myelomas, leukemias, lymphomas and mixed type cancers.

In one embodiment, the cancer is a bone cancer, for example, Ewing's sarcoma, osteosarcoma and rhabdomyosarcoma and other soft-tissue sarcomas. In another embodiment, the cancer is a brain tumor, for example, oligodendroglioma, ependymoma, menengioma, lymphoma, schwannoma or medulloblastoma. In another embodiment, the cancer is breast cancer, for example, ductal carcinoma *in situ* of the breast. In another embodiment, the cancer is an endocrine system cancer, for example, adrenal, pancreatic, parathyroid, pituitary and thyroid cancers. In another embodiment, the cancer is a gastrointestinal cancer, for example, anal, colorectal, esophogeal, gallbladder, gastric, liver, pancreatic, and small intestine cancers. In another embodiment, the cancer is a gynecological cancer, for example, cervical, endometrial, uterine, fallopian tube, gestational trophoblastic disease, choriocarcinoma, ovarian, vaginal, and vulvar cancers. In another embodiment, the cancer is a head and neck cancer, for example, laryngeal, oropharyngeal, parathryroid or thyroid cancer. In another embodiment, the cancer is a leukemic cancer, for example, acute lymphocytic leukemia, acute myelogenous leukemia, chronic lymphocytic leukemia, chronic myelogenous leukemia, hairy cell leukemia, or a myeloproliferative disorder. In another embodiment, the cancer is a lung cancer, for example, a mesothelioma, non-small cell small cell lung cancer. In another embodiment, the cancer is a lymphoma, for example, AIDS-related lymphoma, cutaneous T cell lymphoma/mucosis fungoides, Hodgkin's disease, or non-Hodgkin's disease. In another embodiment, the cancer is metastatic cancer. In another embodiment, the cancer is a myeloma, for example, a multiple myeloma. In another embodiment, the cancer is a pediatric cancer, for example, a brain tumor, Ewing's sarcoma, leukemia (*e.g*., acute lymphocytic leukemia or acute myelogenous leukemia), liver cancer, a lymphoma (*e.g*., Hodgkin's lymphoma or non-Hodgkin's lymphoma), neuroblastoma, retinoblastoma, a sarcoma (*e.g*., osteosarcoma or rhabdomyosarcoma), or Wilms' Tumor. In another embodiment, the cancer is penile cancer. In another embodiment, the cancer is prostate cancer. In another embodiment, the cancer is a sarcoma, for example, Ewing's sarcoma, osteosarcoma, rhabdomyosarcoma and other soft-tissue sarcomas. In another embodiment, the cancer is a skin cancer, for example, cutaneous T cell lymphoma, mycosis fungoides, Kaposi's sarcoma or melanoma. In another embodiment, the cancer is testicular cancer. In another embodiment, the cancer is thyroid cancer, for example, papillary, follicular, medullary, or anaplastic or undifferentiated thyroid carcinoma. In another embodiment, the cancer is urinary tract cancers, for example, bladder, kidney or urethral cancers. In another embodiment, the cancer or cancer-related condition is ataxia-telangiectasia, carcinoma of unknown primary origin, Li-Fraumeni syndrome, or thymoma.

In another aspect, the cancer-related target is a molecule associated with a cancerous cell or tissue. In one embodiment, the molecule is a tumor or tumor stroma antigen, for example, GD2, Lewis-Y, 72 kd glycoprotein (gp72, decay-accelerating factor, CD55, DAF, C3/C5 convertases), CO17-1A (EpCAM, 17-1A, EGP-40), TAG-72, CSAg-P (CSAp), 45kd glycoprotein, HT-29 ag, NG2., A33 (43kd gp), 38kd gp, MUC-1, CEA, EGFR (HER1), HER2, HER3, HER4, HN-1 ligand, CA125, syndecan-1, Lewis X, PgP, FAP stromal Ag (fibroblast activation protein), EDG receptors (endoglin receptors), ED-B, laminin-5 (gamma2), cox-2 (+LN-5), PgP (P-glycoprotein), alphaVbeta3 integrin, alphaVbeta5, integrin, uPAR (urokinase plasminogen activator receptor), endoglin (CD105), folate receptor osteopontin (EDG 1,3), p97 (melanotransferrin), farnesyl transferase or a molecule in an apoptotic pathway (e.g., a death receptor, fas, caspase or bcl-2) or a lectin.

In another aspect, the target is a hematopoietic cell. Hematopoietic cells encompass hematopoietic stem cells (HSCs), erythrocytes, neutrophils, monocytes, platelets, mast cells, eosinophils, basophils, B and T cells, macrophages, and natural killer cells. In one embodiment, the HSC has a surface antigen expression profile of CD34⁺ Thy-1⁺, and preferably CD34⁺ Thy-1⁺ Lin⁻. Lin⁻ refers to a cell population selected on the basis of the lack of expression of at least one lineage specific marker. Methods for isolating and selecting HSCs are well known in the art and reference is made to U.S. Patent Nos. 5,061,620, 5,677,136, and 5,750,397, each of which is incorporated herein in its entirety.

In another aspect, the target is a molecule. In one embodiment, the molecule is an organic molecule. In another embodiment, the molecule is a biological molecule. In another embodiment, the biological molecule is a cell-associated molecule. In another embodiment, the cell-associated molecule is associated with the outer surface of a cell. In another embodiment, the cell-associated molecule is part of the extracellular matrix. In another embodiment, the cell-associated molecule is associated with the outer surface of a cell is a protein. In another embodiment, the protein is a receptor. In another embodiment, the cell-associated molecule is specific to a type of cell in a subject. In another embodiment, the type of cell is a diseased cell. In another embodiment, the diseased cell is a cancer cell. In another embodiment, the diseased cell is an infected cell. Other molecules that can serve as targets according to the invention include, but are not limited to, proteins, peptides, nucleic acids, carbohydrates, lipids; polysaccharides, glycoproteins, hormones, receptors, antigens, antibodies, toxic substances, metabolites, inhibitors, drugs, dyes, nutrients and growth factors.

In another aspect, the target is a surface feature, the surface feature comprising two or more molecules. The two or more molecule may include, but are not limited to, proteins, peptides, nucleic acids, carbohydrates, lipids, polysacharrides, glycoproteins, hormones, receptors, antigens, antibodies, toxic substances, metabolites, inhibitors, drugs, dyes, nutrients or growth factors.

Non-limiting examples of protein and chemical targets encompassed by the invention include chemokines and cytokines and their receptors. Cytokines as used herein refer to any one of the numerous factors that exert a variety of effects on cells, for example inducing growth or proliferation. Non-limiting examples include interleukins (IL), IL-2, IL-3, IL-4 IL-6, IL-10, IL-12, IL-13, IL-14 and IL-16; soluble IL-2 receptor; soluble IL-6 receptor; erythropoietin (EPO); thrombopoietin (TPO); granulocyte macrophage colony stimulating factor (GM-CSF); stem cell factor (SCF); leukemia inhibitory factor (LIF); interferons; oncostatin M (OM); the immunoglobulin superfamily; tumor necrosis factor (TNF) family, particularly TNF-α; TGFβ; and IL-1α; and vascular endothelial growth factor (VEGF) family, particularly VEGF (also referred to in the art as VEGF-A), VEGF-B, VEGF-C, VEGF-D and placental growth factor (PLGF). Cytokines are commercially available from several vendors including Amgen (Thousand Oaks, CA), Immunex (Seattle, WA) and Genentech (South San Francisco, CA). Particularly preferred are VEGF and TNF-α. Antibodies against TNF-α show that blocking interaction of the TNF-α with its receptor is useful in modulating over-expression of TNF-α in several disease states such as septic shock, rheumatoid arthritis, or other inflammatory processes. VEGF is an angiogenic inducer, a mediator of vascular permeability, and an endothelial cell specific mitogen. VEGF has also been implicated in tumors. Targeting members of the VEGF family and their receptors may have significant therapeutic applications, for example blocking VEGF may have therapeutic value in ovarian hyper stimulation syndrome (OHSS). Reference is made to N. Ferrara et al., (1999) Nat. Med. 5:1359 and Gerber et al., (1999) Nat. Med. 5:623. Other preferred targets include cell-surface receptors, such as T-cell receptors.

Chemokines are a family of small proteins that play an important role in cell trafficking and inflammation. Members of the chemokine family include, but are not limited to, IL-8, stomal-derived factor-1 (SDF-1), platelet factor 4, neutrophil activating protein-2 (NAP-2) and monocyte chemo attractant protein-1 (MCP-1).

Other protein and chemical targets include, but are not limited to:
immunoregulation modulating proteins, such as soluble human leukocyte antigen (HLA, class I and/or class II, and non-classical class I HLA (E, F and G)); surface proteins, such as soluble T or B cell surface proteins; human serum albumin; arachadonic acid metabolites, such as prostaglandins, leukotrienes, thromboxane and prostacyclin; IgE, auto or alloantibodies for autoimmunity or allo- or xenoimmunity, Ig Fc receptors or Fc receptor binding factors; G-protein coupled receptors; cell-surface carbohydrates; angiogenesis factors; adhesion molecules; ions, such as calcium, potassium, magnesium, aluminum, and iron; fibril proteins, such as prions and tubulin; enzymes, such as proteases, aminopeptidases, kinases, phosphatases, DNAses, RNAases, lipases, esterases, dehydrogenases, oxidases, hydrolases, sulphatases, cyclases, transferases, transaminases, carboxylases, decarboxylases, superoxide dismutase, and their natural substrates or analogs; hormones and their corresponding receptors, such as follicle stimulating hormone (FSH), leutinizing hormone (LH), thyroxine (T4 and T3), apolipoproteins, low density lipoprotein (LDL), very low density lipoprotein (VLDL), cortisol, aldosterone, estriol, estradiol, progesterone, testosterone, dehydroepiandrosterone (DHBA) and its sulfate (DHEA-S); peptide hormones, such as renin, insulin, calcitonin, parathyroid hormone (PTH), human growth hormone (hGH), vasopressin and antidiuretic hormone (AD), prolactin, adrenocorticotropic hormone (ACTH), LHRH, thyrotropin-releasing hormone (THRH), vasoactive intestinal peptide (VIP), bradykinin and corresponding prohormones; catechcolamines such as adrenaline and metabolites; cofactors including atrionatriutic factor (AdF), vitamins A, B, C, D, E and K, and serotonin; coagulation factors, such as prothrombin, thrombin, fibrin, fibrinogen, Factor VIII, Factor IX, Factor XI, and von Willebrand factor; plasminogen factors, such as plasmin, complement activation factors, LDL and ligands thereof, and uric acid; compounds regulating coagulation, such as hirudin, hirulog, hementin, hepurin, and tissue plasminigen activator (TPA); nucleic acids for gene therapy; compounds which are enzyme antagonists; and compounds binding ligands, such as inflammation factors; and receptors and other proteins that bind to one or more of the preceding molecules.

Non-human derived targets include without limitation drugs, especially drugs subject to abuse, such as cannabis, heroin and other opiates, phencyclidine (PCP), barbiturates, cocaine and its derivatives, and benzadiazepine; toxins, such as heavy metals like mercury and lead, arsenic, and radioactive compounds; chemotherapeutic agents, such as paracetamol, digoxin, and free radicals; bacterial toxins, such as lipopolysaccharides (LPS) and other gram negative toxins, *Staphylococcus* toxins, Toxin A, Tetanus toxins, Diphtheria toxin and Pertussis toxins; plant and marine toxins; snake and other venoms, virulence factors, such as aerobactins, or pathogenic microbes; infectious viruses, such as hepatitis, cytomegalovirus (CMV), herpes simplex virus (HSV types 1, 2 and 6), Epstein-Barr virus (EBV), varicella zoster virus (VZV), human immunodeficiency virus (HIV-1, -2) and other retroviruses, adenovirus, rotavirus, influenzae, rhinovirus, parvovirus, rubella, measles, polio, pararyxovirus, papovavirus, poxvirus and picornavirus, prions, plasmodia tissue factor, protozoans, such as *Entamoeba histolitica,* Filaria, Giardia, Kalaazar, and toxoplasma; bacteria, gram-negative bacteria responsible for sepsis and nosocomial infections such as *E*. *coli, Acynetobacter, Pseudomonas, Proteus* and *Klebsiella,* also gram-positive bacteria such as *Staphylococcus, Streptococcus, Meningococcus* and *Llycobacteria, Chlamydiae Legionnella* and Anaerobes; fungi such as *Candida, Pneumocystis, Aspergillus,* and *Mycoplasma.*

In one aspect the target includes an enzyme such as proteases, aminopeptidases, kinases, phosphatases, DNAses, RNAases, lipases, esterases, dehydrogenases, oxidases, hydrolases, sulphatases, cellulases, cyclases, transferases, transaminases, carboxylases, decarboxylases, superoxide dismutase, and their natural substrates or analogs. Particularly preferred enzymes include hydrolases, particularly alpha/beta hydrolases; serine proteases, such as subtilisins, and chymotrypsin serine proteases; cellulases; and lipases.

In another embodiment, the target is a non-biological material. In another embodiment, the non-biological material is a fabric. In another embodiment, the fabric is a natural fabric. In another embodiment, the fabric is cotton. In another embodiment, the fabric is silk. In another embodiment, the fabric is wool. In another embodiment, the fabric is a non-natural fabric. In another embodiment, the fabric is nylon. In another embodiment, the fabric is rayon. In another embodiment, the fabric is polyester. In another embodiment, the non-biological material is a plastic. In another embodiment, the non-biological material is a ceramic. In another embodiment, the non-biological material is a metal. In another embodiment, the non-biological material is rubber. In another embodiment, the non-biological material is wood.

In another embodiment the target is a microcircuit. This circuit can be in its finished form or in any stage of circuit manufacturing. *See, e.g.,* van Zant, 2000, Microchip Fabrication, McGraw-Hill, New York, incorporated herein by reference in its entirety.

In another embodiment, the target is not an antibody (*e.g.*, a polyclonal antibody, a monoclonal antibody, an scFv, or another antigen-binding fragment of an antibody).

### Methods of Selecting Variant Binding Molecules

In one aspect, the present invention provides methods of screening reporter fusions comprising variant binding molecules and reporter molecules to identify binding molecules with desired binding characteristics. Any method of screening reporter fusions comprising variant binding molecules and reporter molecules that can identify binding molecules with improved binding characteristics can be used.

Reporter fusions can be used in various ways that allow one to assay for different properties of the binding molecule. For instance, by allowing for sufficient time between measurements to reach binding equilibrium one can identify variants with improved binding affinity. Alternatively, one can screen a population of variants under two or more different conditions to identify variants that differentiate between various targets or variants that show differential binding in dependence of the reaction conditions. *See, e.g.,* U.S. Pat. App. Ser. No. 60/388,387 (attorney docket no. 9342-042-999), filed concurrently with the present application, incorporated herein by reference in its entirety.

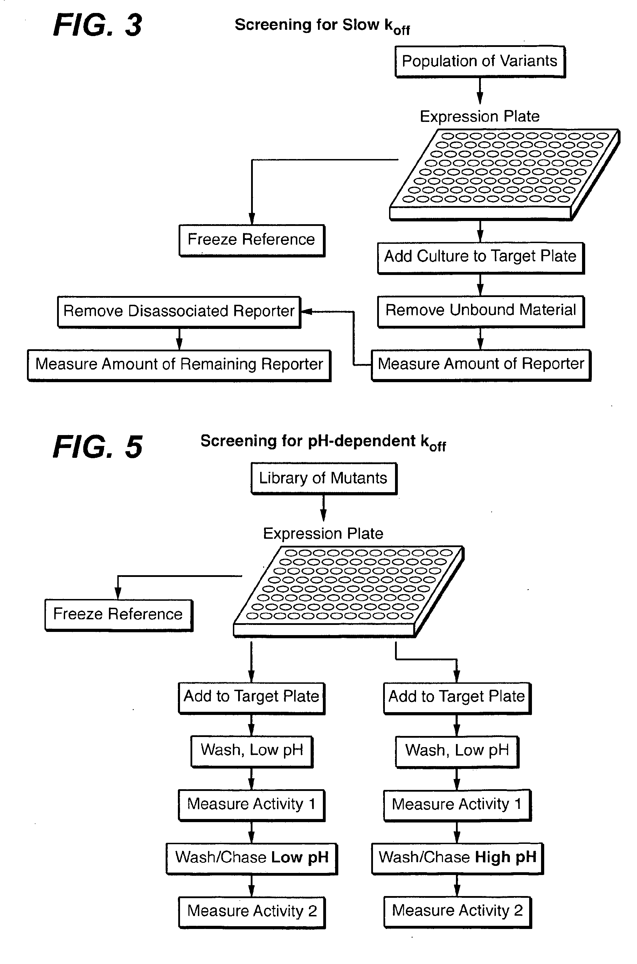

In one embodiment, the binding molecule is a binding sequence. One embodiment of a screen for selecting an improved binding sequence is illustrated in Figure 3. The process starts with a population of clones, where various clones produce a different reporter fusion comprising a different variant of a prototype binding sequence. The clones are cultured under conditions that facilitate the expression of the reporter fusions. In one embodiment, reporter fusions are released by the clones into the culture medium. Subsequently, a part of the culture is transferred to a microtiter plate to which the target of interest has been bound. After incubation to allow target-reporter fusion interaction, unbound reporter fusion is removed, for example, by washing or filtration. Then a chromogenic substrate is added to determine the quantity of bound reporter fusion for each variant During this measurement, a fraction of the bound reporter fusion can dissociate from the target. Subsequently, one can remove the dissociated reporter fusion and add fresh substrate to measure the remaining concentration of bound reporter fusion. This process of washing and measuring can be repeated several times. As a result, one can determine the dissociation rate of each variant in the population and detect variants with improved binding properties.

Figure 5 shows, as an example, a screen at two different pH values. By comparing the values obtained under both pH conditions one can identify variants that show pH-dependent binding to their target. Further examples of methods of screening for binding sequences that bind to a target better under a first set of conditions than they bind under a second set of conditions are provided in copending U.S. Pat. App. Ser. No. 60/388,387 (attorney docket no. 9342-042-999), filed concurrently with the present application, incorporated herein by reference in its entirety. In a similar way, one can compare binding in the presence of different effector molecules to obtain variants which show effector-dependent binding to a target.

In another embodiment, an improved binding molecule is selected by contacting a non-biological target with the reporter fusion, for example, a computer chip at any stage during its manufacture, or after it is manufactured, or a surface (*e.g*., glass, plastic, fabric, film or membrane) exhibiting, *e.g*., coated with, a target molecule.

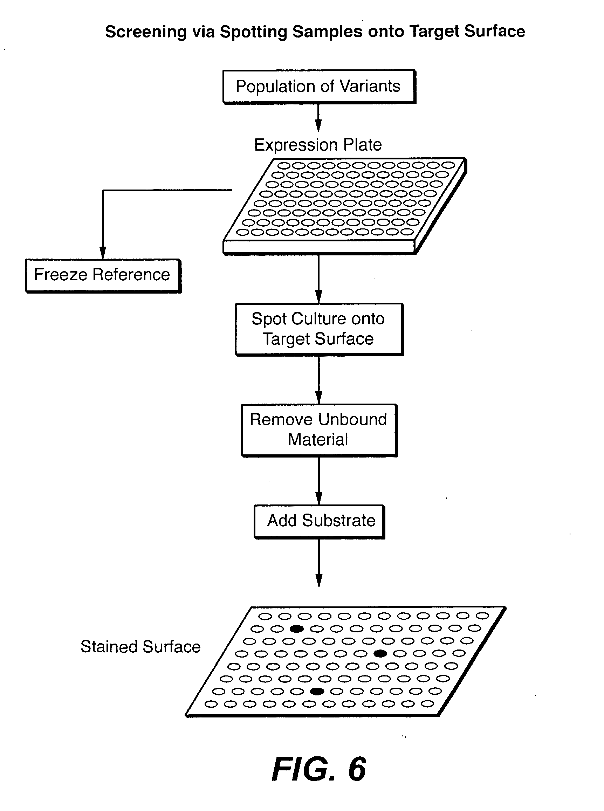

Several methods have been described for manufacturing arrays of compounds. These methods can be adapted to screen populations of reporter fusions for binding to a target. One embodiment of this process is illustrated in Figure 6. Aliquots of variants are transferred onto a surface (*e.g.*, membrane, plastic or glass) which carries bound target. The target can be, for example, a molecule or a cell of interest. Unbound reporter fusions are removed by washing and a substrate is added to detect the remaining bound reporter fusion. It is important to use a substrate that can be used to detect surface-bound reporter fusion. Such substrates are commonly used for immunohistochemistry, *e.g.,* 5-bromo-4-chloro-2-indoyl β-D-galactopyranoside, diaminobenzidine, ELF® 97 esterase substrate (Molecular Probes, Eugene, OR), ELF® 97 phosphatase substrate (Molecular Probes), ELF® 97 β-D-glucuronide, ELF® 97 N-acetylglucosaminide.

In addition, one can use fluorescent reporters to screen for variant binding sequences that bind to a target of interest, *e.g*., a cell. In one embodiment, the assay comprises one or more of the following steps:
Generating population of reporter fusions in a suitable host;
Growing host clones to produce reporter fusions;
Mixing the reporter fusions with a cell suspension;
Adding a fluorescent reference protein that shows fluorescence that can be distinguished from the fluorescent reporter; and
Analyzing each cell suspension in a fluorescence activated cell sorter (FACS) to identify clones of reporter fusions that differ in their binding behavior from the control protein.

In one embodiment, a variant binding sequence is selected by immobilizing reporter fusion-producing cells in agarose beads or similar material to which the target has been attached. The reporter fusion can bind to the target in the bead and it can be detected by, for example, using a fluoregenic substrate, which allows stained beads to be sorted using a fluorescence-activated cell sorter (FACS). *See, e.g.,* Gray et al., 1995, J. Immun. Meth. 182:155-63.

In another embodiment, the screening method comprises multiple rounds of generating a variant binding molecule of a prototype binding molecule, contacting the target with a reporter fusion comprising the variant binding molecule and a reporter molecule, and selecting the variant sequence if it binds to the target with a desired binding characteristic, wherein the variant binding molecule selected in a previous selection step is the prototype binding molecule of the subsequent generating step. Using this approach, multiple rounds of screening can be used to select binding molecules with increasingly refined binding characteristics.

In another embodiment, the screening method comprises contacting the target with a library of reporter fusions, wherein various reporter fusions in the library comprise a different variant binding molecules. In a more particularly defined embodiment, the method comprises using multiple rounds of screening, as described herein, wherein in each round the target is contacted with a library of reporter fusions comprising variant binding molecules that are derived from the binding molecule selected in a previous round.

### Nucleic Acids and Methods of Making Reporter Sequences, Binding Sequences and Reporter Fusions

In another aspect, the present invention provides a nucleic acid encoding a polypeptide comprising all or part of a reporter sequence, a binding sequence or a reporter fusion. The nucleic acid can be, for example, a DNA or an RNA. The present invention also provides a plasmid comprising a nucleic acid encoding a polypeptide comprising all or part of a reporter sequence, a binding sequence or a reporter fusion. The plasmid can be, for example, an expression plasmid that allows expression of the polypeptide in a host cell or organism, or *in vitro.* The expression vector can allow expression of the polypeptide in, for example, a bacterial cell. The bacterial cell can be, for example, an *E. coli* cell.

Because of the redundancy in the genetic code, typically a large number of DNA sequences encode any given amino acid sequence and are, in this sense, equivalent. As described below, it may be desirable to select one or another equivalent DNA sequences for use in a expression vector, based on the preferred codon usage of the host cell into which the expression vector will be inserted. The present invention is intended to encompass all DNA sequences that encode the reporter sequence, binding sequence or reporter fusion.

Production of the reporter sequence, binding sequence or reporter fusion of the invention can be carried out using a recombinant expression clone. The construction of the recombinant expression clone, the transformation of a host cell with the expression clone, and the culture of the transformed host cell under conditions which promote expression, can be carried out in a variety of ways using techniques of molecular biology well understood in the art. Methods for each of these steps are described in general below. Preferred methods are described in detail in the examples.

An operable expression clone is constructed by placing the coding sequence in operable linkage with a suitable control sequences in an expression vector. The vector can be designed to replicate autonomously in the host cell or to integrate into the chromosomal DNA of the host cell. The resulting clone is used to transform a suitable host, and the transformed host is cultured under conditions suitable for expression of the coding sequence. The expressed reporter sequence, binding sequence or reporter fusion is isolated from the medium or from the cells, although recovery and purification of the reporter sequence, binding sequence or reporter fusion may not be necessary in some instances.

Construction of suitable clones containing the coding sequence and a suitable control sequence employs standard ligation and restriction techniques that are well understood in the art. In general, isolated plasmids, DNA sequences, or synthesized oligonucleotides are cleaved, modified, and religated in the form desired. Suitable restriction sites can, if not normally available, be added to the ends of the coding sequence so as to facilitate construction of an expression clone.

Site-specific DNA cleavage is performed by treating with a suitable restriction enzyme (or enzymes) under conditions that are generally understood in the art and specified by the manufacturers of commercially available restriction enzymes. *See, e.g.,* product catalogs from Amersham (Arlington Heights, IL), Roche Molecular Biochemicals (Indianapolis, IN), and New England Biolabs (Beverly, MA). In general, about 1 µg of plasmid or other DNA is cleaved by one unit of enzyme in about 20µl of buffer solution; in the examples below, an excess of restriction enzyme is generally used to ensure complete digestion of the DNA. Incubation times of about one to two hours at a temperature which is optimal for the particular enzyme are typical. After each incubation, protein is removed by extraction with phenol and chloroform; this extraction can be followed by ether extraction and recovery of the DNA from aqueous fractions by precipitation with ethanol. If desired, size separation of the cleaved fragments may be performed by polyacrylamide gel or agarose gel electrophoresis using standard techniques. *See, e.g.,* Maxam et al., 1980, Methods in Enzymology 65:499-560.

Restriction enzyme-cleaved DNA fragments with single-strand "overhanging" termini can be made blunt-ended (double-strand ends) by, for example, treating with the large fragment of *E. coli_*DNA polymerase I (Klenow) in the presence of the four deoxynucleoside triphosphates (dNTPs) using incubation times of about 15 to 25 minutes at 20° C to 25° C in 50 mM Tris, pH 7.6, 50 mM NaCl, 10 mM MgCl₂, 10 mM DTT, and 5 to 10 µM dNTPs. The Klenow fragment fills in at 5' protruding ends, but chews back protruding 3' single strands, even though the four dNTPs are present. If desired, selective repair can be performed by supplying one or more selected dNTPs, within the limitations dictated by the nature of the protruding ends. After treatment with Klenow, the mixture is extracted with phenol/chloroform and ethanol precipitated. Similar results can be achieved using S 1 nuclease, because treatment under appropriate conditions with S1 nuclease results in hydrolysis of any single-stranded portion of a nucleic acid.

Ligations can be performed, for example, in 15-30 µl volumes under the following standard conditions and temperatures: 20 mM Tris-Cl, pH 7.5, 10 mM MgCl₂, 10 mM DTT, 33 µg/ml BSA, 10-50 mM NaCl, and either 40 µM ATP and 0.01-0.02 (Weiss) units T4 DNA ligase at 0° C (for ligation of fragments with complementary single-stranded ends) or 1mM ATP and 0.3-0.6 units T4 DNA ligase at 14° C (for "blunt end" ligation). Intermolecular ligations of fragments with complementary ends are usually performed at 33-100 µg/ml total DNA concentrations (5-100 nM total ends concentration). Intermolecular blunt end ligations (usually employing a 20-30 fold molar excess of linkers, optionally) are performed at 1 µM total ends concentration.

In vector construction, the vector fragment is commonly treated with bacterial or calf intestinal alkaline phosphatase (BAP or CIAP) to remove the 5' phosphate and prevent religation and reconstruction of the vector. BAP and CIAP digestion conditions are well known in the art, and published protocols usually accompany the commercially available BAP and CIAP enzymes. To recover the nucleic acid fragments, the preparation is extracted with phenol-chloroform and ethanol precipitated to remove the phosphatase and purify the DNA. Alternatively, religation of unwanted vector fragments can be prevented by restriction enzyme digestion before or after ligation, if appropriate restriction sites are available.

Correct ligations for plasmid construction can be confirmed using any suitable method known in the art. For example, correct ligations for plasmid construction can be confirmed by first transforming a suitable host, such as *E*. *coli* strain DG101 (ATCC 47043) or *E*. *coli* strain DG116 (ATCC 53606), with the ligation mixture. Successful transformants are selected by ampicillin, tetracycline or other antibiotic resistance or sensitivity or by using other markers, depending on the mode of plasmid construction, as is understood in the art. Plasmids from the transformants are then prepared according to the method of Clewell et al., 1969, Proc. Natl. Acad. Sci. USA 62:1159, optionally following chloramphenicol amplification. *See* Clewell, 1972, J. Bacteriol. 110:667. Alternatively, plasmid DNA can be prepared using the "Base-Acid" extraction method at page 11 of the Bethesda Research Laboratories publication Focus 5 (2), and very pure plasmid DNA can be obtained by replacing steps 12 through 17 of the protocol with CsCl/ethidium bromide ultracentrifugation of the DNA. As another alternative, a commercially available plasmid DNA isolation kit, *e.g*., HISPEED^{™}, QIAFILTER^{™} and QIAGEN^{™} plasmid DNA isolation kits (Qiagen, Valencia CA) can be employed following the protocols supplied by the vendor. The isolated DNA can be analyzed by, for example, restriction enzyme digestion and/or sequenced by the dideoxy method of Sanger et al., 1977, Proc. Natl. Acad. Sci. USA 74:5463, as further described by Messing et al., 1981, Nuc. Acids Res. 9:309, or by the method of Maxam et al., 1980, Methods in Enzymology 65:499.

The control sequences, expression vectors, and transformation methods are dependent on the type of host cell used to express the gene. Generally, procaryotic, yeast, insect, or mammalian cells are used as hosts. Procaryotic hosts are in general the most efficient and convenient for the production of recombinant proteins and are therefore preferred for the expression of the protein.

The procaryote most frequently used to express recombinant proteins is *E*. *coli.* However, microbial strains other than *E*. *coli* can also be used, such as bacilli, for example *Bacillus subtilis,* various species of *Pseudomonas* and *Salmonella,* and other bacterial strains. In such procaryotic systems, plasmid vectors that contain replication sites and control sequences derived from the host or a species compatible with the host are typically used.

For expression of constructions under control of most bacterial promoters, *E. coli* K12 strain MM294, obtained from the *E. coli* Genetic Stock Center under GCSC #6135, can be used as the host. For expression vectors with the P_{L}N_{RBS} or P_{L} T7_{RBS} control sequence, *E. coli* K12 strain MC1000 lambda lysogen, N₇N₅₃cI857 SusP₈₀, ATCC 39531, may be used. *E. coli* DG116 , which was deposited with the ATCC (ATCC 53606) on April 7, 1987, and *E. coli* KB2, which was deposited with the ATCC (ATCC 53075) on March 29, 1985, are also useful host cells. For M13 phage recombinants, *E. coli* strains susceptible to phage infection, such as *E. coli* K12 strain DG98 (ATCC 39768), are employed. The DG98 strain was deposited with the ATCC on July 13, 1984.

For example, *E. coli* is typically transformed using derivatives of pBR322, described by Bolivar et al., 1977, Gene 2:95. Plasmid pBR322 contains genes for ampicillin and tetracycline resistance. These drug resistance markers can be either retained or destroyed in constructing the desired vector and so help to detect the presence of a desired recombinant. Commonly used procaryotic control sequences, i.e., a promoter for transcription initiation, optionally with an operator, along with a ribosome binding site sequence, include the β-lactamase (penicillinase) and lactose (lac) promoter systems, *see* Chang et al., 1977, Nature 198:1056, the tryptophan (trp) promoter system, *see* Goeddel et al., 1980, Nuc. Acids Res. 8:4057, and the lambda-derived P_{L} promoter, *see* Shimatake et al., 1981, Nature 292:128, and gene N ribosome binding site (N_{RBS}). A portable control system cassette is set forth in U.S. Patent No. 4,711,845, issued December 8, 1987. This cassette comprises a P_{L} promoter operably linked to the N_{RBS} in turn positioned upstream of a third DNA sequence having at least one restriction site that permits cleavage within six base pairs 3' of the N_{RBS} sequence. Also useful is the phosphatase A (phoA) system described by Chang *et al.,* in European Patent Publication No. 196,864, published October 8, 1986. However, any available promoter system compatible with procaryotes can be used to construct a expression vector of the invention.

In addition to bacteria, eucaryotic microbes, such as yeast, can also be used as recombinant host cells. Laboratory strains of *Saccharomyces cerevisiae,* Baker's yeast, are most often used, although a number of other strains are commonly available. While vectors employing the two micron origin of replication are common, *see* Broach, 1983, Meth. Enz. 101:307, other plasmid vectors suitable for yeast expression are known. *See, e.g.,* Stinchcomb et al., 1979, Nature 282:39; Tschempe et al., 1980, Gene 10:157; and Clarke et al., 1983, Meth. Enz. 101:300. Control sequences for yeast vectors include promoters for the synthesis of glycolytic enzymes. *See* Hess et al., 1968, J. Adv. Enzyme Reg. 7:149; Holland et al., 1978, Biotechnology 17:4900; and Holland et al., 1981, J. Biol. Chem. 256:1385. Additional promoters known in the art include the promoter for 3-phosphoglycerate kinase, *see* Hitzeman et al., 1980, J. Biol. Chem. 255:2073, and those for other glycolytic enzymes, such as glyceraldehyde 3-phosphate dehydrogenase, hexokinase, pyruvate decarboxylase, phosphofructokinase, glucose-6-phosphate isomerase, 3-phosphoglycerate mutase, pyruvate kinase, triosephosphate isomerase, phosphoglucose isomerase, and glucokinase. Other promoters that have the additional advantage of transcription controlled by growth conditions are the promoter regions for alcohol dehydrogenase 2, isocytochrome C, acid phosphatase, degradative enzymes associated with nitrogen metabolism, and enzymes responsible for maltose and galactose utilization (Holland, *supra*)*.*

Terminator sequences may also be used to enhance expression when placed at the 3' end of the coding sequence. Such terminators are found in the 3' untranslated region following the coding sequences in yeast-derived genes. Any vector containing a yeast-compatible promoter, origin of replication, and other control sequences is suitable for use in constructing yeast expression vectors.

The coding sequence can also be expressed in eucaryotic host cell cultures derived from multicellular organisms. *See, e.g.,* Tissue Culture, Academic Press, Cruz and Patterson, editors (1973). Useful host cell lines include COS-7, COS-A2, CV-1, murine cells such as murine myelomas N51 and VERO, HeLa cells, and Chinese hamster ovary (CHO) cells. Expression vectors for such cells ordinarily include promoters and control sequences compatible with mammalian cells such as, for example, the commonly used early and late promoters from Simian Virus 40 (SV 40), *see* Fiers et al., 1978, Nature 273:113, or other viral promoters such as those derived from polyoma, adenovirus 2, bovine papilloma virus (BPV), or avian sarcoma viruses, or immunoglobulin promoters and heat shock promoters. A system for expressing DNA in mammalian systems using a BPV vector system is disclosed in United States Patent No. 4,419,446. A modification of this system is described in U.S. Patent No. 4,601,978. General aspects of mammalian cell host system transformations have been described by Axel, U.S. Patent No. 4,399,216. "Enhancer" regions are also important in optimizing expression; these are, generally, sequences found upstream of the promoter region. Origins of replication may be obtained, if needed, from viral sources. However, integration into the chromosome is a common mechanism for DNA replication in eucaryotes.

Plant cells can also be used as hosts, and control sequences compatible with plant cells, such as the nopaline synthase promoter and polyadenylation signal sequences, *see* Depicker et al., 1982, J. Mol. Appl. Gen. 1:561, are available. Expression systems employing insect cells utilizing the control systems provided by baculovirus vectors have also been described. *See* Miller et al., in Genetic Engineering (1986), Setlow et al., eds., Plenum Publishing, Vol. 8, pp. 277-97. Insect cell-based expression can be accomplished in *Spodoptera frugipeida.* These systems are also successful in producing recombinant enzymes.

Depending on the host cell used, transformation is done using standard techniques appropriate to such cells. The calcium treatment employing calcium chloride, as described by Cohen, 1972, Proc. Natl. Acad. Sci. USA 69:2110 is used for procaryotes or other cells that contain substantial cell wall barriers. Infection with *Agrobacterium tumefaciens, see* Shaw et al., 1983, Gene 23:315, is used for certain plant cells. For mammalian cells, the calcium phosphate precipitation method of Grahamet al., 1978, Virology 52:546 is preferred. Transformations into yeast are carried out according to the method of Van Solingen et al., 1977, J. Bact. 130:946, and Hsiao et al., 1979, Proc. Natl. Acad. Sci. USA 76:3829.

It may be desirable to modify the sequence of a DNA encoding a polypeptide comprising all or part of a reporter sequence, binding sequence or reporter fusion of the invention to provide, for example, a sequence more compatible with the codon usage of the host cell without modifying the amino acid sequence of the encoded protein. Such modifications to the initial 5-6 codons may improve expression efficiency. DNA sequences which have been modified to improve expression efficiency, but which encode the same amino acid sequence, are considered to be equivalent and encompassed by the present invention.

A variety of site-specific primer-directed mutagenesis methods are available and well-known in the art. *See, e.g.,* Sambrook et al., Molecular Cloning: A Laboratory Manual, Cold Spring Harbor, 1989, second edition, chapter 15.51, "Oligonucleotide-mediated mutagenesis," which is incorporated herein by reference. The polymerase chain reaction (PCR) can be used to perform site-specific mutagenesis. In another technique now standard in the art, a synthetic oligonucleotide encoding the desired mutation is used as a primer to direct synthesis of a complementary nucleic acid sequence contained in a single-stranded vector, such as pBSM13+ derivatives, that serves as a template for construction of the extension product of the mutagenizing primer. The mutagenized DNA is transformed into a host bacterium, and cultures of the transformed bacteria are plated and identified. The identification of modified vectors may involve transfer of the DNA of selected transformants to a nitrocellulose filter or other membrane and the "lifts" hybridized with kinased synthetic mutagenic primer at a temperature that permits hybridization of an exact match to the modified sequence but prevents hybridization with the original unmutagenized strand. Transformants that contain DNA that hybridizes with the probe are then cultured (the sequence of the DNA is generally confirmed by sequence analysis) and serve as a reservoir of the modified DNA.

Once the polypeptide has been expressed in a recombinant host cell, purification of the polypeptide may be desired. A variety of purification procedures can be used.

For long-term stability, the purified polypeptide can be stored in a buffer that contains one or more non-ionic polymeric detergents. Such detergents are generally those that have a molecular weight in the range of approximately 100 to 250,00 preferably about 4,000 to 200,000 daltons and stabilize the enzyme at a pH of from about 3.5 to about 9.5, preferably from about 4 to 8.5. Examples of such detergents include those specified on pages 295-298 of McCutcheon's Emulsifiers & Detergents, North American edition (1983), published by the McCutcheon Division of MC Publishing Co., 175 Rock Road, Glen Rock, NJ (USA), the entire disclosure of which is incorporated herein by reference. Preferably, the detergents are selected from the group comprising ethoxylated fatty alcohol ethers and lauryl ethers, ethoxylated alkyl phenols, octylphenoxy polyethoxy ethanol compounds, modified oxyethylated and/or oxypropylated straight-chain alcohols, polyethylene glycol monooleate compounds, polysorbate compounds, and phenolic fatty alcohol ethers. More particularly preferred are Tween 20^{™}, a polyoxyethylated (20) sorbitan monolaurate from ICI Americas Inc. (Wilmington, DE), and Iconol^{™} NP-40, an ethoxylated alkyl phenol (nonyl) from BASF Wyandotte Corp. (Parsippany, NJ).

The following series of examples are presented by way of illustration and not by way of limitation on the scope of the invention.

### 6. EXAMPLES

The present invention is not to be limited in scope by the specific embodiments described herein. Indeed, various modifications of the invention in addition to those described herein will become apparent to those skilled in the art from the foregoing description and accompanying figures. Such modifications are intended to fall within the scope of the appended claims.

### Example 1: SGN17 His Scan Method

This example demonstrates that a binding sequence can be modified to generate a binding sequence with a higher binding affinity and a binding sequence with a pH dependent binding affinity.

### pADEPT06 DNA Template:

A schematic of plasmid pADEPT06 is shown in Figure 8. This plasmid is 5.2 kb and encodes a single chain antibody variable region fragment (scFv) fused to β-lactamase (BLA) with a pe1B leader sequence, and is driven by a lac promoter (P lac). The plasmid also carries a chloramphenicol resistance gene (CAT) as a selectable marker. This particular SGN17 plasmid was made by.a 3-piece ligation utilizing a linker. Two plasmids were used to make pADEPT06: pCB04 for the vector fragment with the pel B leader sequence, and pCR13 for the scFv-b1a gene: pCBO4 was digested with *Hind*III and *Dra*III (both from New England Biolabs, Beverly, MA) resulting in a 2.7kb fragment with the pCB04 backbone. pCR13 was digested with *Nde*I (Roche Molecular Biochemicals, Indianapolis, Indiana) and *Dra*III resulting in the 2.4kb fragment containing the fusion protein with the pelB leader sequence. Digests pGR13 were done in NEB2 buffer from NEB (50mM NaCl, 10mN Tris-HCl, 10mM MgCl₂, 1mM dithiothreitol (pH 7.9 @ 25° C). Both fragments were gel purified from 1% agarose gel using a Qiagen kit (Qiagen, Valencia, CA). A linker sequence with 5' HindIII complementary ends and *3' Nde*I complementary ends was used to link the 2.7kb fragment and the 2.4kb fragment upstream of the pel B leader sequence. The pCB04 fragment was combined with the pCR13 fragment and the linker in a 1:1:10 molar ratio (respectively), using 17 µl DNA volume (95ng total DNA) and 17 µl Takara ligase solution I (Panvera, Madison, WI) and incubated overnight at 16° C in a PTC-200™ machine (MJ Research, Waltham, MA). Sequencing information shows that the linker region is repeated upstream of the leader sequence.

### Mutagenic Primers:

Overlapping mutagenic primers were designed to replace certain amino acids with histidine residues in the CDR3 regions of both the heavy and light chains of the scFv portion of the scFv-BLA fusion. The wild-type codon to be mutated was changed to the codon CAT (encoding histidine) in a pair of primers. The mutated codon in each primer was flanked on each side by 17 nucleotides of wild-type sequence, unless the primer ended in a stretch of A residues; in this case, the flanking sequence was extended so that it ended with a G or C residue. Each primer was designed so that its mutant codon had the same number of nucleotides flanking it on each side. Each primer was named according to the mutation it was designed to create. For example, HCL100F is the forward primer for the heavy chain (HC) mutating the Leucine (L) in position 100. Its overlapping primer is called HCL100R.

The names and sequences of the mutagenic oligos are provided in Table 1.

**Table 1**

| | SGN17 His Scan Primers |
|---|---|
| Heavy Chain | |
| HCK64F | ACTACAATCCATCTCTC**CAT**AGTCGCATTTCCATCAC |
| HCK64R | GTGATGGAAATGCGACTATGGAGAGATGGATTGTAGT |
| HCR97F | GCCACATATTACTGTGCA**CAT**AGGACTCTGGCTACTTAC |
| HCR97R | GTAAGTAGCCAGAGTCCTATGTGCACAGTAATATGTGGC |
| HCR98F | CATATTACTGTGCAAGA**CAT**ACTCTGGCTACTTACTA |
| HCR98R | TAGTAAGTAGCCAGAGTATGTCTTGCACAGTAATATG |
| HCT99F | ATTACTGTGCAAGAAGG**CAT**CTGGCTACTTACTATGC |
| HCT99R | GCATAGTAAGTAGCCAGATGCCTTCTTGCACAGTAAT |
| HCL100F | ACTGTGCAAGAAGGACT**CAT**GCTACTTACTATGCTAT |
| HCL100R | ATAGCATAGTAAGTAGCATGAGTCCTTCTTGCACAGT |
| HCA101F | GTGCAAGAAGGACTCTG**CAT**ACTTACTATGCTATGGA |
| HCA10IR | TCCATAGCATAGTAAGTATGCAGAGTCCTTCTTGCAC |
| HCT102F | CAAGAAGGACTCTGGCT**CAT**TACTATGCTATGGACTA |
| HCT102R | TAGTCCATAGCATAGTAATGAGCCAGAGTCCTTCTTG |
| HCY103F | GAAGGACTCTGGCTACT**CAT**TATGCTATGGACTACTG |
| HCY103R | CAGTAGTCCATAGCATAATGAGTAGCCAGAGTCCTTC |
| HCYI04F | GGACTCTGGCTACTTAC**CAT**GCTATGGACTACTGGGG |
| HCY104R | CCCCAGTAGTCCATAGCATGGTAAGTAGCCAGAGTCC |
| HCA105F | CTCTGGCTACTTACTAT**CAT**ATGGACTACTGGGGTCA |
| HCA105R | TGACCCCAGTAGTCCATATGATAGTAAGTAGCCAGAG |
| HCM106F | TGGCTACTTACTATGCT**CAT**GACTACTGGGGTCAAGG |
| HCM106R | CCTTGACCCCAGTAGTCATGAGCATAGTAAGTAGCCA |
| HCD107F | CTACTTACTATGCTATG**CAT**TACTGGGGTCAAGGAAC |
| HCD107R | GTTCCTTGACCCCAGTAATGCATAGCATAGTAAGTAG |
| HCY108F | CTTACTATGCTATGGAC**CAT**TGGGGTCAAGGAACCTC |
| HCY108R | GAGGTTCCTTGACCCCAATGGTCCATAGCATAGTAAG |
| HCW109F | ACTATGCTATGGACTAC**CAT**GGTCAAGGAACCTCTGT |
| HCW109R | ACAGAGGTTCCTTGACCATGGTAGTCCATAGCATAGT |
| Light Chain | |
| LCR54 F | CAAAGCTCCTGATCTAC**CAT**GTTTCCAACCGATTTTC |
| LCR54R | GAAAATCGGTTGGAAACATGGTAGATCAGGAGCTTTG |
| LCR58F | GATTTTCTGGGGTCCCAGAC**CAT**TTCAGTGGCAGTGGATCAGG |
| LCR58R | CCTGATCCACTGCCACTGAAATGGTCTGGGACCCCAGAAAATC |
| LCQ94F | GAGTTTATTTCTGCTCT**CAT**AGTACACATGTTCCTCC |
| LCQ94R | GGAGGAACATGTGTACTATGAGAGCAGAAATAAACTC |
| LCS95F | GTTTATTTCTGCTCTCAA**CAT**ACACATGTTCCTCCGACG |
| LCS95R | CGTCGGAGGAACATGTGTATGTTGAGAGCAGAAATAAAC |
| LCT96F | GTTTATTTCTGCTCTCAAAGT**CAT**CATGTTCCTCCGACGTTCGGT |
| LCT96R | ACCGAACGTCGGAGGAACATGATGACTTTGAGAGCAGAAATAAAC |
| LCH97F | TCTGCTCTCAAAGTACA**CAT**GTTCCTCCGACGTTCGG |
| LCH97R | CCGAACGTCGGAGGAACATGTGTACTTTGAGAGCAGA |
| LCV98F | GCTCTCAAAGTACACAT**CAT**CCTCCGACGTTCGGTGG |
| LCV98R | CCACCGAACGTCGGAGGATGATGTGTACTTTGAGAGC |
| LCP99F | CTCAAAGTACACATGTT**CAT**CCGACGTTCGGTGGAGG |
| LCP99R | CCTCCACCGAACGTCGGATGAACATGTGTACTTTGAG |
| LCP100F | CAAAGTACACATGTTCCT**CAT**ACGTTCGGTGGAGGCACC |
| LCP100R | GGTGCCTCCACCGAACGTATGAGGAACATGTGTACTTTG |
| LCT101F | AGTACACATGTTCCTCCG**CAT**TTCGGTGGAGGCACCAAG |
| LCT101R | CTTGGTGCCTCCACCGAAATGCGGAGGAACATGTGTACT |

A11 sequences written 5'-3'. Mutagenic codon in bold and underlined.

A QUICKCHANGE^{™} site directed mutagenesis kit (Stratagene, La Jolla, CA) was used to set up PCR amplifications as follows:

| | |
|---|---|
| H₂O | 39 µl |
| 10x buffer | 5 µl |
| dNTP mix | 1.5 µl |
| Forward primer | 1 µl (0.5 µM final concentration) |
| Reverse primer | 1 µl (0.5 µM final concentration) |
| *pfu* polymerase | 1 µl |
| Plasmid DNA | 1.5 µl (150 ng) |
| Total | 50 µl |

The buffer comprised 100mM KCI, 100mM (NH₄)SO₄, 200mM Tris-HCl (pH 8.8), 20mM MgSO_{4,} 1% Triton X-100, 1mg/ml nuclease-free bovine serum albumin (BSA).

The following touchdown PCR program was used in a PTC-200™ machine (MJ Research, Waltham, MA):
1) 95° C, 2 minutes
2) 95° C, 45 seconds
3) 60° C, 1 minute
   (Reduced by 1.0 °C per cycle)
4) 68° C 11 minutes (i.e., 2 minutes per kb, 5 kb plasmid, plus an additional minute)
5) Go to step (2) for 9 cycles
6) 95° C, 45 seconds
7) 50° C, 1 minute
8) 68° C, 11 minutes
9) Go to step (6) for 5 cycles
10) Hold at 4° C

A negative control without primers was also set up and carried through all steps.

### DpnI digest:

*Dpn*I is a restriction enzyme that cuts methylated and hemimethylated, but not unmethylated, double-stranded DNA. After PCR, 1 µl of *DpnI* was added to each reaction to digest template DNA, which is methylated, but not amplified DNA, most of which is unmethylated, thus reducing the background of wild-type sequence. A sample of the control was saved before digestion. Digests were incubated at 37° C for 1.5 hrs, then each reaction was spiked with an additional 1 µl of *DpnI* and incubated another 1.5 hrs. Reactions were run on a gel after digests alongside the control amplification before and after *Dpn*I digestion. All reactions appeared to work, and, as expected, the control band was fully digested by *DpnI.*

### Transformation:

1 µl of each reaction (not purified), including the digested control, were used to transform 50 µl of Top 10 electro-competent cells (Invitrogen, Carlsbad, CA) and 250 µl SOC medium (2% Bacto-Tryptone, 0.5% Bacto Yeast Extract, 10 mM NaCl, 2.5 mM KCl) was added. The cells were shaken at 37° C for 45 min, then 30 µl of a 1 to 10 dilution was plated (i.e., one tenth of the total volume of each transformation was plated) on both 5ppm chloramphenicol (CMP) and 5ppm CMP + 0.1ppm cefotaxime (CTX) plates. Plates were incubated overnight at 37° C. Transformation results are provided in Table 2.

**Table 2**

| | CMP | CMP + CTX | % ACTIVE |
|---|---|---|---|
| (control) | 0 | 0 | 0 |
| ME43 | 14 | 5 | 36 |
| ME44 | 120 | 34 | 28 |
| ME45 | 784 | 236 | 32 |
| ME46 | 440 | 159 | 36 |
| ME47 | 516 | 184 | 36 |
| ME48 | 268 | 62 | 23 |
| ME49 | 30 | 10 | 33 |
| ME50 | 488 | 61 | 12.5 |
| ME51 | 316 | 57 | 18 |
| ME52 | 380 | 192 | 50 |
| ME53 | 440 | 80 | 18 |
| ME54 | 968 | 308 | 32 |
| ME55 | 356 | 148 | 42 |
| ME56 | 90 | 17 | 19 |
| ME57 | 424 | 112 | 26 |
| ME58 | 38 | 10 | 26 |
| ME59 | 141 | 53 | 38 |
| ME60 | 212 | 144 | 68 |
| ME61 | 90 | 27 | 30 |
| ME62 | 268 | 87 | 32 (WT codon) |
| ME63 | 296 | 88 | 30 |
| ME64 | 196 | 112 | 57 |
| ME65 | 168 | 128 | 76 |
| ME66 | 236 | 76 | 32 |

All bacteria transformed by and expressing a plasmid produced colonies on the CTX plate, and thus provided a measure of the efficiency of transformation. However, only bacteria transformed by plasmids containing a functional BLA grew on the CTX + CMP plates.

Clone names in Table 2 are listed in the same order as the primer pairs used to make them are listed in Table 1, e.g., ME43 was created using primer pair HCK64F/R, ME44 was created using primer pair HCR97F/R, and so on.

Four colonies were picked for each transformation (excluding LCH97 because this represents the wild-type sequence; pADEPT06 WT colonies were picked as a control). Picked colonies were first swirled into a 96 well plate with membrane bottom, each well containing 200ul LB + 5ppm CMP, and then put into the corresponding well of another 96 well plate without filter, to be used as a stock plate.

The 96 well plates were incubated at 25° C in a humidified box with shaking for 48 hrs. Glycerol was added to the stock plate to a final concentration of 10% and stored at -80° C.

### Screening mutants:

Target protein p97 (prepared, for example, by the method set forth in Siemers, N.O., D.E. Kerr, S. Yarnold, M.R. Stebbins, V.M. Vrudhula, I. Hellstrom and P.D. Senter (1997), Bioconj Chem 8, 510-519., Construction, expression and activities of L49-sFv-beta-lactamase, a single-chain antibody fusion protein for anticancer prodrug activation) was immobilized on a polystyrene plate by adding 100 µl of 1 µg/ml p97 in PBS and incubating the plate at 4° C overnight. The plate is then washed with PBST (PBS+0.25%Tween 20) and blocked with 200 µl/well of 1% casein in PBS overnight at 4° C. On the day of screening, the plate was washed with PBST, then each well received 80 µl of 50mM PBS pH7.4 and 20 µl of cell culture broth from each mutant. The plate was incubated at room temperature with gentle shaking to let SGN-17 bind to immobilized p97 on the plate. The amount of each mutant enzyme bound to p97 was determined at two time points. After 1 hour, the plate was washed with PBST, and 200 µl of the BLA substrate nitrocefin in 50mM PBS buffer pH7.4 or pH6.5 was added into each well. The amount of bound SGN-17 was measured by monitoring hydrolysis of nitrocefin at wavelength 490nm. This was the To time point measurement. The plate was then incubated in each substrate buffer for one hour, providing an opportunity for bound mutant SGN-17 to dissociate, then quickly rinsed with PBST. The remaining bound SGN-17 was measured by again monitoring the hydrolysis of substrate nitrocefin in each buffer. This was the T₁ time point measurement. A ratio of bound activity at T₁ vs. To was calculated for each mutant, and an index was calculated by dividing the ratio of mutant over parent, as shown in Table 3.

**Table 3**

| Mutants | sequence | position | region | Index pH7.4 | Index pH6.5 |
|---|---|---|---|---|---|
| ME43 | K | HC62 | CDR2 | 0.61 | 0.65 |
| ME44 | R | HC94 | CDR3 | 0.24 | 0 |
| ME45 | R | HC95 | CDR3 | 0 | 0 |
| ME46 | T | HC96 | CDR3 | 0.38 | 0.09 |
| ME47 | L | HC97 | CDR3 | 0.24 | 0 |
| ME48 | A | HC98 | CDR3 | 0.49 | 0.33 |
| ME50 | Y | HC100 | CDR3 | 0.33 | 0 |
| ME51 | Y | HC101 | CDR3 | 0.26 | 0 |
| ME52 | A | HC102 | CDR3 | 0 | 0 |
| ME53 | M | HC103 | CDR3 | 0.97 | 0.8 |
| ME54 | D | HC104 | CDR3 | 0.41 | 0.7 |
| ME55 | Y | HC105 | CDR3 | 0.8 | 0.7 |
| ME56 | W | HC106 | CDR3 | 0.57 | 0.41 |
| ME58 | R | LC58 | CDR2 | 0.92 | 0.76 |
| ME59 | Q | LC94 | CDR3 | 0.28 | 0 |
| ME60 | S | LC95 | CDR3 | 1.04 | 1.09 |
| ME61 | T | LC96 | CDR3 | 0.82 | 0.81 |
| ME63 | V | LC98 | CDR3 | 0.21 | 0 |
| ME64 | P | LC99 | CDR3 | 0.35 | 0 |
| ME65 | P | LC100 | CDR3 | 0 | 0 |
| ME66 | T | LC101 | CDR3 | 1.36 | 1.73 |

A high index value for a mutant indicates that it has a slow k_{off.} An index value of 0 indicates that no binding was detected for the mutant at that pH.

These data illustrate that many residues in the CDR3 of SGN-17 can be replaced with His while retaining various degrees of binding affinity. Mutagenesis at position LC101 actually leads to an increase in binding affinity which is larger at pH 6.5 as compared to pH 7.4. Thus, the introduction of a His in position LC101 affects the pH-dependence of target binding of SGN-17. Comparing the index values at both pH values shows that several of the tested mutations affect pH-dependence of binding. Stronger effects can be achieved by adding further mutations, by testing substitutions other then His, by testing substitutions, insertions or deletions at more positions of the binding moiety, or by extending the mutagenesis to the BLA part of the fusion protein.

### Example 2: Affinity maturation of an scFv by site saturation scanning mutagenesis

### A. Generation of site saturation libraries

64 site saturation mutagenesis libraries were generated. In each of these libraries, one codon, that codes for a CDR position (as defined by the Kabat nomenclature) in ME66.4-scFv, exactly the same as ME66, was randomized. The libraries were generated using the QuikChange protocol (Stratagene, La Jolla, CA) essentially as recommended by the manufacturer. Each reaction used two mutagenic oligonucleotides which had the following design: 17 perfect matches flanking the random codon on each side, NNS in place of the random codon. For example, library ME67 used the forward primer CTGGCGACTCCATCACCNNSGGTTACTGGAACTGGAT and the reverse primer ATCCAGTTCCAGTAACCSNNGGTGATGGAGTCGCCAG, where N represents a mixture of A, T, G, and C and S represents a mixture of G and C. This approach allows for the generation of 32 different codons which encode all 20 amino acids. After the QuikChange reaction and Dpn I digest, which degrades parent plasmid, the reaction mixture was used to transform TOP 10 cells (Invitrogen, Carlsbad, CA) by electroporation.

**Table 4: oligonucleotides used to generate the 64 site saturation libraries: Heavy Chain**

| | |
|---|---|
| **H31** | CTGGCGACTCCATCACCNNSGGTTACTGGAACTGGAT |
| **H31** | ATCCAGTTCCAGTAACCSNNGGTGATGGAGTCGCCAG |
| **H32** | GCGACTCCATCACCAGTNNSTACTGGAACTGGATCCG |
| **H32** | CGGATCCAGTTCCAGTASNNACTGGTGATGGAGTCGC |
| **H33** | ACTCCATCACCAGTGGTNNSTGGAACTGGATCCGGCA |
| **H33** | TGCCGGATCCAGTTCCASNNACCACTGGTGATGGAGT |
| **H34** | TCCATCACCAGTGGTTACNNSAACTGGATCCGGCAGTTC |
| **H34** | GAACTGCCGGATCCAGTTSNNGTAACCACTGGTGATGGA |
| **H50** | AACTTGAATATATGGGTNNSATAAGCGACAGTGGTAT |
| **H50** | ATACCACTGTCGCTTATSNNACCCATATATTCAAGTT |
| **H51** | TTGAATATATGGGTTACNNSAGCGACAGTGGTATCAC |
| **H51** | GTGATACCACTGTCGCTSNNGTAACCCATATATTCAA |
| **H52** | GAATATATGGGTTACATANNSGACAGTGGTATCACTTAC |
| **H52** | GTAAGTGATACCACTGTCSNNTATGTAACCCATATATTC |
| **H53** | TATATGGGTTACATAAGCNNSAGTGGTATCACTTACTAC |
| **H53** | GTAGTAAGTGATACCACTSNNGCTTATGTAACCCATATA |
| **H54** | ATGGGTTACATAAGCGACNNSGGTATCACTTACTACAAT |
| **H54** | ATTGTAGTAAGTGATACCSNNGTCGCTTATGTAACCCAT |
| **H55** | GTTACATAAGCGACAGTNNSATCACTTACTACAATCC |
| **H55** | GGATTGTAGTAAGTGATSNNACTGTCGCTTATGTAAC |
| **H56** | ACATAAGCGACAGTGGTNNSACTTACTACAATCCATC |
| **H56** | GATGGATTGTAGTAAGTSNNACCACTGTCGCTTATGT |
| **H57** | TAAGCGACAGTGGTATCNNSTACTACAATCCATCTCT |
| **H57** | AGAGATGGATTGTAGTASNNGATACCACTGTCGCTTA |
| **H58** | TAAGCGACAGTGGTATCACTNNSTACAATCCATCTCTCAAAAG |
| **H58** | CTTTTGAGAGATGGATTGTASNNAGTGATACCACTGTCGCTTA |
| **H59** | GACAGTGGTATCACTTACNNSAATCCATCTCTCAAAAGT |
| **H59** | ACTTTTGAGAGATGGATTSNNGTAAGTGATACCACTGTC |
| **H60** | GTGGTATCACTTACTACNNSCCATCTCTCAAAAGTCG |
| **H60** | CGACTTTTGAGAGATGGSNNGTAGTAAGTGATACCAC |
| **H61** | GTATCACTTACTACAATNNSTCTCTCAAAAGTCGCAT |
| **H61** | ATGCGACTTTTGAGAGASNNATTGTAGTAAGTGATAC |
| **H62** | TCACTTACTACAATCCANNSCTCAAAAGTCGCATTTC |
| **H62** | GAAATGCGACTTTTGAGSNNTGGATTGTAGTAAGTGA |
| **H63** | CTTACTACAATCCATCTNNSAAAAGTCGCATTTCCAT |
| **H63** | ATGGAAATGCGACTTTTSNNAGATGGATTGTAGTAAG |
| **H64** | ACTACAATCCATCTCTCNNSAGTCGCATTTCCATCAC |
| **H64** | GTGATGGAAATGCGACTSNNGAGAGATGGATTGTAGT |
| **H65** | ACAATCCATCTCTCAAANNSCGCATTTCCATCACTCG |
| **H65** | CGAGTGATGGAAATGCGSNNTTTGAGAGATGGATTGT |
| **H97** | GCCACATATTACTGTGCANNSAGGACTCTGGCTACTTAC |
| **H97** | GTAAGTAGCCAGAGTCCTSNNTGCACAGTAATATGTGGC |
| **H98** | CATATTACTGTGCAAGANNSACTCTGGCTACTTACTA . |
| **H98** | TAGTAAGTAGCCAGAGTSNNTCTTGCACAGTAATATG |
| **H99** | ATTACTGTGCAAGAAGGNNSCTGGCTACTTACTATGC |
| **H99** | GCATAGTAAGTAGCCAGSNNCCTTCTTGCACAGTAAT |
| **H100** | ACTGTGCAAGAAGGACTNNSGCTACTTACTATGCTAT |
| **H100** | ATAGCATAGTAAGTAGCSNNAGTCCTTCTTGCACAGT |
| **H101** | GTGCAAGAAGGACTCTGNNSACTTACTATGCTATGGA |
| **H101** | TCCATAGCATAGTAAGTSNNCAGAGTCCTTCTTGCAC |
| **H102** | CAAGAAGGACTCTGGCTNNSTACTATGCTATGGACTA |
| **H102** | TAGTCCATAGCATAGTASNNAGCCAGAGTCCTTCTTG |
| **H103** | GAAGGACTCTGGCTACTNNSTATGCTATGGACTACTG |
| **H103** | CAGTAGTCCATAGCATASNNAGTAGCCAGAGTCCTTC |
| **H104** | GGACTCTGGCTACTTACNNSGCTATGGACTACTGGGG |
| **H104** | CCCCAGTAGTCCATAGCSNNGTAAGTAGCCAGAGTCC |
| **H105** | CTCTGGCTACTTACTATNNSATGGACTACTGGGGTCA |
| **H105** | TGACCCCAGTAGTCCATSNNATAGTAAGTAGCCAGAG |
| **H106** | TGGCTACTTACTATGCTNNSGACTACTGGGGTCAAGG |
| **H106** | CCTTGACCCCAGTAGTCSNNAGCATAGTAAGTAGCCA |
| **H107** | CTACTTACTATGCTATGNNSTACTGGGGTCAAGGAAC |
| **H107** | GTTCCTTGACCCCAGTASNNCATAGCATAGTAAGTAG |
| **H108** | CTTACTATGCTATGGACNNSTGGGGTCAAGGAACCTC |
| **H108** | GAGGTTCCTTGACCCCASNNGTCCATAGCATAGTAAG |
| **H109** | ACTATGCTATGGACTACNNSGGTCAAGGAACCTCTGT |
| **H109** | ACAGAGGTTCCTTGACCSNNGTAGTCCATAGCATAGT |
| **Light Chain** | |
| **L24** | CCTCCATCTCTTGCAGGNNSAGTCAGAGCCTTGTACA |
| **L24** | TGTACAAGGCTCTGACTSNNCCTGCAAGAGATGGAGG |
| **L25** | CCATCTCTTGCAGGGCTNNSCAGAGCCTTGTACACAG |
| **L25** | CTGTGTACAAGGCTCTGSNNAGCCCTGCAAGAGATGG |
| **L26** | ATCTCTTGCAGGGCTAGTNNSAGCCTTGTACACAGTAAT |
| **L26** | ATTACTGTGTACAAGGCTSNNACTAGCCCTGCAAGAGAT |
| **L27** | CTTGCAGGGCTAGTCAGNNSCTTGTACACAGTAATGG |
| **L27** | CCATTACTGTGTACAAGSNNCTGACTAGCCCTGCAAG |
| **L28** | TGCAGGGCTAGTCAGAGCNNSGTACACAGTAATGGAAAC |
| **L28** | GTTTCCATTACTGTGTACSNNGCTCTGACTAGCCCTGCA |
| **L29** | GGGCTAGTCAGAGCCTTNNSCACAGTAATGGAAACAC |
| **L29** | GTGTTTCCATTACTGTGSNNAAGGCTCTGACTAGCCC |
| **L30** | CTAGTCAGAGCCTTGTANNSAGTAATGGAAACACCTA |
| **L30** | TAGGTGTTTCCATTACTSNNTACAAGGCTCTGACTAG |
| **L31** | TAGTCAGAGCCTTGTACACNNSAATGGAAACACCTATTTAC |
| **L31** | GTAAATAGGTGTTTCCATTSNNGTGTACAAGGCTCTGACTA |
| **L32** | AGAGCCTTGTACACAGTNNSGGAAACACCTATTTACA |
| **L32** | TGTAAATAGGTGTTTCCSNNACTGTGTACAAGGCTCT |
| **L33** | GCCTTGTACACAGTAATNNSAACACCTATTTACATTG |
| **L33** | CAATGTAAATAGGTGTTSNNATTACTGTGTACAAGGC |
| **L34** | TTGTACACAGTAATGGANNSACCTATTTACATTGGTA |
| **L34** | TACCAATGTAAATAGGTSNNTCCATTACTGTGTACAA |
| **L35** | TACACAGTAATGGAAACNNSTATTTACATTGGTACC |
| **L35** | GGTACCAATGTAAATASNNGTTTCCATTACTGTGTA |
| **L36** | ACAGTAATGGAAACACCNNSTTACATTGGTACCTGCA |
| **L36** | TGCAGGTACCAATGTAASNNGGTGTTTCCATTACTGT |
| **L37** | AGTAATGGAAACACCTATNNSCATTGGTACCTGCAGAAG |
| **L37** | CTTCTGCAGGTACCAATGSNNATAGGTGTTTCCATTACT |
| **L38** | ATGGAAACACCTATTTANNSTGGTACCTGCAGAAGCC |
| **L38** | GGCTTCTGCAGGTACCASNNTAAATAGGTGTTTCCAT |
| **L53** | CTCCAAAGCTCCTGATCNNSAGAGTTTCCAACCGATT |
| **L53** | AATCGGTTGGAAACTCTSNNGATCAGGAGCTTTGGAG |
| **L54** | CAAAGCTCCTGATCTACNNSGTTTCCAACCGATTTTC |
| **L54** | GAAAATCGGTTGGAAACSNNGTAGATCAGGAGCTTTG |
| **L55** | AGCTCCTGATCTACAGANNSTCCAACCGATTTTCTGG |
| **L55** | CCAGAAAATCGGTTGGASNNTCTGTAGATCAGGAGCT |
| **L56** | TCCTGATCTACAGAGTTNNSAACCGATTTTCTGGGGT |
| **L56** | ACCCCAGAAAATCGGTTSNNAACTCTGTAGATCAGGA |
| **L57** | TGATCTACAGAGTTTCCNNSCGATTTTCTGGGGTCCC |
| **L57** | GGGACCCCAGAAAATCGSNNGGAAACTCTGTAGATCA |
| **L58** | TCTACAGAGTTTCCAACNNSTTTTCTGGGGTCCCAGA |
| **L58** | TCTGGGACCCCAGAAAASNNGTTGGAAACTCTGTAGA |
| **L59** | ACAGAGTTTCCAACCGANNSTCTGGGGTCCCAGACAG |
| **L59** | CTGTCTGGGACCCCAGASNNTCGGTTGGAAACTCTGT |
| **L60** | GAGTTTCCAACCGATTTNNSGGGGTCCCAGACAGGTT |
| **L60** | AACCTGTCTGGGACCCCSNNAAATCGGTTGGAAACTC |
| **L94** | GAGTTTATTTCTGCTCTNNSAGTACACATGTTCCTCC |
| **L94** | GGAGGAACATGTGTACTSNNAGAGCAGAAATAAACTC |
| **L95** | GAGTTTATTTCTGCTCTCAANNSACACATGTTCCTCCGCATTT |
| **L95** | AAATGCGGAGGAACATGTGTSNNTTGAGAGCAGAAATAAACTC |
| **L96** | TATTTCTGCTCTCAAAGTNNSCATGTTCCTCCGCATTTC |
| **L96** | GAAATGCGGAGGAACATGSNNACTTTGAGAGCAGAAATA |
| **L97** | TCTGCTCTCAAAGTACANNSGTTCCTCCGCATTTCGG |
| **L97** | CCGAAATGCGGAGGAACSNNTGTACTTTGAGAGCAGA |
| **L98** | GCTCTCAAAGTACACATNNSCCTCCGCATTTCGGTGG |
| **L98** | CCACCGAAATGCGGAGGSNNATGTGTACTTTGAGAGC |
| **L99** | CTCAAAGTACACATGTTNNSCCGCATTTCGGTGGAGG |
| **L99** | CCTCCACCGAAATGCGGSNNAACATGTGTACTTTGAG |
| **L100** | CAAAGTACACATGTTCCTNNSCATTTCGGTGGAGGCACC |
| **L100** | GGTGCCTCCACCGAAATGSNNAGGAACATGTGTACTTTG |
| **L101** | AGTACACATGTTCCTCCGNNSTTCGGTGGAGGCACCAAG |
| **L101** | CTTGGTGCCTCCACCGAASNNCGGAGGAACATGTGTACT |

### B. Screen for improved binding

Libraries were plated onto agar plates containing LB medium and 5 mg/l chloramphenicol and 0.1 mg/l cefotaxime (Sigma): 88 colonies from each library and parent colonies were picked and inoculated into 384-well plates containing 80 ul LB containing 5 mg/l chloramphenicol and 0.1 mg/l cefotaxime. Plates were incubated at 25 C in humidified boxes with shaking for 48 hrs.

Target protein p97 was immobilized in 384-well polystyrene plates by adding 40 ul of 1 ug/ml p97 in PBS and incubating the plate at 4C overnight. The plates were then washed with PBST (PBS+0.1%Tween-20) and blocked with 200ul/well of 1% Casein in PBS overnight at 4C. On the day of screening, the plates were washed with PBST. Subsequently, 24ul/well of 50mM PBS pH7.4 was first added into plate each well followed by 8 ul of cell culture broth from expression plates. The plate was incubated at room temperature with gentle shaking to let ME66-scFv to bind to immobilized P97 on the plate. After 1 hour, the plate was washed with PBST and 200ul of BLA assay buffer containing 0.1 mg/ml nitrocefin (Oxoid, New York) in 50mM PBS buffer pH6.5 was added into each well, the bound ME66scFv was measured by monitoring hydrolysis of nitrocefin at wavelength 490nm. The plate was then left incubated in substrate buffer to allow the bound ME66scFv-BLA to dissociate, after 1.5 hour the plate was quickly rinsed with PBST. The remaining bound ME66scFv-BLA was again measured by monitoring the hydrolysis of freshly added substrate nitrocefin. Dissociation of ME66-scFv from p97 was monitored again after 3-5 hours. A ratio of bound activity at time 1 vs. time 0 or time 2 vs. time 0 was calculated for each mutant from dissociation data, an index at each time point was further calculated by dividing ratio of mutant over parent, and winner mutants were chosen if they had a high index.

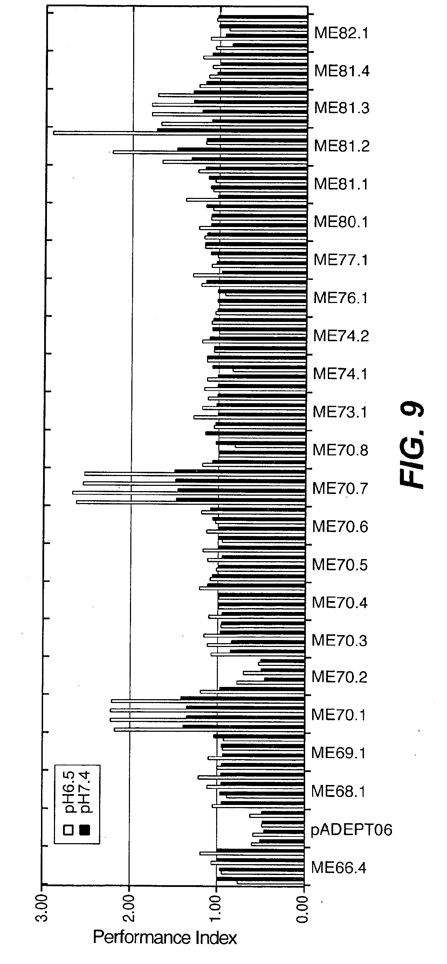

After the primary screening, 21 winners were chosen for repeat analysis in quadruplicates. Each winner was streaked out on LA agar containing 5 mg/l chloramphenicol, 4 colonies from each winner were transferred in 96 well plate containing 200ul/well LB containing 5 mg/l chloramphenicol. Some wells were inoculated with ME66.4 as a reference. The plate was incubated at 25C for 70 hours. Target protein p97 was bitotinylated and immobilized in 96 well neutravidin (Pierce, Rockford, 11) plate at a p97 concentration of Sug/ml of 100ul/well, the plate was then blocked with 1% Casein. On the day of screening, 70ul/well of PBS buffer pH7.4 was added into target plate, and 20ul/well of culture broth was transferred from expression plate to target plate. The plates were incubated at room temperature for 1 hour, and were then washed with PBST. 200ul of BLA substrate nitrocefin in 50mM PBS buffer pH6.5 were added into each well, and the bound ME66scFv was measured by monitoring hydrolysis of nitrocefin at wavelength 490nm. The plate was left incubated in substrate buffer for an additional 1.5 hour. After quick rinsing with PBST, the bound ME66scFv-BLA was again measured using nitrocefin. The dissociation of ME66scFv from p97 was again measured between 3-6 hours after the initial time point and a binding index was calculated at 2 time points. In parallel, the plate was screened under identical conditions but using 50 mM PBS buffer at pH 7.4. Data were normalized as described in Example 1. The normalized screening results measured at pH 6.5 and at pH 7.4 are shown in the Figure 9.

Table 6, below, shows mutations that have been observed in the three best variants.

**Table 4: Mutations in affinity matured variants**

| Clone | mutation |
|---|---|
| ME70.1 | heavy chain, S65K |
| ME70.7 | heavy chain, S65P |
| ME81.3 | heavy chain, N60R |

### Example 3: Stabilization of an scFv

### A. Construction of pME27.1

Plasmid pME27.1 was generated by inserting a Bgl I EcoRV fragment encoding a part of the pelB leader, the CAB1-scFv and a small part of BLA into plasmid the expression vector pME25. The insert, encoding for the CAB1-scFv, has been synthesized by Aptagen (Herndon, VA) based on the sequence of the scFv MFE-23 that was described in [Boehm, M. K., A. L. Corper, T. Wan, M. K. Sohi, B. J. Sutton, J. D. Thornton, P. A. Keep, K. A. Chester, R. H. Begent and S. J. Perkins (2000) Biochem J 346 Pt 2, 519-28, Crystal structure of the anti-(carcinoembryonic antigen) single-chain Fv antibody MFE-23 and a model for antigen binding based on intermolecular contacts]. Both the plasmid containing the synthetic gene (pPCR-GME1) and pME25 were digested with BglI and EcoRV, gel purified and ligated together with Takara ligase. Ligation was transformed into TOP10(Invitrogen, Carlsbad, CA) electrocompetent cells, plated on LA medium containing 5 mg/l chloramphenicol and 0.1 mg/l cefotaxime.

**Plasmid pME27.1 contains the following features:**

| | |
|---|---|
| **P lac:** | 4992-5113 bp |
| **pel B leader**: | 13-78 |
| **CAB 1 scFv:** | 79-810 |
| **BLA**: | 811-1896 |
| **T7 term.:** | 2076-2122 |
| **CAT:** | 3253-3912 |

A schematic of plasmid pME27.1 can be found in Figure 10A. The CAB1 sequence, indicating heavy and light chain domains, can be found in Figure 10B; the amino acid sequence can also be found in 10D, with linker and BLA.

### B. Choosing mutations for mutagenesis

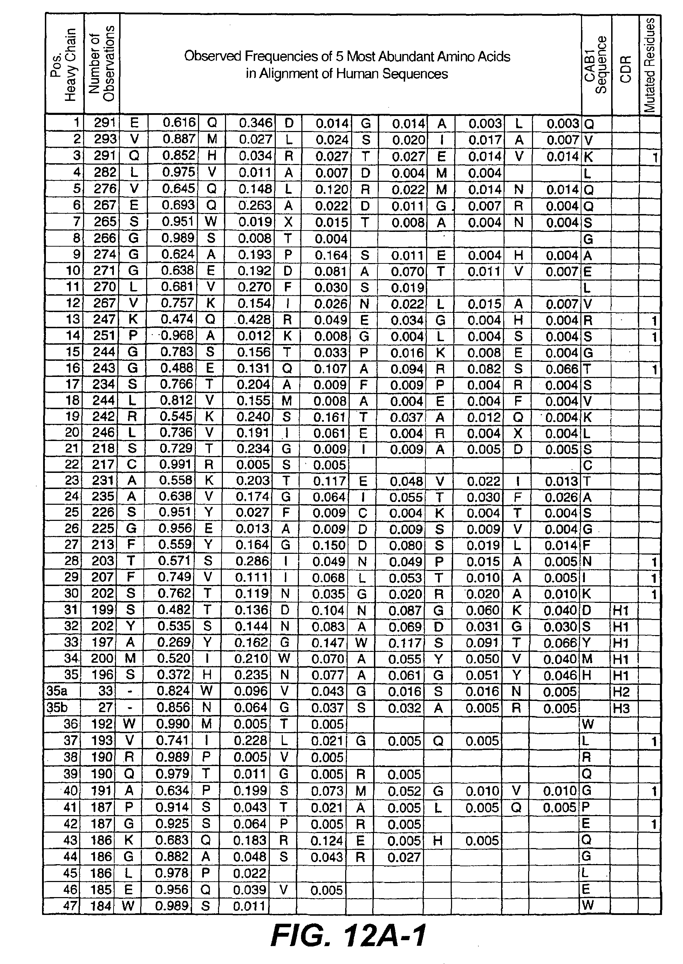

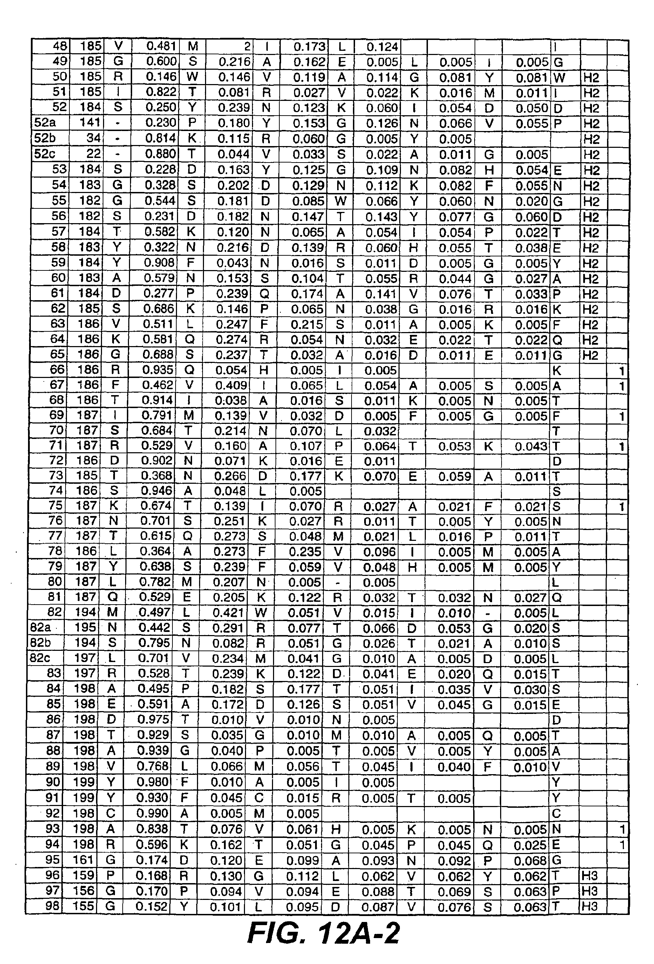

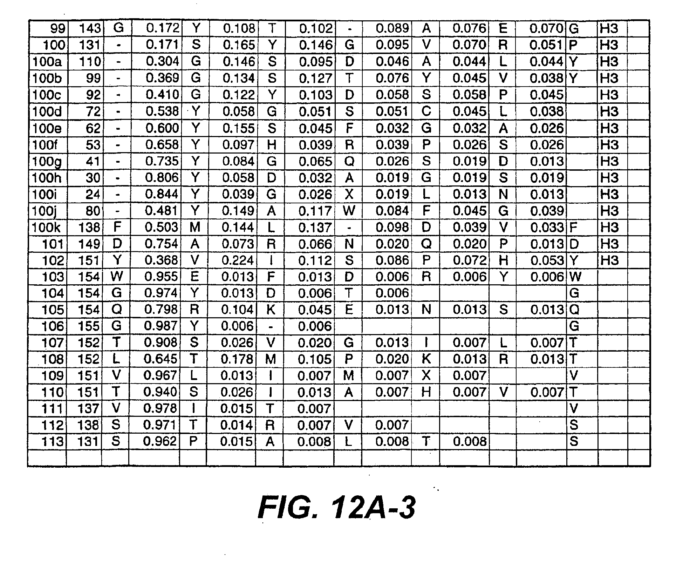

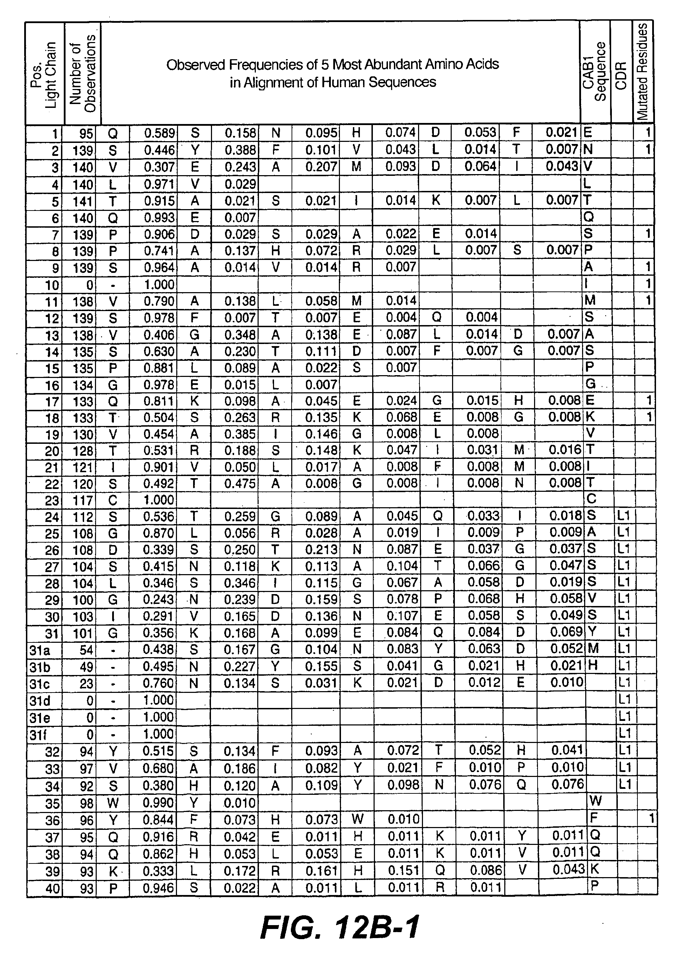

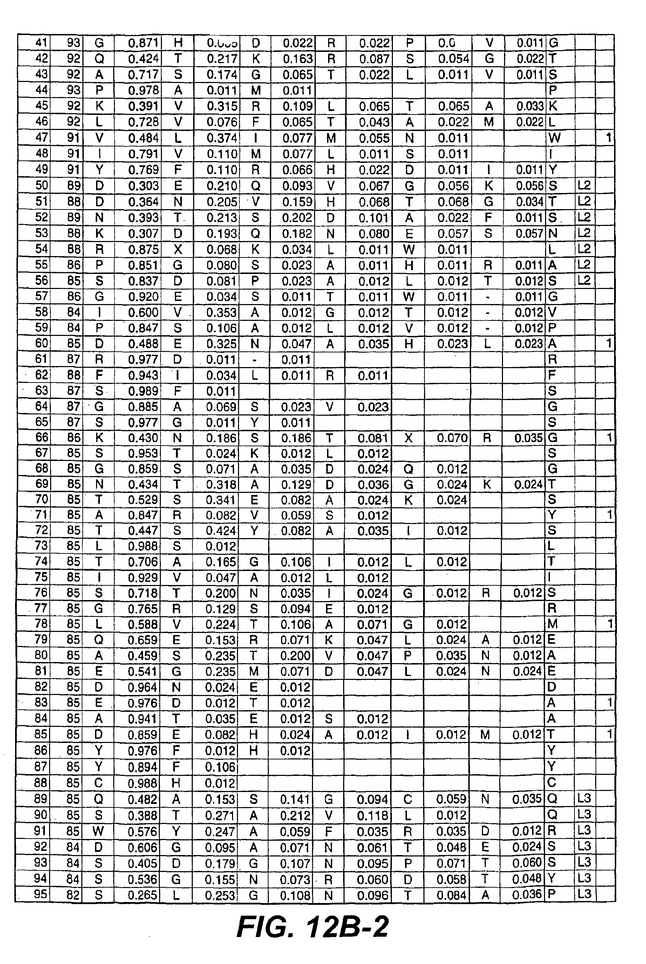

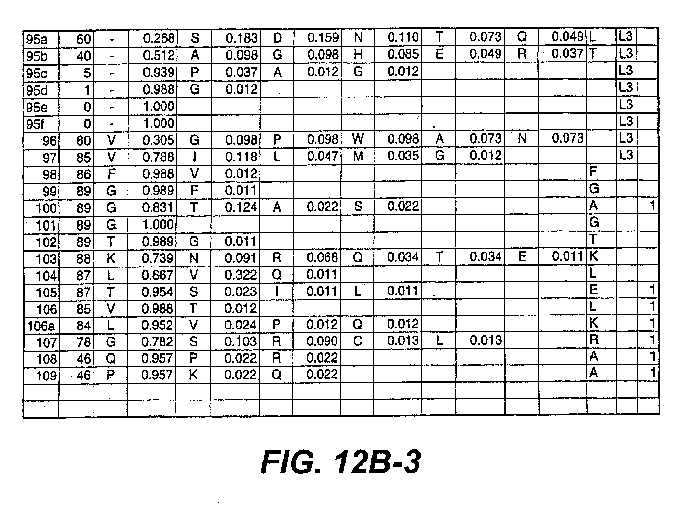

The sequence of the vH and vL sequences of CAB1-scFv were compared with a published frequency analysis of human antibodies (Boris Steipe (1998) Sequenzdatenanalyse. ("Sequence Data Analysis", available in German only) in Bioanalytik eds. H. Zorbas und F. Lottspeich, Spektrum Akademischer Verlag. S. 233-241). The authors aligned sequences of variable segments of human antibodies as found in the Kabat data base and calculated the frequency of occurrence of each amino acid for each position. These alignment can be seen in Figure 12. Specifically, Figure 12A shows an alignment of the observed frequencies of the five most abundant amino acids in alignment of human sequences in the heavy chain. Figure 12B shows an alignment of the observed frequencies of the five most abundant amino acids in alignment of human sequences in the light chain.

We compared these frequencies with the actual amino acid sequence of CAB 1 and identified 33 positions that fulfilled the following criteria:
- The position is not part of a CDR as defined by the Kabat nomenclature.
- The amino acid found in CAB1-scFv is observed in the homologous position in less than 10% of human antibodies
- The position is not one of the last 6 amino acids in the light chain of scFv. The resulting 33 positions were chosen for combinatorial mutagenesis. Mutagenic oligonucleotides were synthesized for each of the 33 positions such that the targeted position would be changed from the amino acid in CAB1-scFv to the most abundant amino acid in the homologous position of a human antibody. Figure 10B shows the sequence of CAB1-scFv, the CDRs, and the mutations that were chosen for combinatorial mutagenesis.

### C. Construction of library NA05

Table 5 listing the sequences of 33 mutagenic oligonucleotides that were used to generate combinatorial library NA05:

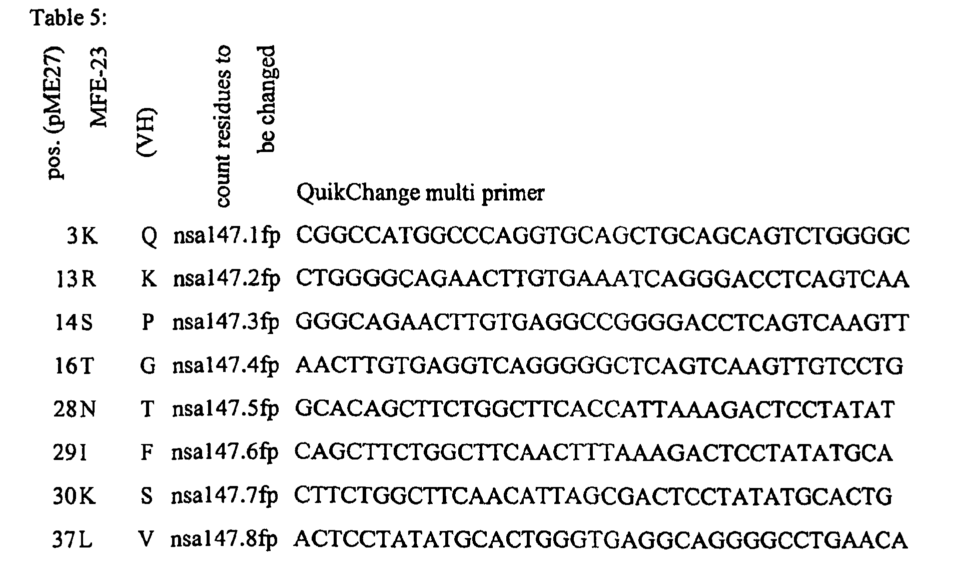

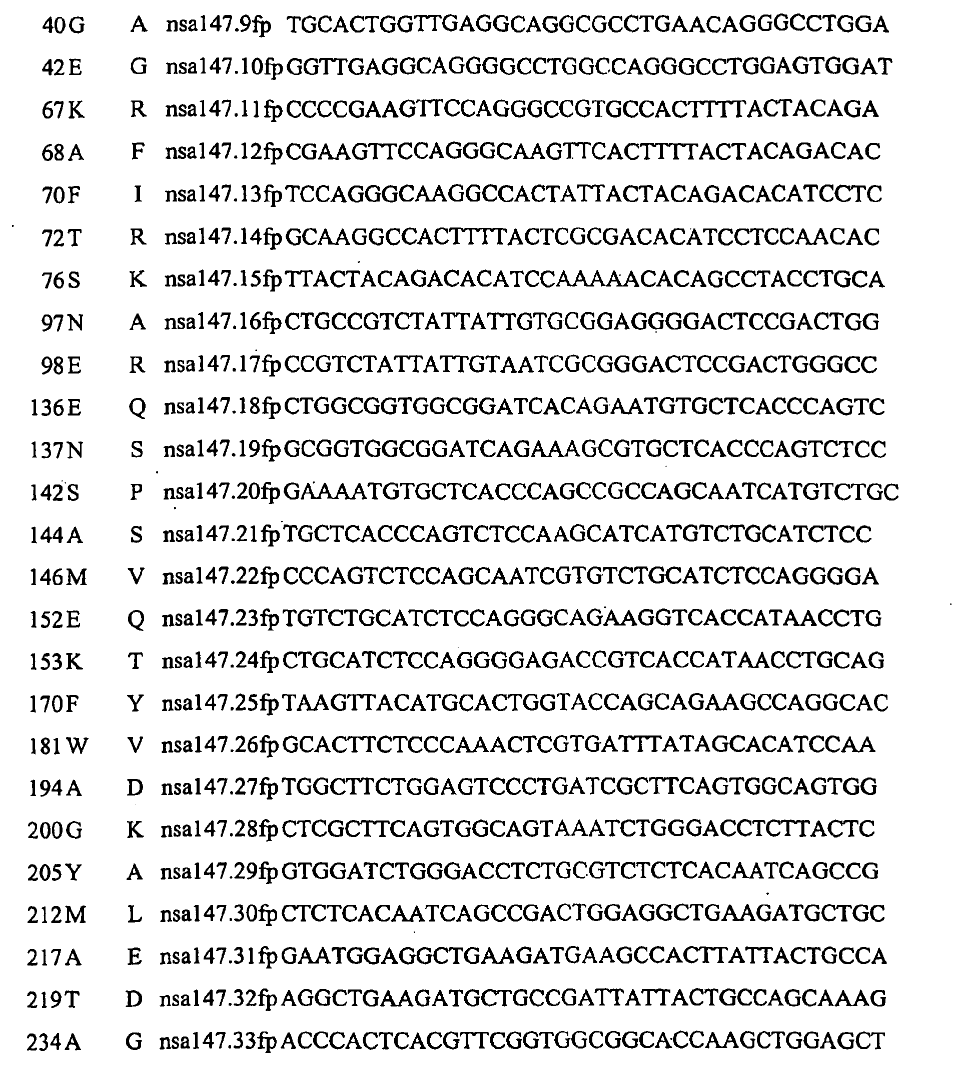

The QuikChange multi site-directed mutagenesis kit (QCMS; Stratagene Catalog # 200514) was used to construct the combinatorial library NA05 using 33 mutagenic primers. The primers were designed so that they had 17 bases flanking each side of the codon of interest based on the template plasmid pME27.1. The codon of interest was changed to encode the appropriate consensus amino acid using an *E.coli* codon usage table. All primers were designed to anneal to the same strand of the template DNA (i.e., all were forward primers in this case). The QCMS reaction was carried out as described in the QCMS manual with the exception of the primer concentration used, which ecommends using 50ng of each primer in the reaction whereas we used around 3 ng of each primer. Other primer amounts may be used. In particular, the reaction contained 50-100 ng template plasmid (pME27.1; 5178bp), 1 µl of primers mix (10 µM stock of all primers combined containing 0.3 µM each primer), 1 µl dNTPs (QCMS kit), 2.5 µl 10x QCMS reaction buffer, 18.5 µl deoinized water, and 1 µl enzyme blend (QCMS kit), for a total volume of 25 µl. The thermocycling program was 1 cycle at 95° for 1 min., followed by 30 cycles of 95°C for 1 min., 55°C for 1 min., and 65°C for 10 minutes. *Dpn*I digestion was performed by adding 1 µl *Dpn*I (provided in the QCMS kit), incubation at 37°C for 2 hours, addition of another 1 µl *Dpn*I, and incubation at 37°C for an additional 2 hours. 1 µl of the reaction was transformed into 50 µl of TOP10 electrocompetent cells from Invitrogen. 250 µl of SOC was added after electroporation, followed by a 1 hr incubation with shaking at 37°C. Thereafter, 10-50 µl of the tranformation mix was plated on LA plates with 5ppm chloramphenicol (CMP) or LA plates with 5ppm CMP and 0.1ppm of cefotaxime (CTX) for selection of active BLA clones. The active BLA clones from the CMP + CTX plates were used for screening, whereas the random library clones from the CMP plates were sequenced to assess the quality of the library.

16 randomly chosen clones were sequenced. The clones contained different combinations of 1 to 7 mutations.

### D. Screen for improved expression

We found that when TOP10/pME27.1 is cultured in LB medium at 37C then the concentration of intact fusion protein peaks after one day and most of the fusion protein is degraded by host proteases after 3 days of culture. Degradation seems to occur mainly in the scFv portion of the CAB1 fusion protein as the cultures contain significant amounts of free BLA after 3 days, which can be detected by Western blotting, or nitrocefin (Oxoid, New York) activity assay. Thus we applied a screen to library NA05 that was able to detect variants of CAB1-scFv that would resist degradation by host proteases over 3 days of culture at 37 C.

Library NA05 was plated onto agar plates with LA medium containing 5 mg/l chloramphenicol and 0.1 mg/l cefotaxime (Sigma). 910 colonies were transferred into a total of 10 96-well plates containing 100 ul/well of LA medium containing 5 mg/l chloramphenicol and 0.1 mg/l cefotaxime. Four wells in each plate were inoculated with TOP 10/pME27.1 as control and one well per plate was left as a blank. The plates were grown overnight at 37 C. The next day the cultures were used to inoculate fresh plates (production plates) containing 100 ul of the same medium using a transfer stamping tool and glycerol was added to the master plates which were stored at -70 C. The production plates were incubated in a humidified shaker at 37C for 3 days. 100 ul of BPER (Pierce, Rockford, IL) per well was added to the production plate to release protein from the cells. The production plate was diluted 100-fold in PBST (PBS containing 0.125% Tween-20) and BLA activity was , measured by transferring 20 ul diluted lysate into 180 ul of nitrocephin assay buffer (0.1 mg/ml nitrocephin in 50 mM PBS buffer containing 0.125% octylglucopyranoside (Sigma)) and the BLA activity was determined at 490 nm using a Spectramax plus plate reader (Molecular Devices, Sunnyvale, CA).

Binding to CEA (carcinoembryonic antigen, Biodesign Intl., Saco, Maine) was measured using the following procedure: 96-swell plates were coated with 100 ul per well of 5 ug/ml of CEA in 50 mM carbonate buffer pH 9.6 overnight. The plates were washed with PBST and blocked for 1-2 hours with 300 ul of casein (Pierce, Rockford, IL). 100 ul of sample from the production plate diluted 100-1000 fold was added to the CEA coated plate and the plates were incubated for 2 h at room temperature. Subsequently, the plates were washed four times with PBST and 200 ul nitrocefin assay buffer was added, and the BLA activity was measured as described above.

The BLA activity that was determined by the CEA-binding assay and the total BLA activity found in the lysate plates were compared and variants were identified which showed high levels of total BLA activity and high levels of CEA-binding activities.

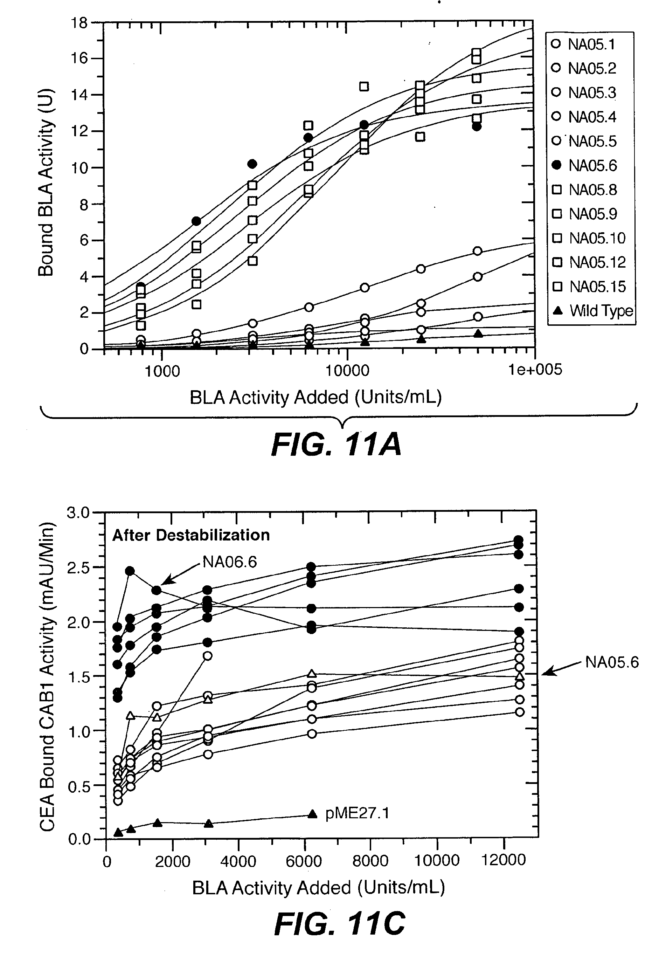

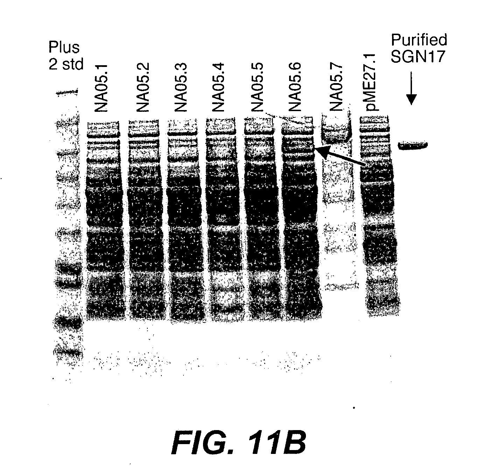

The winners were confirmed in 4 replicates using a similar protocol: the winners were cultured in 2 ml of LB containing 5 mg/l chloramphenicol and 0.1 mg/l cefotaxime for 3 days. Protein was released from the cells using BPER reagent. The binding assay was performed as described above but different dilutions of culture lysate were tested for each variant. Thus one can generate a binding curve which provides a measure of the binding affinity of the variant for the target CEA. Fig. 11A shows binding curves. Culture supernatants were also analyzed by SDS polyacrylamid electrophoresis. Fig. 11B shows the electropherogram of 7 variants from NA05. The band of the fusion protein is labeled for variant NA05.6. Table 6 shows a ranking of 6 variants. The data clearly show that NA05.6 produces significantly larger quantities of fusion protein compared to the fusion construct pME27.1.

**Table 6 showing the sequence of 6 variants with the largest improvement in stability:**

| **clone** | **mutations** |
|---|---|
| NA05.6 | R13K, T16G, W181V |
| NA05.8 | R13K, F170Y, A234G |
| NA05.9 | K3Q, S14P, L37V, E42G, E136Q, M146V, W181V, A234G |
| NA05.10 | K3Q, L37V, P170Y, W181V |
| NA05.12 | K3Q, S14P, L37V, M146V |
| NA05.15 | M146V, F170Y, A194D |

### E. Construction of library NA06

Clone NA05.6 was chosen as the best variant and was used as the template for a second round of combinatorial mutagenesis. We used a subset of the same mutagenic primers that had been used to generate library NA05 to generate combinatorial variants with the following mutations: K3Q, L37V, E42G, E136Q, M146V, F170Y, A194D, A234G which had been identified in other winners from library NA05. We did not use the primer encoding mutation S 14P as its sequence overlapped with mutations R13K and T16G that are present in NA05.6. A combinatorial library was constructed using QuikChange Multisite as described above and was called NA06. Template was pNA05.6 and 1 µl of primers mix (10 µM stock of all primers combined containing 1.25 µM each primer) were used.

### F. Screening of library NA06

The screen was performed as described above with the following modifications: 291 variants were screened on 3 96-well plates. 10 µl sample from the lysate plates was added to 180 µl of 10 µg/ml thermolysin (Sigma) in 50 mM imidazole buffer pH 7.0 containing 0.005% Tween-20 and 10 mM calcium chloride. This mixture was incubated for 1 h at 37C to hydrolyze unstable variants of NA05.6. This protease-treated sample was used to perform the CEA-binding assay as described above.
Promising variants were cultured in 2 ml medium as described above and binding curves were obtained for samples after thermolysin treatments. Figure 11C shows binding curves for selected clones. It can be seen that a number of variants retain much more binding activity after thermolysin incubation than the parent NA05.6.

**Table 7 showing 6 variants which are significantly more protease resistant than NA05.6:**

| **Clone** | **mutations** |
|---|---|
| NA06.2 | R13K, T16G, W181V, L37V, E42G, A194D |
| NA06.4 | R13K, T16G, W181V, L37V, M146V |
| NA06.6 | R13K, T16G, W181V, L37V, M146V, K3Q |
| NA06.10 | R13K, T16G, W181V, L37V, M146V, A194D |
| NA06.11 | R13K, T16G, W181V, L37V, K3Q, A194D |
| NA06.12 | R13K, T16G, W181V, L37V, E136Q |

All 6 variants have the mutation L37V which was rare in randomly chosen clones from the same library. Further testing showed that variant NA06.6 had the highest level of total BLA activity and the highest protease resistance of all variants.

### Example 4: Generation of an scFV that has pH-dependent binding

### A. Choosing positions for mutagenesis

The 3D structure of the scFv portion of NA06.6 was modeled based on the published crystal structure of a close homologue, MFE-23 [Boehm, M. K., A. L. Corper, T. Wan, M. K. Sohi, B. J. Sutton, J. D. Thornton, P. A. Keep, K. A. Chester, R. H. Begent and S. J. Perkins (2000) Biochem J346 Pt 2, 519-28, Crystal structure of the anti-(carcinoembryonic antigen) single-chain Fv antibody MFE-23 and a model for antigen binding based on intermolecular contacts] using the software package MOE (Chemical Computing Group, Montreal, Canada) and using default parameters. A space filling model of the structure was visually inspected. Side chains in the CDRs were ranked as follows: 0 = burried; 1= partially exposed; 2=completely exposed. Side chain distance to CDR3 was ranked as: 0= side chain is in CDR3; 1= side chain is one amino acid away from CDR3; 2=side chain is two amino acids away from CDR3. In a few cases, residues flanking the CDRs were included if they fit the distance and exposure criteria.

Based on this ranking, the following side chains were targeted for mutagenesis:
a) exposure = 2 and distance = 2 or smaller
b) exposure = 1 and distance <2
   40 positions in the CDRs matched these criteria.

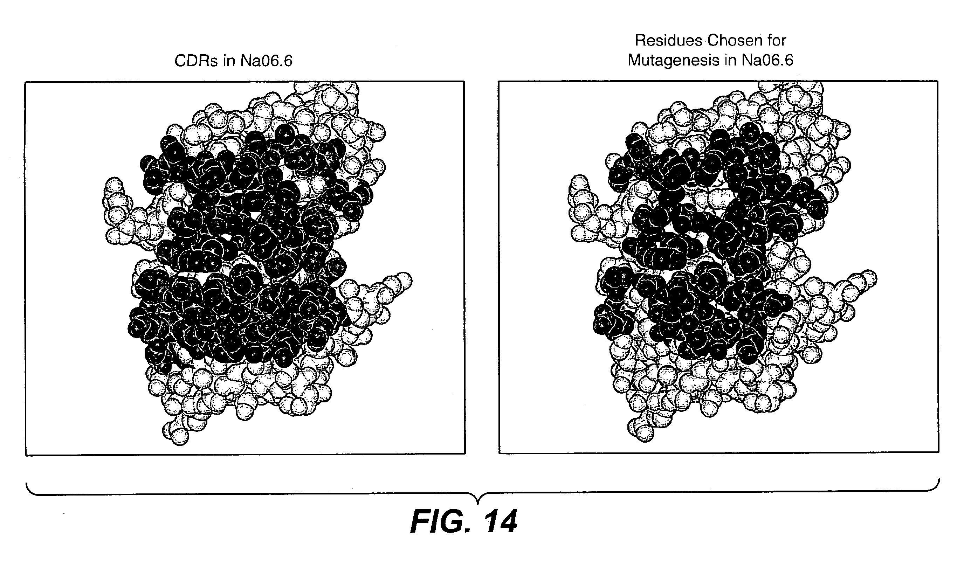

   Fig. 14 shows the CDRs and the residues that were chosen for mutagenesis.

The table below shows the criteria and position of the 40 sites that were chosen for mutagenesis.

### B. Construction of library NA08

A combinatorial library was constructed where the 40 selected positions were randomly replaced with aspartate or histidine. The substitutions were chosen as it has been reported that ionic interactions between histidine side chains and carboxyl groups form the structural basis for the pH-dependence of the interaction between IgG molecules and the Fc receptor [Vaughn, D. E. and P. J. Bjorkman (1998) Structure 6, 63-73., Structural basis of pH-dependent antibody binding by the neonatal Fc receptor].

The QuikChange multi site-directed mutagenesis kit (QCMS; Stratagene Catalog # 200514) was used to construct the combinatorial library NA08 using 40 mutagenic primers. The primers were designed so that they had 17 bases flanking each side of the codon of interest based on the template plasmid NA06.6. The codon of interest was changed to the degenerate codon SAT to encode for aspartate and histidine. All primers were designed to anneal to the same strand of the template DNA (i.e., all were forward primers in this case). The QCMS reaction was carried out as described in the QCMS manual with the exception of the primer concentration used; the manual recommends using 50-100ng of each primer in the reaction, whereas significantly lower amounts of each primer were used in this library as this results in a lower parent template background. In particular, 0.4µM of all primers together were used. The individual degenerate primer concentration in the final reaction was 0.01 µM (approximately 2.5ng).

The QCMS reaction contained 50-100 ng template plasmid (NA06.6, 5178bp), 1 µl of primers mix (10µM stock of all primers to give the desired primer concentration mentioned above), 1 µl dNTPs (QCMS kit), 2.5 µl 10x QCMS reaction buffer, 18.5 µl deoinized water, and 1 µl enzyme blend (QCMS kit), for a total volume of 25 µl. The thermocycling program was 1 cycle at 95° for 1 min., followed by 30 cycles of 95°C for 1 min., 55°C for 1 min., and 65°C for 10 minutes. *Dpn*I digestion was performed by adding 1 µl *Dpn*I (provided in the QCMS kit), incubation at 37°C for 2 hours, addition of 0.5 µl *Dpn*I, and incubation at 37°C for an additional 2 hours. 1 µl of each reaction was transformed into 50 µl of TOP10 electrocompetent cells from Invitrogen. 250 µl of SOC was added after electroporation, followed by a 1 hr incubation with shaking at 37°C. Thereafter, 10-50 µl of the transformation mix was plated on LA plates with 5ppm chloramphenicol (CMP) or LA plates with 5ppm CMP and 0.1ppm of cefotaxime (CTX) for selection of active BLA clones. The number of colonies obtained on both types of plates was comparable (652 on the CMP plate and 596 colonies on the CMP + CTX plate for 10 µl of the transformation mix plated). Active BLA clones from the CMP + CTX plates were used for screening, whereas random library clones from the CMP plates were sequenced to assess the quality of the library.

Primers for the reaction are shown in Table 8.

**Table 8 Primers for CDRs:**

| **distance to** | | | | |
|---|---|---|---|---|
| **residue** | **CDRs** | **position exposure** | **CDR3** | **primer sequence** |
| K | | 30 | 2 | 2 cttctggcttcaacattsatgactcctatatgcactg |
| D | H1 | 31 | 2 | 1 ctggcttcaacattaaasattcctatatgcactgggt |
| S | H1 | 32 | 1 | 1 gcttcaacattaaagacsattatatgcactgggtgag |
| Y | H1 | 33 | 2 | 1 tcaacattaaagactccsatatgcactgggtgaggca |
| H | H1 | 35 | 1 | 1 ttaaagactcctatatgsattgggtgaggcaggggcc |
| W | H2 | 50 | 2 | 1 gcctggagtggattggasatattgatcctgagaatgg |
| D | H2 | 52 | 2 | 2 agtggattggatggattsatcctgagaatggtgatac |
| E | H2 | 54 | 2 | 2 ttggatggattgatcctsataatggtgatactgaata |
| N | H2 | 55 | 2 | 2 gatggattgatcctgagsatggtgatactgaatatgc |
| D | H2 | 57 | 2 | 1 ttgatcctgagaatggtsatactgaatatgccccgaa |
| T | H2 | 58 | 1 | 1 atcctgagaatggtgatsatgaatatgccccgaagtt |
| E | H2 | 59 | 2 | 1 ctgagaatggtgatactsattatgccccgaagttcca |
| P | H2 | 62 | 2 | 1 gtgatactgaatatgccsataagttccagggcaaggc |
| K | H2 | 63 | 2 | 3 atactgaatatgccccgsatttccagggcaaggccac |
| Q | H2 | 65 | 2 | 2 aatatgccccgaagttcsatggcaaggccacttttac |
| E | | 98 | 1 | 0 ccgtctattattgtaatsatgggactccgactgggcc |
| G | | 99 | 1 | 0 tctattattgtaatgagsatactccgactgggccgta |
| T | H3 | 100 | 2 | 0 attattgtaatgaggggsatccgactgggccgtacta |
| P | H3 | 101 | 2 | 0 attgtaatgaggggactsatactgggccgtactactt |
| T | H3 | 102 | 2 | 0 gtaatgaggggactccgsatgggccgtactactttga |
| G | H3 | 103 | 2 | 0 atgaggggactccgactsatccgtactactttgacta |
| P | H3 | 104 | 2 | 0 aggggactccgactgggsattactactttgactactg |
| Y | H3 | 106 | 2 | 0 ctccgactgggccgtacsattttgactactggggcca |
| S | L1 | 162 | 2 | 2 taacctgcagtgccagcsatagtgtaagttacatgca |
| S | L1 | 163 | 2 | 1 cctgcagtgccagctcasatgtaagttacatgcactg |
| V | L1 | 164 | 1 | 1 gcagtgccagctcaagtsatagttacatgcactggtt |
| S | L1 | 165 | 2 | 1 gtgccagctcaagtgtasattacatgcactggttcca |
| Y | L1 | 166 | 2 | 1 ccagctcaagtgtaagtsatatgcactggttccagca |
| Y | | 183 | 1 | 0 ctcccaaactcgtgattsatagcacatccaacctggc |
| S | L2 | 184 | 2 | 0 ccaaactcgtgatttatsatacatccaacctggcttc |
| T | L2 | 185 | 1 | 1 aactcgtgatttatagcsattccaacctggcttctgg |
| S | L2 | 186 | 2 | 2 tcgtgatttatagcacasataacctggcttctggagt |
| N | L2 | 187 | 2 | 1 tgatttatagcacatccsatctggcttctggagtccc |
| A | L2 | 189 | 1 | 1 atagcacatccaacctgsattctggagtccctgctcg |
| S | L2 | 190 | 2 | 1 gcacatccaacctggctsatggagtccctgctcgctt |
| R | L3 | 225 | 2 | 2 cttattactgccagcaasattctagttacccactcac |
| S | L3 | 226 | 2 | 2 attactgccagcaaagasatagttacccactcacgt |
| S | L3 | 227 | 1 | 2 actgccagcaaagatctsattacccactcacgttcg |
| Y | L3 | 228 | 1 | 2 gccagcaaagatctagtsatccactcacgttcggtg |
| L | L3 | 230 | 1 | 2 aaagatctagttacccasatacgttcggtgctggcac |

### C. Sequencing of variants

Variants were grown overnight with shaking at 37°C in 5mL cultures of LA containing 5ppm of CMP. Miniprep DNA was prepared using a Qiagen kit and the BLA gene within each clone was sequenced using the M13 reverse and nsa154f primers.
M13 reverse: CAGGAAACAGCTATGAC
nsa154f: GGACCACGGTCACCGTCTCCTC

### D. Screen pH-dependent binding

Library NA08 was plated onto agar plates with LA medium containing 5 mg/l chloramphenicol and 0.1 mg/l cefotaxime (Sigma). 552 colonies were transferred into a total of six 96-well plates containing 100 ul/well of LA medium containing 5 mg/l chloramphenicol and 0.1 mg/l cefotaxime. Four wells in each plate were inoculated with TOP10/NA06.6 as a reference. The plates were grown overnight at 37 C. The next day the cultures were used to inoculate fresh plates (production plates) containing 100 ul of the same medium using a transfer stamping tool and glycerol was added to the master plates which were stored at -70 C. The production plates were incubated in a humidified shaker at 37C for 2 days. 100 ul of BPER (Pierce, Rockford, IL) per well was added to the production plates to release protein from the cells. The production plates were diluted 100-fold in PBST (PBS containing 0.125% Tween-20) and BLA activity was measured by transferring 20 ul diluted lysate into 180 ul of nitrocefin assay buffer (0.1 mg/ml nitrocefin in 50 mM PBS buffer containing 0.125% octylglucopyranoside (Sigma)) and the BLA activity was determined at 490 nm using a Spectramax plus plate reader (Molecular Devices, Sunnyvale, CA).

Binding to CEA (carcinoembryonic antigen, Biodesign Intl., Saco, Maine) was measured using the following procedure: 96-well plates were coated with 100 ul per well of 5 ug/ml of CEA in 50 mM carbonate buffer pH 9.6 overnight. The plates were washed with PBST and blocked for 1-2 hours with 300 ul of casein (Pierce, Rockford, IL). 100 ul of sample from the production plate diluted 100-1000 fold was added to the CEA coated plate and the plates were incubated for 2 h at room temperature. Subsequently, the plates were washed four times with PBST and 200 ul nitrocefin assay buffer was added, and the BLA activity was measured as described above. CEA binding was measured in 50 mM phosphate buffer pH 6.5 and in a separate experiment in 50 mM phosphate buffer pH 7.4.

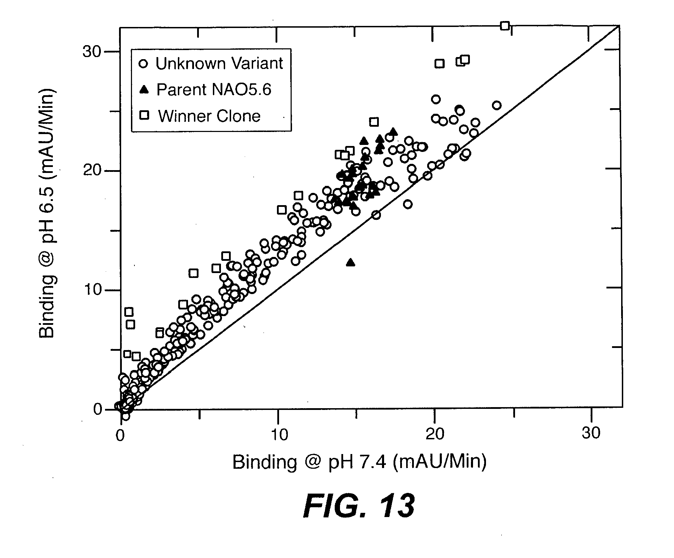

The BLA activity that was determined by the CEA-binding assay at pHs of 6.5 and 7.4, and the total BLA activity found in the lysate plates were compared and variants were identified which showed good binding to CEA at pH 6.5 but significantly weaker binding at pH 6.5. A comparison of the binding at pH6.5 versus pH 7.4 is shown in Fig. 13.

Winners were confirmed by culturing them in 5 ml of LB medium containing 5 mg/l chloramphenicol and 0.1 mg/l cefotaxime (Sigma) for 2 days at 37 C. Subsequently, the cultures were centrifuged and the pellet was suspended in 375 ul of BPER reagent to release the fusion protein. The BLA activity in each sample was determined by transferring 20 ul of the appropriately diluted sample to 180 ul of 180 ul of nitrocefin assay buffer (0.1 mg/ml nitrocefin in 50 mM PBS buffer containing 0.125% octylglucopyranoside (Sigma)) and the absorbance at 490 nm was monitored. One unit of activity was defined as the amount of BLA that leads to an absorbance increase of one mOD per minute. The samples were diluted based on their total content of BLA activity and the CEA-binding assay was performed as described above but adding various sample dilutions to each well.

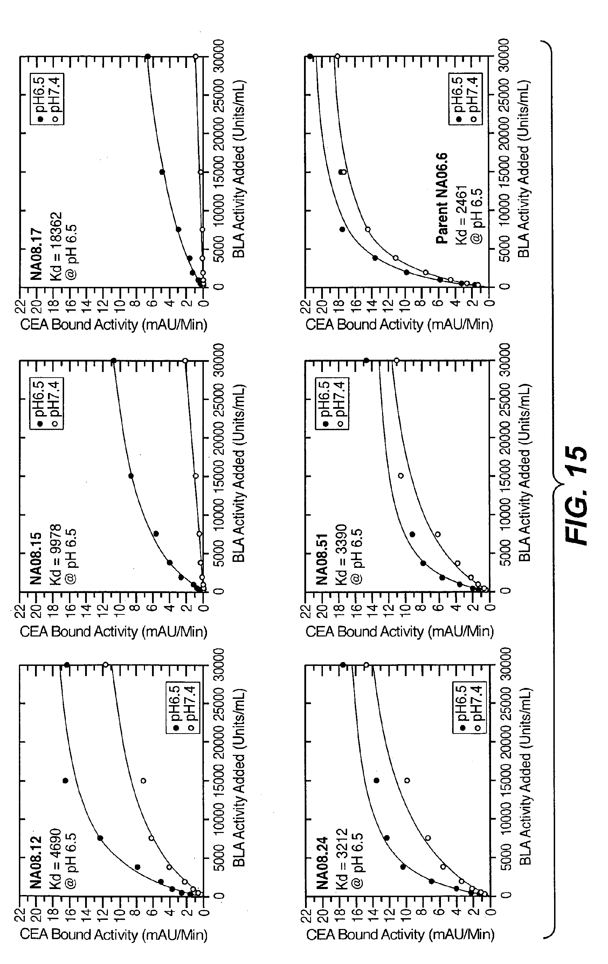

Thus, one can obtain binding curves for each sample that reflect the affinity of the variants to CEA. Figure 15 shows CEA-binding curves measured at pH 7.4 and pH 6.5 for several variants of interest. All 5 variants show increased pH-dependence of CEA binding. Whereas, the parent NA06.6 binds only slightly better at pH 6.5 compared to pH 7.4, some of the variant show much stronger binding to CEA at pH 6.5 compared to pH 7.4. Of particular interest are variants NA08.15 and NA08.17 which show very weak binding to CEA at pH 7.4 but significant binding at pH 6.5.

Table 9, below, shows variants with the greatest binding improvement.

**Table 9:**

| **clone** | **mutations** |
|---|---|
| NA08.1 | W50H, Y166D |
| NA08.3 | S190D, S226D |
| NA08.4 | S190D, T100D |
| NA08.9 | Y166D |
| NA08.12 | T102H, Y166D, S226D |
| NA08.13 | Q65H, S 184D, S226D |
| NA08.14 | P101D |
| NA08.15 | S184D, S226D |
| NA08.17 | S184D, W50H |
| NA08.24 | T102D, S226D |
| NA08.45 | T102D, Y166D |
| NA08.51 | P104H, Y166D |
| NA08.64 | Q65D, Y166D |

### Example 5: purification of ME27.1

Purification of ME27.1 from cell extract was done using Cation Exchange Chromatography. This was performed with the aid of a high performance liquid chromatographic system (AKTA™explorer 10, Amersham Biosciences) on a 7.3mL CM Ceramic HyperF cation exchange column (Ciphergen Biosystems). The column was first equilibrated with loading/equilibration buffer. The prepared ME27.1 extract was applied to the column at 300cm/hr, followed by washing with the equilibration buffer. The bound proteins were eluted using a sodium chloride gradient. The eluted fractions were analyzed using colorimetric activity assay (o-nitrocefan as substrate) and 4-12% Bis-Tris SDS-PAGE reducing gel with MES running buffer (Novex).

### Buffers

The following buffers were used for the purification using Cation Exchange Chromatography:
Loading/Equilibration: 50mM Sodium Acetate, pH 5
Elution: 50mM Sodium Acetate containing 1M Sodium Chloride, pH 5
Regeneration: 0.5M Sodium Hydroxide and 1M Sodium Acetate, pH 4

### a) Extract Sample Preparation

E. coli cells containing gene of interest were cultured in TB broth. The production media flasks were incubated at 30° C, 150-200 rpm for 18-24 hours. After fermentation, the E. coli cells were centrifuged at 4,000 rpm for 30 minutes. The supernatant was discarded and BPER detergent (Pierce product #78266) was added to the pellet to lyse the cells (27 mL of B-PER per gram of cell pellet). The lysed cells were centrifuged at 18,000 rpm for 20 minutes to remove cell debris. The supernatant containing the *ME27.1,* referred to as the "extract", was used for the subsequent purification experiments. The extract was stored at 4°C until use for subsequent purification experiments.

The following sample pretreatment steps were followed to prepare the extract for subsequent chromatography purification experiments.

Dilute ME27.1 extract (conductivity 9.5mS/cm and pH 7.3) with 1 part of equilibration buffer (see below). pH of the diluted extract was 6.51.
Adjust pH to 5.0 using ∼20% acetic acid.
Filter pH adjusted extract through 0.2µm filter unit with ∼1% diatomaceous earth as admix.

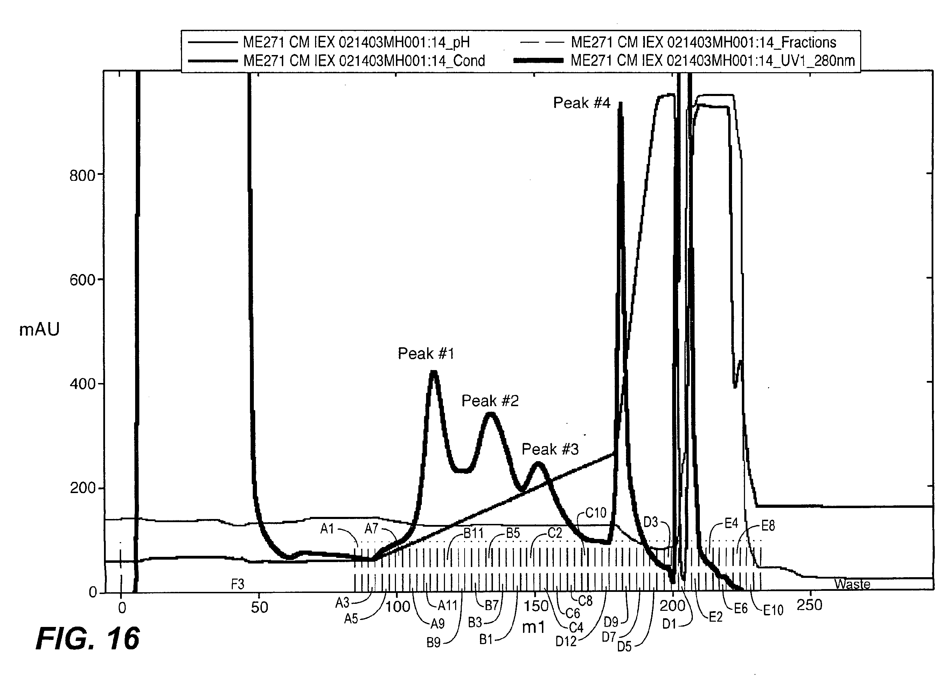

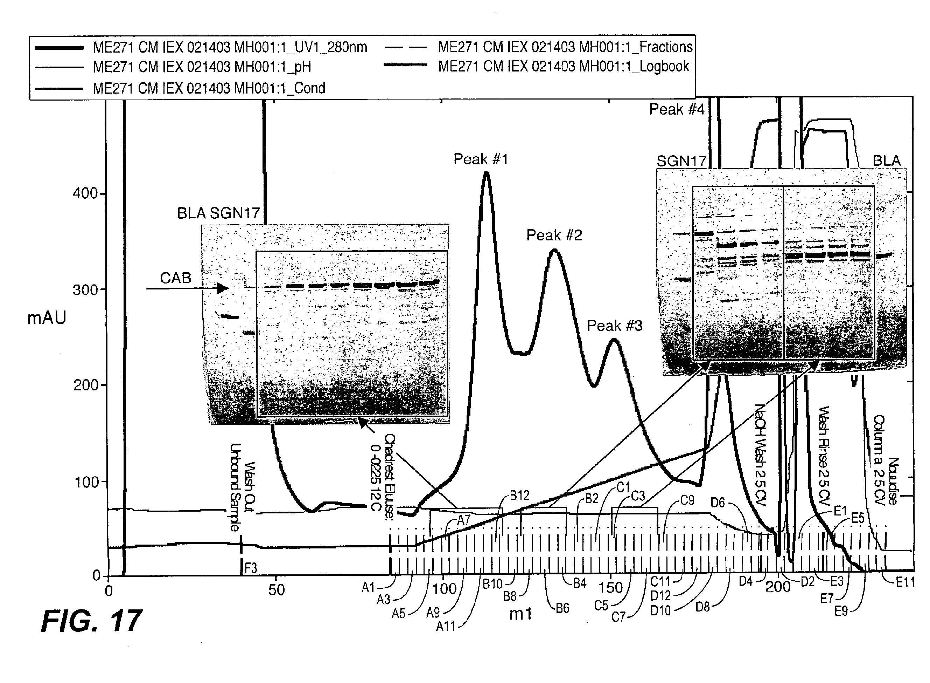

Figure 16 shows the overall the chromatogram obtained using the 18 hours old extract. The fractions from peak #1 constitute 23% of the total eluted activity, with the major protein band near molecular weight (MW) equivalent to the *ME27.1* MW (see Figure 17, gel on left). Peaks #2, #3 and #4 constitute 76.6% of the total eluted activity but SDS-PAGE gel (see Figure 17, gel on right) shows that these fractions contains relatively small amount of protein near *ME27.1* MW. There was an increasing amount protein bands at MW below *ME27.1* MW.

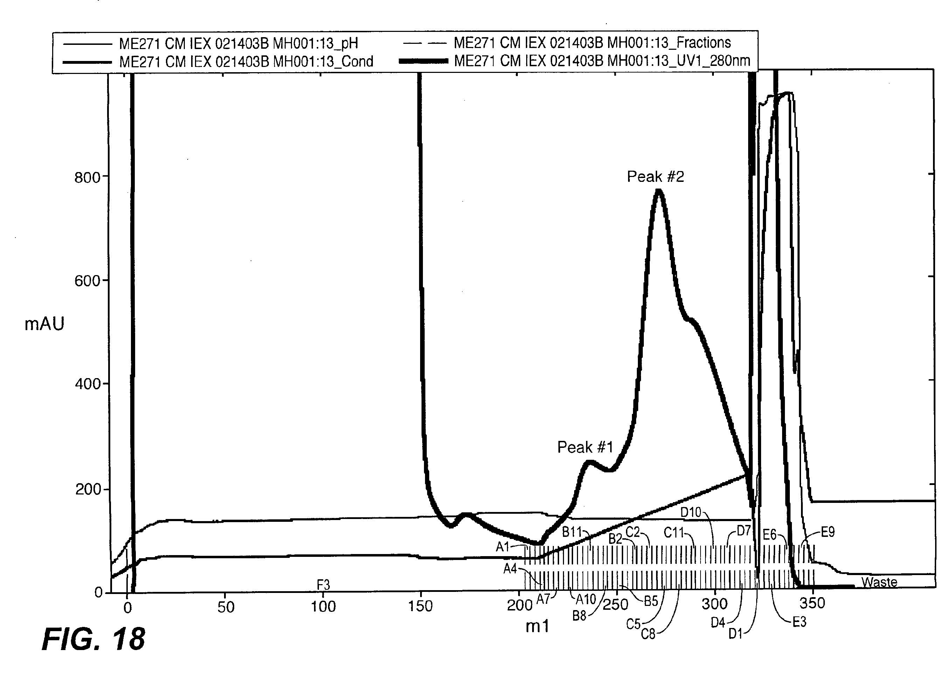

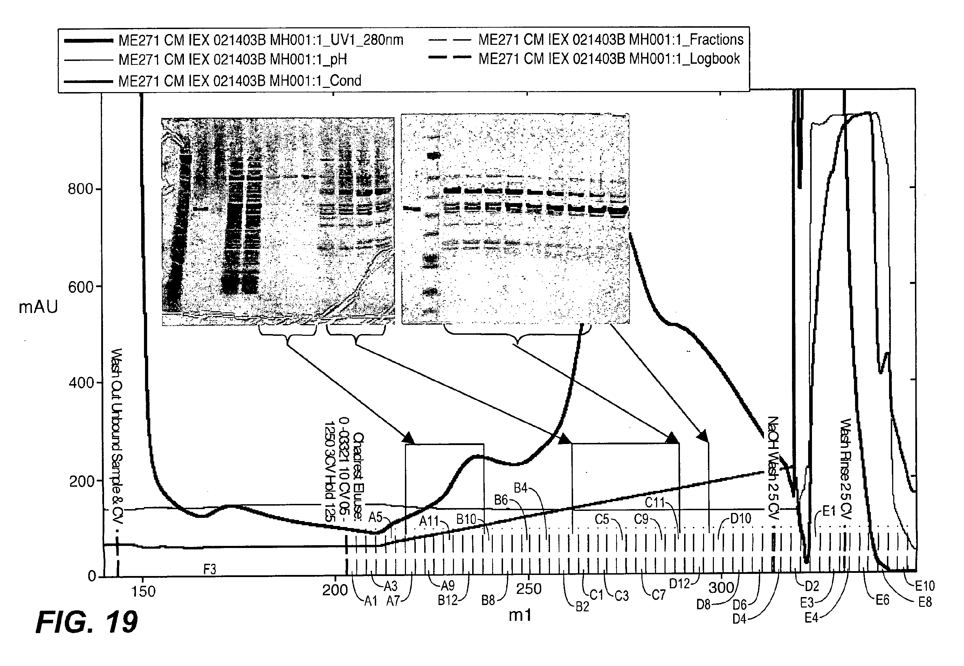

Figure 18 shows the overall chromatogram obtained using 26 hours old extract. The chromatogram is significantly different from Figure 16. The relative proportion of peak #1 to peak #2 has decreased significantly for the 26 hours extract compared to the 18 hour extract. SDS-PAGE gels shows that the small peak #1 contains mostly protein near *ME27.1* MW but the remainder fractions (from peak #2 and onwards) contain mostly protein bands with MW lower that *ME27.1* (see Figure 19).

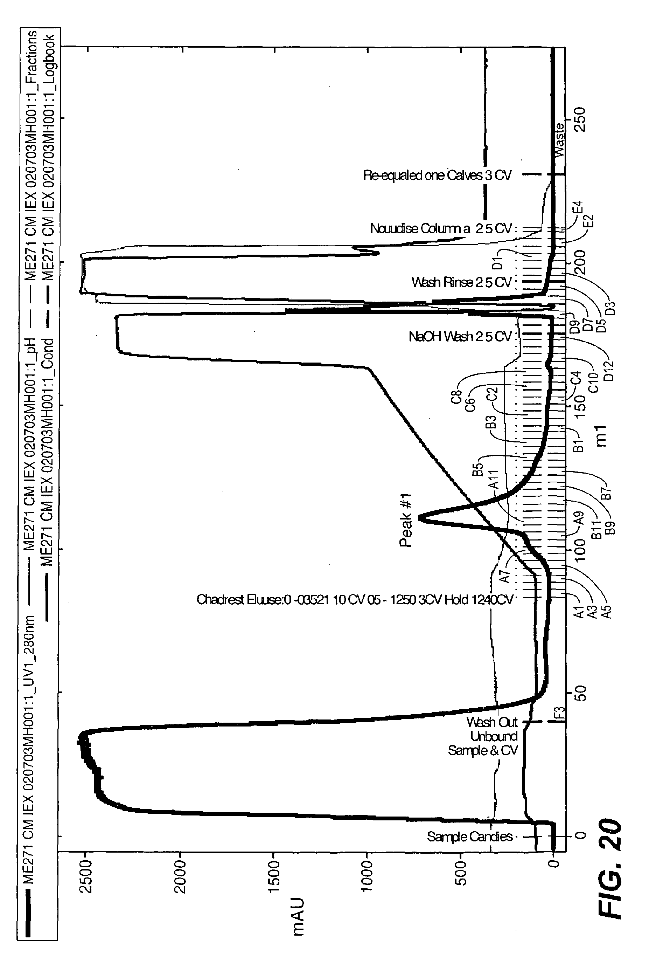

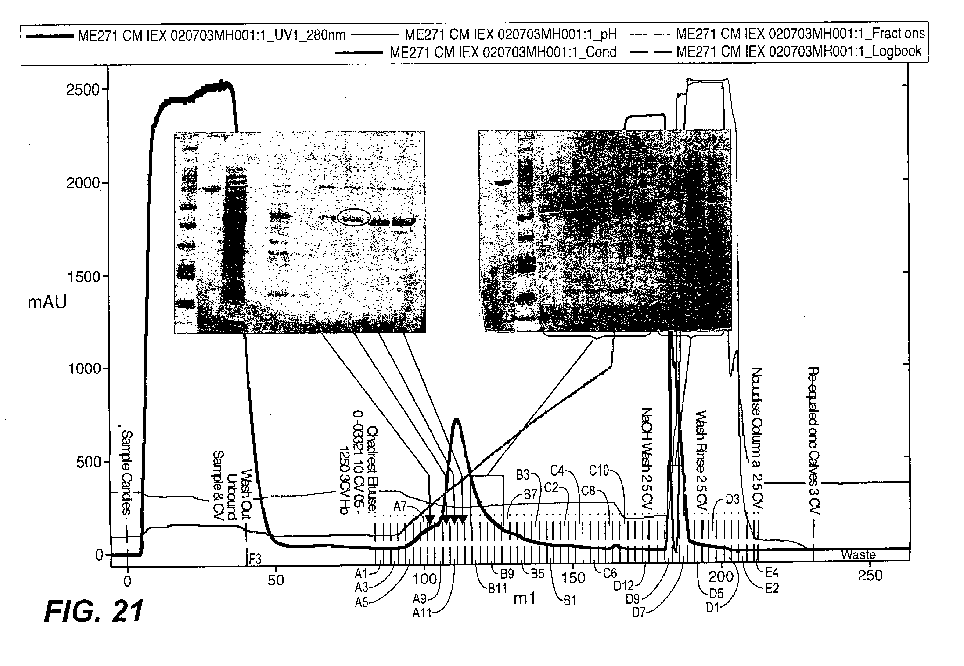

Figure 20 shows the overall chromatogram obtained using » 26 hours old (4-5days) extract. The chromatogram is significantly different from Figure 16 and Figure 18. The distinct peak #1 found in 18 hrs (Figure 16) and 26 hours (Figure 18) old extract has collapsed into a shoulder of equivalent peak #2 found in Figure 16 and Figure 18. SDS-PAGE gels shows that the shoulder contains two main bands, one near the ME27.1 MW and the other at lower MW. The main peak #1 fractions contain 88% of the activity eluted, and yet the main protein band is below the MW of *ME27.1.* Mass spec analysis confirmed that the lower band is degraded ME27.1 (see Figure 21, left gel circled band).

Purification of *NA05.6* extract was done using Anion Exchange Chromatography follow by Affinity chromatography using aminophenylboronic acid (PBA) resin.

The anion exchange chromatography was performed with the aid of a high performance liquid chromatographic system (AKTA™explorer 10, Amersham Biosciences) on a 7mL Poros HQ anion exchange column (PE Biosystems). The column was first equilibrated with loading/equilibration buffer. The prepared *NA05.6* extract was applied to the column at 300cm/hr. The *NA05.6* was collected as the flow through and wash fractions. These fractions were analyzed for activity using colorimetric assay (o-nitrocefan as substrate) and purity using 4-12% Bis-Tris SDS-PAGE reducing gel with MES running buffer (Novex).

The PBA step was done using a 5 mL column containing PBA resin (Sigma A-8530 m-aminophenylboronic acid resin). The column was first equilibrated with 20mL of each equilibration buffers by gravity flow. The anion exchange partially purified flow through was used as the PBA feed. The feed was applied to the column by gravity flow and wash with wash buffer. The bound sample was eluted with elution buffer. Samples were analyzed for activity using colorimetric assay (o-nitrocefan as substrate) and purity using 4-12% Bis-Tris SDS-PAGE reducing gel with MES running buffer (Novex).

### Buffers

The following buffers were used for the purification using Anion Exchange Chromatography:
Load/ Equilibration: 50mM Tris, pH 7.4
Regeneration: 0.5M NaOH

The following buffers were used for the purification using PBA Affinity Chromatography:

Equilibration Buffers: 0.5 M sorbitol with 1 M NaCI; 0.5 M Borate at pH 7; and 20 mM TEA with 0.5 M NaCI, pH 7
Wash buffer: 20mM TEA with 0.5M NaCl
Elution Buffer: 0.5 M borate with 0.5M NaCl

### b) Extract Sample Preparation for Anion Exchange Chromatography

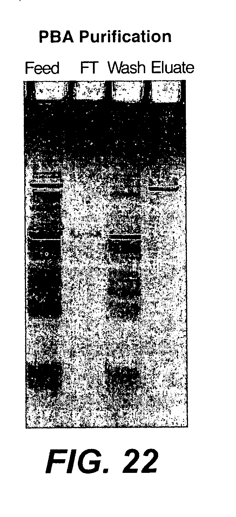

The flow through contains 79% of the activity loaded onto the anion exchange column. Subsequent purification of the flow through fraction using PBA affinity chromatography shows that all the protein eluted from contain protein near NA05.6 MW (*See* Figure 22) and 80% of the loaded activity was recovered. This indicates that all the NA05.6 molecule is intact.

Various publications are cited herein, the disclosures of which are incorporated by reference in their entireties.

## Claims

1. A method of improving the binding affinity of a binding sequence for a target comprising:
(a) making a reporter fusion by covalently linking a prototype binding sequence to a reporter sequence,
(b) modifying the binding sequence to produce a variant binding sequence,
(c) contacting the target with the reporter fusion under conditions that allow the reporter fusion to bind the target, and
(d) selecting the reporter fusion if it has an improved binding characteristic.

2. The method of claim 1 wherein the selected reporter fusion binds to the target with an affinity that is greater than the binding affinity of the prototype binding sequence for the target.

3. The method of claim 1 wherein the selected reporter fusion binds to the target with a greater specificity than the prototype binding sequence has for the target.

4. The method according to claim 1 further comprising repeating steps (a) through (d) one or more times, wherein the variant binding sequence of a reporter fusion selected in a previous step (d) is the prototype binding sequence of the subsequent step (a).

5. The method according to claim 1 further comprising removing the reporter sequence from the binding sequence of the reporter fusion selected in step (d).

6. A method of improving a binding characteristic of a binding sequence for a target comprising:
(a) contacting the target with a reporter fusion under conditions that allow the reporter fusion to bind to the target, wherein the reporter fusion comprises a reporter sequence and a variant binding sequence that is derived from a prototype binding sequence that binds the target, and
(b) selecting the reporter fusion if it has an improved binding characteristic compared to the prototype binding sequence.

7. The method of claim 1:
(a) wherein the selected reporter fusion binds to the target with an affinity that is greater than the binding affinity of the prototype binding sequence for the target; or
(b) the selected reporter fusion binds to the target with a greater specificity than the prototype binding sequence has for the target.

8. The method of claim 6, wherein step (b) comprises incubating the reporter fusion in the presence of proteases and/or under conditions which degrade or destabilize the reporter fusion.

9. The method of claim 8, wherein the conditions are at least one of heat, pH or incubation in the presence of solutes that affect stability.

10. The method of claim 8 further comprising:
(i) repeating steps (a) and (b) one or more times, wherein the binding sequence of the reporter fusion selected in a previous step (b) is the prototype binding sequence of the subsequent step (a); and/or
(ii) comprising removing the reporter sequence from the binding sequence of the reporter fusion selected in step (b).

11. A method of improving a binding characteristic of a binding sequence for a target comprising :
(a) contacting a target with a library comprising a multiplicity of reporter fusions, under conditions that allow a reporter fusion to bind the target, wherein said reporter fusions comprise a reporter sequence and a variant binding sequence derived from a prototype binding sequence that binds the target, and
(b) selecting a reporter fusion bound to the target that has an improved binding characteristic.

12. The method of claim 11 wherein the selected reporter fusion binds to the target with an affinity that is greater than the binding affinity of the prototype binding sequence for the target.

13. The method of claim 11 wherein the selected reporter fusion binds to the target with a greater specificity than the prototype binding sequence has for the target.

14. The method of claim 11 further comprising repeating steps (a) and (b) one or more times, wherein the binding sequence of the reporter fusion selected in a previous step (b) is the prototype binding sequence of the subsequent step (a).

15. The method according to claim 11 further comprising removing the reporter sequence from the binding sequence of the reporter fusion selected in step (b).


SEQUENCE LISTING

<110> Genencor International, Inc.

<120> Methods for Improving a Binding
  Characteristic of a Molecule

<130> SJK/FP6601447

<140> EPA No. TBC
<141> 2003-06-09

<150> EPA No. 03760249.7
<151> 2003-06-09

<150> PCT/US03/18187
<151> 2003-06-09

<150> US 60/388,386
<151> 2002-06-09

<160> 262

<170> FastSEQ for Windows Version 4.0

<210> 1
<211> 361
<212> PRT
<213> Artificial Sequence

<220>
<223> beta-lactamase

<400> 1
Thr Pro Val Ser Glu Lys Gln Leu Ala Glu Val Val Ala Asn Thr Ile
 1               5                  10                  15
Thr Pro Leu Met Lys Ala Gln Ser Val Pro Gly Met Ala Val Ala Val
            20                  25                  30
Ile Tyr Gln Gly Lys Pro His Tyr Tyr Thr Phe Gly Lys Ala Asp Ile
        35                  40                  45
Ala Ala Asn Lys Pro Val Thr Pro Gln Thr Leu Phe Glu Leu Gly Ser
    50                  55                  60
Ile Ser Lys Thr Phe Thr Gly Val Leu Gly Gly Asp Ala Ile Ala Arg
65                  70                  75                  80
Gly Glu Ile Ser Leu Asp Asp Ala Val Thr Arg Tyr Trp Pro Gln Leu
                85                  90                  95
Thr Gly Lys Gln Trp Gln Gly Ile Arg Met Leu Asp Leu Ala Thr Tyr
            100                 105                 110
Thr Ala Gly Gly Leu Pro Leu Gln Val Pro Asp Glu Val Thr Asp Asn
        115                 120                 125
Ala Ser Leu Leu Arg Phe Tyr Gln Asn Trp Gln Pro Gln Trp Lys Pro
    130                 135                 140
Gly Thr Thr Arg Leu Tyr Ala Asn Ala Ser Ile Leu Gly Phe Gly Ala
145                 150                 155                 160
Leu Ala Val Lys Pro Ser Gly Met Pro Tyr Glu Gln Ala Met Thr Thr
                165                 170                 175
Arg Val Leu Lys Pro Leu Lys Leu Asp His Thr Trp Ile Asn Val Pro
            180                 185                 190
Lys Ala Glu Glu Ala His Tyr Ala Trp Gly Tyr Arg Asp Gly Lys Ala

195                 200                 205
Val Arg Val Ser Pro Gly Met Leu Asp Ala Gln Ala Tyr Gly Val Lys
    210                 215                 220
Thr Asn Val Gln Asp Met Ala Asn Trp Val Met Ala Asn Met Ala Pro
225                 230                 235                 240
Glu Asn Val Ala Asp Ala Ser Leu Lys Gln Gly Ile Ala Leu Ala Gln
                245                 250                 255
Ser Arg Tyr Trp Arg Ile Gly Ser Met Tyr Gln Gly Leu Gly Trp Glu
            260                 265                 270
Met Leu Asn Trp Pro Val Glu Ala Asn Thr Val Val Glu Gly Ser Asp
        275                 280                 285
Ser Lys Val Ala Leu Ala Pro Leu Pro Val Ala Glu Val Asn Pro Pro
    290                 295                 300
Ala Pro Pro Val Lys Ala Ser Trp Val His Lys Thr Gly Ser Thr Gly
305                 310                 315                 320
Gly Phe Gly Ser Tyr Val Ala Phe Ile Pro Glu Lys Gln Ile Gly Ile
                325                 330                 335
Val Met Leu Ala Asn Thr Ser Tyr Pro Asn Pro Ala Arg Val Glu Ala
            340                 345                 350
Ala Tyr His Ile Leu Glu Ala Leu Gln
        355                 360

<210> 2
<211> 244
<212> PRT
<213> Artificial Sequence

<220>
<223> CAB1 single chain antibody

<400> 2
Gln Val Lys Leu Gln Gln Ser Gly Ala Glu Leu Val Arg Ser Gly Thr
1               5                   10                  15
Ser Val Lys Leu Ser Cys Thr Ala Ser Gly Phe Asn Ile Lys Asp Ser
            20                  25                  30
Tyr Met His Trp Leu Arg Gln Gly Pro Glu Gln Gly Leu Glu Trp Ile
        35                  40                  45
Gly Trp Ile Asp Pro Glu Asn Gly Asp Thr Glu Tyr Ala Pro Lys Phe
    50                  55                  60
Gln Gly Lys Ala Thr Phe Thr Thr Asp Thr Ser Ser Asn Thr Ala Tyr
65                  70                  75                  80
Leu Gln Leu Ser Ser Leu Thr Ser Glu Asp Thr Ala Val Tyr Tyr Cys
                85                  90                  95
Asn Glu Gly Thr Pro Thr Gly Pro Tyr Tyr Phe Asp Tyr Trp Gly Gln
            100                 105                 110
Gly Thr Thr Val Thr Val Ser Ser Gly Gly Gly Gly Ser Gly Gly Gly
        115                 120                 125
Gly Ser Gly Gly Gly Gly Ser Glu Asn Val Leu Thr Gln Ser Pro Ala
    130                 135                 140
Ile Met Ser Ala Ser Pro Gly Glu Lys Val Thr Ile Thr Cys Ser Ala
145                 150                 155                 160
Ser Ser Ser Val Ser Tyr Met His Trp Phe Gln Gln Lys Pro Gly Thr
                165                 170                 175
Ser Pro Lys Leu Trp Ile Tyr Ser Thr Ser Asn Leu Ala Ser Gly Val
            180                 185                 190
Pro Ala Arg Phe Ser Gly Ser Gly Ser Gly Thr Ser Tyr Ser Leu Thr
        195                 200                 205
Ile Ser Arg Met Glu Ala Glu Asp Ala Ala Thr Tyr Tyr Cys Gln Gln
    210                 215                 220
Arg Ser Ser Tyr Pro Leu Thr Phe Gly Ala Gly Thr Lys Leu Glu Leu
225                 230                 235                 240

Lys Arg Ala Ala

<210> 3
<211> 120
<212> PRT
<213> Artificial Sequence

<220>
<223> CAB1 heavy chain

<400> 3
Gln Val Lys Leu Gln Gln Ser Gly Ala Glu Leu Val Arg Ser Gly Thr
1               5                   10                  15
Ser Val Lys Leu Ser Cys Thr Ala Ser Gly Phe Asn Ile Lys Asp Ser
            20                  25                  30
Tyr Met His Trp Leu Arg Gln Gly Pro Glu Gln Gly Leu Glu Trp Ile
        35                  40                  45
Gly Trp Ile Asp Pro Glu Asn Gly Asp Thr Glu Tyr Ala Pro Lys Phe
    50                  55                  60
Gln Gly Lys Ala Thr Phe Thr Thr Asp Thr Ser Ser Asn Thr Ala Tyr
65                  70                  75                  80
Leu Gln Leu Ser Ser Leu Thr Ser Glu Asp Thr Ala Val Tyr Tyr Cys
                85                  90                  95
Asn Glu Gly Thr Pro Thr Gly Pro Tyr Tyr Phe Asp Tyr Trp Gly Gln
            100                 105                 110
Gly Thr Thr Val Thr Val Ser Ser
        115                 120

<210> 4
<211> 15
<212> PRT
<213> Artificial Sequence

<220>
<223> CAB1 linker

<400> 4
Gly Gly Gly Gly Ser Gly Gly Gly Gly Ser Gly Gly Gly Gly Ser
1               5                   10                  15

<210> 5
<211> 109
<212> PRT
<213> Artificial Sequence

<220>
<223> CAB1 light chain

<400> 5
Glu Asn Val Leu Thr Gln Ser Pro Ala Ile Met Ser Ala Ser Pro Gly
1               5                   10                  15
Glu Lys Val Thr Ile Thr Cys Ser Ala Ser Ser Ser Val Ser Tyr Met
            20                  25                  30
His Trp Phe Gln Gln Lys Pro Gly Thr Ser Pro Lys Leu Trp Ile Tyr
        35                  40                  45
Ser Thr Ser Asn Leu Ala Ser Gly Val Pro Ala Arg Phe Ser Gly Ser
    50                  55                  60
Gly Ser Gly Thr Ser Tyr Ser Leu Thr Ile Ser Arg Met Glu Ala Glu
65                  70                  75                  80
Asp Ala Ala Thr Tyr Tyr Cys Gln Gln Arg Ser Ser Tyr Pro Leu Thr

                85                  90                  95
Phe Gly Ala Gly Thr Lys Leu Glu Leu Lys Arg Ala Ala
            100                 105

<210> 6
<211> 360
<212> PRT
<213> Artificial Sequence

<220>
<223> beta-lactamase

<400> 6
Pro Val Ser Glu Lys Gln Leu Ala Glu Val Val Ala Asn Thr Ile Thr
 1               5                  10                  15
Pro Leu Met Lys Ala Gln Ser Val Pro Gly Met Ala Val Ala Val Ile
            20                  25                  30
Tyr Gln Gly Lys Pro His Tyr Tyr Thr Phe Gly Lys Ala Asp Ile Ala
        35                  40                  45
Ala Asn Lys Pro Val Thr Pro Gln Thr Leu Phe Glu Leu Gly Ser Ile
    50                  55                  60
Ser Lys Thr Phe Thr Gly Val Leu Gly Gly Asp Ala Ile Ala Arg Gly
65                  70                  75                  80
Glu Ile Ser Leu Asp Asp Ala Val Thr Arg Tyr Trp Pro Gln Leu Thr
                85                  90                  95
Gly Lys Gln Trp Gln Gly Ile Arg Met Leu Asp Leu Ala Thr Tyr Thr
            100                 105                 110
Ala Gly Gly Leu Pro Leu Gln Val Pro Asp Glu Val Thr Asp Asn Ala
        115                 120                 125
Ser Leu Leu Arg Phe Tyr Gln Asn Trp Gln Pro Gln Trp Lys Pro Gly
    130                 135                 140
Thr Thr Arg Leu Tyr Ala Asn Ala Ser Ile Gly Leu Phe Gly Ala Leu
145                 150                 155                 160
Ala Val Lys Pro Ser Gly Met Pro Tyr Glu Gln Ala Met Thr Thr Arg
                165                 170                 175
Val Leu Lys Pro Leu Lys Leu Asp His Thr Trp Ile Asn Val Pro Lys
            180                 185                 190
Ala Glu Glu Ala His Tyr Ala Trp Gly Tyr Arg Asp Gly Lys Ala Val
        195                 200                 205
Arg Val Ser Pro Gly Met Leu Asp Ala Gln Ala Tyr Gly Val Lys Thr
    210                 215                 220
Asn Val Gln Asp Met Ala Asn Trp Val Met Ala Asn Met Ala Pro Glu
225                 230                 235                 240
Asn Val Ala Asp Ala Ser Leu Lys Gln Gly Ile Ala Leu Ala Gln Ser
                245                 250                 255
Arg Tyr Trp Arg Ile Gly Ser Met Tyr Gln Gly Leu Gly Trp Glu Met
            260                 265                 270
Leu Asn Trp Pro Val Glu Ala Asn Thr Val Val Glu Thr Ser Phe Gly
        275                 280                 285
Asn Val Ala Leu Ala Pro Leu Pro Val Ala Glu Val Asn Pro Pro Ala
    290                 295                 300
Pro Pro Val Lys Ala Ser Trp Val His Lys Thr Gly Ser Thr Gly Gly
305                 310                 315                 320
Phe Gly Ser Tyr Val Ala Phe Ile Pro Glu Lys Gln Ile Gly Ile Val
                325                 330                 335
Met Leu Ala Asn Thr Ser Tyr Pro Asn Pro Ala Arg Val Glu Ala Ala
            340                 345                 350
Tyr His Ile Leu Glu Ala Leu Gln
        355                 360

<210> 7

<211> 5178
<212> DNA
<213> Artificial Sequence

<220>
<223> plasmid pME27.1

<400> 7
aggaattatc atatgaaata cctgctgccg accgctgctg ctggtctgct gctcctcgct 60
gcccagccgg ccatggccca ggtgaaactg cagcagtctg gggcagaact tgtgaggtca 120
gggacctcag tcaagttgtc ctgcacagct tctggcttca acattaaaga ctcctatatg 180
cactggttga ggcaggggcc tgaacagggc ctggagtgga ttggatggat tgatcctgag 240
aatggtgata ctgaatatgc cccgaagttc cagggcaagg ccacttttac tacagacaca 300
tcctccaaca cagcctacct gcagctcagc agcctgacat ctgaggacac tgccgtctat 360
tattgtaatg aggggactcc gactgggccg tactactttg actactgggg ccaaggaacc 420
acggtcaccg tctcctcagg tggaggcggt tcaggcggag gtggctctgg cggtggcgga 480
tcagaaaatg tgctcaccca gtctccagca atcatgtctg catctccagg ggagaaggtc 540
accataacct gcagtgccag ctcaagtgta agttacatgc actggttcca gcagaagcca 600
ggcacttctc ccaaactctg gatttatagc acatccaacc tggcttctgg agtccctgct 660
cgcttcagtg gcagtggatc tgggacctct tactctctca caatcagccg aatggaggct 720
gaagatgctg ccacttatta ctgccagcaa agatctagtt acccactcac gttcggtgct 780
ggcaccaagc tggagctgaa acgggcggcc acaccggtgt cagaaaaaca gctggcggag 840
gtggtcgcga atacgattac cctgctgatg aaagcccagt ctgttccagg catggcggtg 900
gccgttattt atcagggaaa accgcactat tacacatttg gcaaggccga tatcgcggcg 960
aataaacccg ttacgcctca gaccctgttc gagctgggtt ctataagtaa aaccttcacc 1020
ggcgttttag gtggggatgc cattgctcgc ggtgaaattt cgctggacga tgcggtgacc 1080
agatactggc cacagctgac gggcaagcag tggcagggta ttcgtatgct ggatctcgcc 1140
acctacaccg ctggcggcct gccgctacag gtaccggatg aggtcacgga taacgcctcc 1200
ctgctgcgct tttatcaaaa ctggcagccg cagtggaagc ctggcacaac gcgtctttac 1260
gccaacgcca gcatcggtct ttttggtgcg ctggcggtca aaccttctgg catgccctat 1320
gagcaggcca tgacgacgcg ggtccttaag ccgctcaagc tggaccatac ctggattaac 1380
gtgccgaaag cggaagaggc gcattacgcc tggggctatc gtgacggtaa agcggtgcgc 1440
gtttcgccgg gtatgctgga tgcacaagcc tatggcgtga aaaccaacgt gcaggatatg 1500
gcgaactggg tcatggcaaa catggcgccg gagaacgttg ctgatgcctc acttaagcag 1560
ggcatcgcgc tggcgcagtc gcgctactgg cgtatcgggt caatgtatca gggtctgggc 1620
tgggagatgc tcaactggcc cgtggaggcc aacacggtgg tcgagacgag ttttggtaat 1680
gtagcactgg cgccgttgcc cgtggcagaa gtgaatccac cggctccccc ggtcaaagcg 1740
tcctgggtcc ataaaacggg ctctactggc gggtttggca gctacgtggc ctttattcct 1800
gaaaagcaga tcggtattgt gatgctcgcg aatacaagct atccgaaccc ggcacgcgtt 1860
gaggcggcat accatatcct cgaggcgcta cagtaggaat tcgagctccg tcgacaagct 1920
tgcggccgca ctcgagatca aacgggctag ccagccagaa ctcgccccgg aagaccccga 1980
ggatgtcgag caccaccacc accaccactg agatccggct gctaacaaag cccgaaagga 2040
agctgagttg gctgctgcca ccgctgagca ataactagca taaccccttg gggcctctaa 2100
acgggtcttg aggggttttt tgctgaaagg aggaactata tccggattgg cgaatgggac 2160
gcgccctgta gcggcgcatt aagcgcggcg ggtgtggtgg ttacgcgcag cgtgaccgct 2220
acacttgcca gcgccctagc gcccgctcct ttcgctttct tcccttcctt tctcgccacg 2280
ttcgccggct ttccccgtca agctctaaat cgggggctcc ctttagggtt ccgatttagt 2340
gctttacggc acctcgaccc caaaaaactt gattagggtg atggttcacg tagtgggcca 2400
tcgccctgat agacggtttt tcgccctttg acgttggagt ccacgttctt taatagtgga 2460
ctcttgttcc aaactggaac aacactcaac cctatctcgg tctattcttt tgatttataa 2520
gggattttgc cgatttcggc ctattggtta aaaaatgagc tgatttaaca aaaatttaac 2580
gcgaatttta acaaaatatt aacgcttaca atttcctgat gcggtatttt ctccttacgc 2640
atctgtgcgg tatttcacac cgcatatggt gcactctcag tacaatctgc tctgatgccg 2700
catagttaag ccagccccga cacccgccaa cacccgctga cgcgccctga cgggcttgtc 2760
tgctcccggc atccgcttac agacaagctg tgaccgtctc cgggagctgc atgtgtcaga 2820
ggttttcacc gtcatcaccg aaacgcgcga gacgaaaggg cctcgtgata cgcctatttt 2880
tataggttaa tgtcatgata ataatggttt cttagacgtc aggtggcact tttcggggaa 2940
atgtgcgcgg aacccctatt tgtttatttt tctaaataca ttcaaatatg tatccgctca 3000
tgagacaata accctgtggc agcatcaccc gacgcacttt gcgccgaata aatacctgtg 3060
acggaagatc acttcgcaga ataaataaat cctggtgtcc ctgttgatac cgggaagccc 3120
tgggccaact tttggcgaaa atgagacgtt gatcggcacg taagaggttc caactttcac 3180

cataatgaaa taagatcact accgggcgta ttttttgagt tatcgagatt ttcaggagct 3240
aaggaagcta aaatggagaa aaaaatcact ggatatacca ccgttgatat atcccaatgg 3300
catcgtaaag aacattttga ggcatttcag tcagttgctc aatgtaccta taaccagacc 3360
gttcagctgg atattacggc ctttttaaag accgtaaaga aaaataagca caagttttat 3420
ccggccttta ttcacattct tgcccgcctg atgaatgctc atccggaatt ccgtatggca 3480
atgaaagacg gtgagctggt gatatgggat agtgttcacc cttgttacac cgttttccat 3540
gagcaaactg aaacgttttc atcgctctgg agtgaatacc acgacgattt ccggcagttt 3600
ctacacatat attcgcaaga tgtggcgtgt tacggtgaaa acctggccta tttccctaaa 3660
gggtttattg agaatatgtt tttcgtctca gccaatccct gggtgagttt caccagtttt 3720
gatttaaacg tggccaatat ggacaacttc ttcgcccccg ttttcacgat gggcaaatat 3780
tatacgcaag gcgacaaggt gctgatgccg ctggcgattc aggttcatca tgccgtctgt 3840
gatggcttcc atgtcggcag aatgcttaat gaattacaac agtactgcga tgagtggcag 3900
ggcggggcgt aaagacagat cgctgagata ggtgcctcac tgattaagca ttggtaactg 3960
tcagaccaag tttactcata tatactttag attgatttaa aacttcattt ttaatttaaa 4020
aggatctagg tgaagatcct ttttgataat ctcatgacca aaatccctta acgtgagttt 4080
tcgttccact gagcgtcaga ccccgtagaa aagatcaaag gatcttcttg agatcctttt 4140
tttctgcgcg taatctgctg cttgcaaaca aaaaaaccac cgctaccagc ggtggtttgt 4200
ttgccggatc aagagctacc aactcttttt ccgaaggtaa ctggcttcag cagagcgcag 4260
ataccaaata ctgttcttct agtgtagccg tagttaggcc accacttcaa gaactctgta 4320
gcaccgccta catacctcgc tctgctaatc ctgttaccag tggctgctgc cagtggcgat 4380
aagtcgtgtc ttaccgggtt ggactcaaga cgatagttac cggataaggc gcagcggtcg 4440
ggctgaacgg ggggttcgtg cacacagccc agcttggagc gaacgaccta caccgaactg 4500
agatacctac agcgtgagct atgagaaagc gccacgcttc ccgaagggag aaaggcggac 4560
aggtatccgg taagcggcag ggtcggaaca ggagagcgca cgagggagct tccaggggga 4620
aacgcctggt atctttatag tcctgtcggg tttcgccacc tctgacttga gcgtcgattt 4680
ttgtgatgct cgtcaggggg gcggagccta tggaaaaacg ccagcaacgc ggccttttta 4740
cggttcctgg ccttttgctg gccttttgct cacatgttct ttcctgcgtt atcccctgat 4800
tctgtggata accgtattac cgcctttgag tgagctgata ccgctcgccg cagccgaacg 4860
accgagcgca gcgagtcagt gagcgaggaa gcggaagagc gcccaatacg caaaccgcct 4920
ctccccgcgc gttggccgat tcattaatgc agctggcacg acaggtttcc cgactggaaa 4980
gcgggcagtg agcgcaacgc aattaatgtg agttagctca ctcattaggc accccaggct 5040
ttacacttta tgcttccggc tcgtatgttg tgtggaattg tgagcggata acaatttcac 5100
acaggaaaca gctatgacca tgattacgcc aagctattta ggtgacacta tagaatactc 5160
aagctttcta gattaagg 5178

<210> 8
<211> 8
<212> PRT
<213> Artificial Sequence

<220>
<223> beta-lactamase reporter sequence

<400> 8
Arg Leu Tyr Ala Asn Ala Ser Ile
1               5

<210> 9
<211> 8
<212> PRT
<213> Artificial Sequence

<220>
<223> beta-lactamase reporter sequence

<400> 9
Val His Lys Thr Gly Ser Thr Gly
1               5

<210> 10
<211> 4

<212> PRT
<213> Artificial Sequence

<220>
<223> beta-lactamase substrate recognition site

<221> VARIANT
<222> 3
<223> Xaa = Any Amino Acid

<400> 10
Lys Thr Xaa Ser
1

<210> 11
<211> 37
<212> DNA
<213> Artificial Sequence

<220>
<223> primer

<400> 11
actacaatcc atctctccat agtcgcattt ccatcac                                37

<210> 12
<211> 37
<212> DNA
<213> Artificial Sequence

<220>
<223> primer

<400> 12
gtgatggaaa tgcgactatg gagagatgga ttgtagt                                37

<210> 13
<211> 39
<212> DNA
<213> Artificial Sequence

<220>
<223> primer

<400> 13
gccacatatt actgtgcaca taggactctg gctacttac                              39

<210> 14
<211> 39
<212> DNA
<213> Artificial Sequence

<220>
<223> primer

<400> 14
gtaagtagcc agagtcctat gtgcacagta atatgtggc                              39

<210> 15
<211> 37
<212> DNA

<213> Artificial Sequence

<220>
<223> primer

<400> 15
catattactg tgcaagacat actctggcta cttacta                                37

<210> 16
<211> 37
<212> DNA
<213> Artificial Sequence

<220>
<223> primer

<400> 16
tagtaagtag ccagagtatg tcttgcacag taatatg                                37

<210> 17
<211> 37
<212> DNA
<213> Artificial Sequence

<220>
<223> primer

<400> 17
attactgtgc aagaaggcat ctggctactt actatgc                                37

<210> 18
<211> 37
<212> DNA
<213> Artificial Sequence

<220>
<223> primer

<400> 18
gcatagtaag tagccagatg ccttcttgca cagtaat                                37

<210> 19
<211> 37
<212> DNA
<213> Artificial Sequence

<220>
<223> primer

<400> 19
actgtgcaag aaggactcat gctacttact atgctat                                37

<210> 20
<211> 37
<212> DNA
<213> Artificial Sequence

<220>
<223> primer

<400> 20

atagcatagt aagtagcatg agtccttctt gcacagt 37

<210> 21
<211> 37
<212> DNA
<213> Artificial Sequence

<220>
<223> primer

<400> 21
gtgcaagaag gactctgcat acttactatg ctatgga 37

<210> 22
<211> 37
<212> DNA
<213> Artificial Sequence

<220>
<223> primer

<400> 22
tccatagcat agtaagtatg cagagtcctt cttgcac 37

<210> 23
<211> 37
<212> DNA
<213> Artificial Sequence

<220>
<223> primer

<400> 23
caagaaggac tctggctcat tactatgcta tggacta 37

<210> 24
<211> 37
<212> DNA
<213> Artificial Sequence

<220>
<223> primer

<400> 24
tagtccatag catagtaatg agccagagtc cttcttg 37

<210> 25
<211> 37
<212> DNA
<213> Artificial Sequence

<220>
<223> primer

<400> 25
gaaggactct ggctactcat tatgctatgg actactg 37

<210> 26
<211> 37
<212> DNA
<213> Artificial Sequence

<220>
<223> primer

<400> 26
cagtagtcca tagcataatg agtagccaga gtccttc                                37

<210> 27
<211> 37
<212> DNA
<213> Artificial Sequence

<220>
<223> primer

<400> 27
ggactctggc tacttaccat gctatggact actgggg                                37

<210> 28
<211> 37
<212> DNA
<213> Artificial Sequence

<220>
<223> primer

<400> 28
ccccagtagt ccatagcatg gtaagtagcc agagtcc                                37

<210> 29
<211> 37
<212> DNA
<213> Artificial Sequence

<220>
<223> primer

<400> 29
ctctggctac ttactatcat atggactact ggggtca                                37

<210> 30
<211> 37
<212> DNA
<213> Artificial Sequence

<220>
<223> primer

<400> 30
tgaccccagt agtccatatg atagtaagta gccagag                                37

<210> 31
<211> 37
<212> DNA
<213> Artificial Sequence

<220>
<223> primer

<400> 31
tggctactta ctatgctcat gactactggg gtcaagg                                37

<210> 32
<211> 37
<212> DNA
<213> Artificial Sequence

<220>
<223> primer

<400> 32
ccttgacccc agtagtcatg agcatagtaa gtagcca                                37

<210> 33
<211> 37
<212> DNA
<213> Artificial Sequence

<220>
<223> primer

<400> 33
ctacttacta tgctatgcat tactggggtc aaggaac                                37

<210> 34
<211> 37
<212> DNA
<213> Artificial Sequence

<220>
<223> primer

<400> 34
gttccttgac cccagtaatg catagcatag taagtag                                37

<210> 35
<211> 37
<212> DNA
<213> Artificial Sequence

<220>
<223> primer

<400> 35
cttactatgc tatggaccat tggggtcaag gaacctc                                37

<210> 36
<211> 37
<212> DNA
<213> Artificial Sequence

<220>
<223> primer

<400> 36
gaggttcctt gaccccaatg gtccatagca tagtaag                                37

<210> 37
<211> 37
<212> DNA
<213> Artificial Sequence

<220>
<223> primer

<400> 37
actatgctat ggactaccat ggtcaaggaa cctctgt                                37

<210> 38
<211> 37
<212> DNA
<213> Artificial Sequence

<220>
<223> primer

<400> 38
acagaggttc cttgaccatg gtagtccata gcatagt                                37

<210> 39
<211> 37
<212> DNA
<213> Artificial Sequence

<220>
<223> primer

<400> 39
caaagctcct gatctaccat gtttccaacc gattttc                                37

<210> 40
<211> 37
<212> DNA
<213> Artificial Sequence

<220>
<223> primer

<400> 40
gaaaatcggt tggaaacatg gtagatcagg agctttg                                37

<210> 41
<211> 43
<212> DNA
<213> Artificial Sequence

<220>
<223> primer

<400> 41
gattttctgg ggtcccagac catttcagtg gcagtggatc agg                         43

<210> 42
<211> 43
<212> DNA
<213> Artificial Sequence

<220>
<223> primer

<400> 42
cctgatccac tgccactgaa atggtctggg accccagaaa atc                         43

<210> 43
<211> 37
<212> DNA
<213> Artificial Sequence

<220>
<223> primer

<400> 43
gagtttattt ctgctctcat agtacacatg ttcctcc                                37

<210> 44
<211> 37
<212> DNA
<213> Artificial Sequence

<220>
<223> primer

<400> 44
ggaggaacat gtgtactatg agagcagaaa taaactc                                37

<210> 45
<211> 39
<212> DNA
<213> Artificial Sequence

<220>
<223> primer

<400> 45
gtttatttct gctctcaaca tacacatgtt cctccgacg                              39

<210> 46
<211> 39
<212> DNA
<213> Artificial Sequence

<220>
<223> primer

<400> 46
cgtcggagga acatgtgtat gttgagagca gaaataaac                              39

<210> 47
<211> 45
<212> DNA
<213> Artificial Sequence

<220>
<223> primer

<400> 47
gtttatttct gctctcaaag tcatcatgtt cctccgacgt tcggt                       45

<210> 48
<211> 45
<212> DNA
<213> Artificial Sequence

<220>

<223> primer

<400> 48
accgaacgtc ggaggaacat gatgactttg agagcagaaa taaac                    45

<210> 49
<211> 37
<212> DNA
<213> Artificial Sequence

<220>
<223> primer

<400> 49
tctgctctca aagtacacat gttcctccga cgttcgg                             37

<210> 50
<211> 37
<212> DNA
<213> Artificial Sequence

<220>
<223> primer

<400> 50
ccgaacgtcg gaggaacatg tgtactttga gagcaga                             37

<210> 51
<211> 37
<212> DNA
<213> Artificial Sequence

<220>
<223> primer

<400> 51
gctctcaaag tacacatcat cctccgacgt tcggtgg                             37

<210> 52
<211> 37
<212> DNA
<213> Artificial Sequence

<220>
<223> primer

<400> 52
ccaccgaacg tcggaggatg atgtgtactt tgagagc                             37

<210> 53
<211> 37
<212> DNA
<213> Artificial Sequence

<220>
<223> primer

<400> 53
ctcaaagtac acatgttcat ccgacgttcg gtggagg                             37

<210> 54

<211> 37
<212> DNA
<213> Artificial Sequence

<220>
<223> primer

<400> 54
cctccaccga acgtcggatg aacatgtgta ctttgag                                37

<210> 55
<211> 39
<212> DNA
<213> Artificial Sequence

<220>
<223> primer

<400> 55
caaagtacac atgttcctca tacgttcggt ggaggcacc                              39

<210> 56
<211> 39
<212> DNA
<213> Artificial Sequence

<220>
<223> primer

<400> 56
ggtgcctcca ccgaacgtat gaggaacatg tgtactttg                              39

<210> 57
<211> 39
<212> DNA
<213> Artificial Sequence

<220>
<223> primer

<400> 57
agtacacatg ttcctccgca tttcggtgga ggcaccaag                              39

<210> 58
<211> 39
<212> DNA
<213> Artificial Sequence

<220>
<223> primer

<400> 58
cttggtgcct ccaccgaaat gcggaggaac atgtgtact                              39

<210> 59
<211> 37
<212> DNA
<213> Artificial Sequence

<220>
<223> primer

<221> misc_feature
<222> 18, 19
<223> n = A,T,C or G

<400> 59
ctggcgactc catcaccnns ggttactgga actggat                                37

<210> 60
<211> 37
<212> DNA
<213> Artificial Sequence

<220>
<223> primer

<221> misc_feature
<222> 19, 20
<223> n = A,T,C or G

<400> 60
atccagttcc agtaaccsnn ggtgatggag tcgccag                                37

<210> 61
<211> 37
<212> DNA
<213> Artificial Sequence

<220>
<223> primer

<221> misc_feature
<222> 18, 19
<223> n = A,T,C or G

<400> 61
gcgactccat caccagtnns tactggaact ggatccg                                37

<210> 62
<211> 37
<212> DNA
<213> Artificial Sequence

<220>
<223> primer

<221> misc_feature
<222> 19, 20
<223> n = A,T,C or G

<400> 62
cggatccagt tccagtasnn actggtgatg gagtcgc                                37

<210> 63
<211> 37
<212> DNA
<213> Artificial Sequence

<220>
<223> primer

<221> misc_feature
<222> 18, 19
<223> n = A,T,C or G

<400> 63
actccatcac cagtggtnns tggaactgga tccggca                                37

<210> 64
<211> 37
<212> DNA
<213> Artificial Sequence

<220>
<223> primer

<221> misc_feature
<222> 19, 20
<223> n = A,T,C or G

<400> 64
tgccggatcc agttccasnn accactggtg atggagt                                37

<210> 65
<211> 39
<212> DNA
<213> Artificial Sequence

<220>
<223> primer

<221> misc_feature
<222> 19, 20
<223> n = A,T,C or G

<400> 65
tccatcacca gtggttacnn saactggatc cggcagttc                              39

<210> 66
<211> 39
<212> DNA
<213> Artificial Sequence

<220>
<223> primer

<221> misc_feature
<222> 20, 21
<223> n = A,T,C or G

<400> 66
gaactgccgg atccagttsn ngtaaccact ggtgatgga                              39

<210> 67
<211> 37
<212> DNA
<213> Artificial Sequence

<220>
<223> primer

<221> misc_feature

<222> 18, 19
<223> n = A,T,C or G

<400> 67
aacttgaata tatgggtnns ataagcgaca gtggtat                                37

<210> 68
<211> 37
<212> DNA
<213> Artificial Sequence

<220>
<223> primer

<221> misc_feature
<222> 19, 20
<223> n = A,T,C or G

<400> 68
ataccactgt cgcttatsnn acccatatat tcaagtt                                37

<210> 69
<211> 37
<212> DNA
<213> Artificial Sequence

<220>
<223> primer

<221> misc_feature
<222> 18, 19
<223> n = A,T,C or G

<400> 69
ttgaatatat gggttacnns agcgacagtg gtatcac                                37

<210> 70
<211> 37
<212> DNA
<213> Artificial Sequence

<220>
<223> primer

<221> misc_feature
<222> 19, 20
<223> n = A,T,C or G

<400> 70
gtgataccac tgtcgctsnn gtaacccata tattcaa                                37

<210> 71
<211> 39
<212> DNA
<213> Artificial Sequence

<220>
<223> primer

<221> misc_feature
<222> 19, 20

<223> n = A,T,C or G

<400> 71
gaatatatgg gttacatann sgacagtggt atcacttac                                39

<210> 72
<211> 39
<212> DNA
<213> Artificial Sequence

<220>
<223> primer

<221> misc_feature
<222> 20, 21
<223> n = A,T,C or G

<400> 72
gtaagtgata ccactgtcsn ntatgtaacc catatattc                                39

<210> 73
<211> 39
<212> DNA
<213> Artificial Sequence

<220>
<223> primer

<221> misc_feature
<222> 19, 20
<223> n = A,T,C or G

<400> 73
tatatgggtt acataagcnn sagtggtatc acttactac                                39

<210> 74
<211> 39
<212> DNA
<213> Artificial Sequence

<220>
<223> primer

<221> misc_feature
<222> 20, 21
<223> n = A,T,C or G

<400> 74
gtagtaagtg ataccactsn ngcttatgta acccatata                                39

<210> 75
<211> 39
<212> DNA
<213> Artificial Sequence

<220>
<223> primer

<221> misc_feature
<222> 19, 20
<223> n = A,T,C or G

<400> 75
atgggttaca taagcgacnn sggtatcact tactacaat                                39

<210> 76
<211> 39
<212> DNA
<213> Artificial Sequence

<220>
<223> primer

<221> misc_feature
<222> 20, 21
<223> n = A,T,C or G

<400> 76
attgtagtaa gtgataccsn ngtcgcttat gtaacccat                                39

<210> 77
<211> 37
<212> DNA
<213> Artificial Sequence

<220>
<223> primer

<221> misc_feature
<222> 18, 19
<223> n = A,T,C or G

<400> 77
gttacataag cgacagtnns atcacttact acaatcc                                  37

<210> 78
<211> 37
<212> DNA
<213> Artificial Sequence

<220>
<223> primer

<221> misc_feature
<222> 19, 20
<223> n = A,T,C or G

<400> 78
ggattgtagt aagtgatsnn actgtcgctt atgtaac                                  37

<210> 79
<211> 37
<212> DNA
<213> Artificial Sequence

<220>
<223> primer

<221> misc_feature
<222> 18, 19
<223> n = A,T,C or G

<400> 79
acataagcga cagtggtnns acttactaca atccatc                                37

<210> 80
<211> 37
<212> DNA
<213> Artificial Sequence

<220>
<223> primer

<221> misc_feature
<222> 19, 20
<223> n = A,T,C or G

<400> 80
gatggattgt agtaagtsnn accactgtcg cttatgt                                37

<210> 81
<211> 37
<212> DNA
<213> Artificial Sequence

<220>
<223> primer

<221> misc_feature
<222> 18, 19
<223> n = A,T,C or G

<400> 81
taagcgacag tggtatcnns tactacaatc catctct                                37

<210> 82
<211> 37
<212> DNA
<213> Artificial Sequence

<220>
<223> primer

<221> misc_feature
<222> 19, 20
<223> n = A,T,C or G

<400> 82
agagatggat tgtagtasnn gataccactg tcgctta                                37

<210> 83
<211> 43
<212> DNA
<213> Artificial Sequence

<220>
<223> primer

<221> misc_feature
<222> 21, 22
<223> n = A,T,C or G

<400> 83

taagcgacag tggtatcact nnstacaatc catctctcaa aag 43

<210> 84
<211> 43
<212> DNA
<213> Artificial Sequence

<220>
<223> primer

<221> misc_feature
<222> 22, 23
<223> n = A,T,C or G

<400> 84
cttttgagag atggattgta snnagtgata ccactgtcgc tta 43

<210> 85
<211> 39
<212> DNA
<213> Artificial Sequence

<220>
<223> primer

<221> misc_feature
<222> 19, 20
<223> n = A,T,C or G

<400> 85
gacagtggta tcacttacnn saatccatct ctcaaaagt 39

<210> 86
<211> 39
<212> DNA
<213> Artificial Sequence

<220>
<223> primer

<221> misc_feature
<222> 20, 21
<223> n = A,T,C or G

<400> 86
acttttgaga gatggattsn ngtaagtgat accactgtc 39

<210> 87
<211> 37
<212> DNA
<213> Artificial Sequence

<220>
<223> primer

<221> misc_feature
<222> 18, 19
<223> n = A,T,C or G

<400> 87
gtggtatcac ttactacnns ccatctctca aaagtcg 37

<210> 88
<211> 37
<212> DNA
<213> Artificial Sequence

<220>
<223> primer

<221> misc_feature
<222> 19, 20
<223> n = A,T,C or G

<400> 88
cgacttttga gagatggsnn gtagtaagtg ataccac                                37

<210> 89
<211> 37
<212> DNA
<213> Artificial Sequence

<220>
<223> primer

<221> misc_feature
<222> 18, 19
<223> n = A,T,C or G

<400> 89
gtatcactta ctacaatnns tctctcaaaa gtcgcat                                37

<210> 90
<211> 37
<212> DNA
<213> Artificial Sequence

<220>
<223> primer

<221> misc_feature
<222> 19, 20
<223> n = A,T,C or G

<400> 90
atgcgacttt tgagagasnn attgtagtaa gtgatac                                37

<210> 91
<211> 37
<212> DNA
<213> Artificial Sequence

<220>
<223> primer

<221> misc_feature
<222> 18, 19
<223> n = A,T,C or G

<400> 91
tcacttacta caatccanns ctcaaaagtc gcatttc                                37

<210> 92
<211> 37
<212> DNA
<213> Artificial Sequence

<220>
<223> primer

<221> misc_feature
<222> 19, 20
<223> n = A,T,C or G

<400> 92
gaaatgcgac ttttgagsnn tggattgtag taagtga                                37

<210> 93
<211> 37
<212> DNA
<213> Artificial Sequence

<220>
<223> primer

<221> misc_feature
<222> 18, 19
<223> n = A,T,C or G

<400> 93
cttactacaa tccatctnns aaaagtcgca tttccat                                37

<210> 94
<211> 37
<212> DNA
<213> Artificial Sequence

<220>
<223> primer

<221> misc_feature
<222> 19, 20
<223> n = A,T,C or G

<400> 94
atggaaatgc gacttttsnn agatggattg tagtaag                                37

<210> 95
<211> 37
<212> DNA
<213> Artificial Sequence

<220>
<223> primer

<221> misc_feature
<222> 18, 19
<223> n = A,T,C or G

<400> 95
actacaatcc atctctcnns agtcgcattt ccatcac                                37

<210> 96

<211> 27
<212> DNA
<213> Artificial Sequence

<220>
<223> primer

<221> misc_feature
<222> 19, 20
<223> n = A,T,C or G

<400> 96
gtgatggaaa tgcgactsnn gagagat                                          27

<210> 97
<211> 10
<212> DNA
<213> Artificial Sequence

<220>
<223> primer

<400> 97
ggattgtagt                                                             10

<210> 98
<211> 37
<212> DNA
<213> Artificial Sequence

<220>
<223> primer

<221> misc_feature
<222> 18, 19
<223> n = A,T,C or G

<400> 98
acaatccatc tctcaaanns cgcatttcca tcactcg                               37

<210> 99
<211> 37
<212> DNA
<213> Artificial Sequence

<220>
<223> primer

<221> misc_feature
<222> 19, 20
<223> n = A,T,C or G

<400> 99
cgagtgatgg aaatgcgsnn tttgagagat ggattgt                               37

<210> 100
<211> 39
<212> DNA
<213> Artificial Sequence

<220>

<223> primer

<221> misc_feature
<222> 19, 20
<223> n = A,T,C or G

<400> 100
gccacatatt actgtgcann saggactctg gctacttac                                39

<210> 101
<211> 39
<212> DNA
<213> Artificial Sequence

<220>
<223> primer

<221> misc_feature
<222> 20, 21
<223> n = A,T,C or G

<400> 101
gtaagtagcc agagtcctsn ntgcacagta atatgtggc                                39

<210> 102
<211> 37
<212> DNA
<213> Artificial Sequence

<220>
<223> primer

<221> misc_feature
<222> 18, 19
<223> n = A,T,C or G

<400> 102
catattactg tgcaaganns actctggcta cttacta                                  37

<210> 103
<211> 37
<212> DNA
<213> Artificial Sequence

<220>
<223> primer

<221> misc_feature
<222> 19, 20
<223> n = A,T,C or G

<400> 103
tagtaagtag ccagagtsnn tcttgcacag taatatg                                  37

<210> 104
<211> 37
<212> DNA
<213> Artificial Sequence

<220>
<223> primer

<221> misc_feature
<222> 18, 19
<223> n = A,T,C or G

<400> 104
attactgtgc aagaaggnns ctggctactt actatgc 37

<210> 105
<211> 37
<212> DNA
<213> Artificial Sequence

<220>
<223> primer

<221> misc_feature
<222> 19, 20
<223> n = A,T,C or G

<400> 105
gcatagtaag tagccagsnn ccttcttgca cagtaat 37

<210> 106
<211> 37
<212> DNA
<213> Artificial Sequence

<220>
<223> primer

<221> misc_feature
<222> 18, 19
<223> n = A,T,C or G

<400> 106
actgtgcaag aaggactnns gctacttact atgctat 37

<210> 107
<211> 37
<212> DNA
<213> Artificial Sequence

<220>
<223> primer

<221> misc_feature
<222> 19, 20
<223> n = A,T,C or G

<400> 107
atagcatagt aagtagcsnn agtccttctt gcacagt 37

<210> 108
<211> 37
<212> DNA
<213> Artificial Sequence

<220>
<223> primer

<221> misc_feature
<222> 18, 19
<223> n = A,T,C or G

<400> 108
gtgcaagaag gactctgnns acttactatg ctatgga                                37

<210> 109
<211> 37
<212> DNA
<213> Artificial Sequence

<220>
<223> primer

<221> misc_feature
<222> 19, 20
<223> n = A,T,C or G

<400> 109
tccatagcat agtaagtsnn cagagtcctt cttgcac                                37

<210> 110
<211> 37
<212> DNA
<213> Artificial Sequence

<220>
<223> primer

<221> misc_feature
<222> 18, 19
<223> n = A,T,C or G

<400> 110
caagaaggac tctggctnns tactatgcta tggacta                                37

<210> 111
<211> 37
<212> DNA
<213> Artificial Sequence

<220>
<223> primer

<221> misc_feature
<222> 19, 20
<223> n = A,T,C or G

<400> 111
tagtccatag catagtasnn agccagagtc cttcttg                                37

<210> 112
<211> 37
<212> DNA
<213> Artificial Sequence

<220>
<223> primer

<221> misc_feature

<222> 18, 19
<223> n = A,T,C or G

<400> 112
gaaggactct ggctactnns tatgctatgg actactg                                37

<210> 113
<211> 37
<212> DNA
<213> Artificial Sequence

<220>
<223> primer

<221> misc_feature
<222> 19, 20
<223> n = A,T,C or G

<400> 113
cagtagtcca tagcatasnn agtagccaga gtccttc                                37

<210> 114
<211> 37
<212> DNA
<213> Artificial Sequence

<220>
<223> primer

<221> misc_feature
<222> 18, 19
<223> n = A,T,C or G

<400> 114
ggactctggc tacttacnns gctatggact actgggg                                37

<210> 115
<211> 37
<212> DNA
<213> Artificial Sequence

<220>
<223> primer

<221> misc_feature
<222> 19, 20
<223> n = A,T,C or G

<400> 115
ccccagtagt ccatagcsnn gtaagtagcc agagtcc                                37

<210> 116
<211> 37
<212> DNA
<213> Artificial Sequence

<220>
<223> primer

<221> misc_feature
<222> 18, 19

<223> n = A,T,C or G

<400> 116
ctctggctac ttactatnns atggactact ggggtca                                37

<210> 117
<211> 37
<212> DNA
<213> Artificial Sequence

<220>
<223> primer

<221> misc_feature
<222> 19, 20
<223> n = A,T,C or G

<400> 117
tgaccccagt agtccatsnn atagtaagta gccagag                                37

<210> 118
<211> 37
<212> DNA
<213> Artificial Sequence

<220>
<223> primer

<221> misc_feature
<222> 18, 19
<223> n = A,T,C or G

<400> 118
tggctactta ctatgctnns gactactggg gtcaagg                                37

<210> 119
<211> 37
<212> DNA
<213> Artificial Sequence

<220>
<223> primer

<221> misc_feature
<222> 19, 20
<223> n = A,T,C or G

<400> 119
ccttgacccc agtagtcsnn agcatagtaa gtagcca                                37

<210> 120
<211> 37
<212> DNA
<213> Artificial Sequence

<220>
<223> primer

<221> misc_feature
<222> 18, 19
<223> n = A,T,C or G

<400> 120
ctacttacta tgctatgnns tactggggtc aaggaac                                37

<210> 121
<211> 37
<212> DNA
<213> Artificial Sequence

<220>
<223> primer

<221> misc_feature
<222> 19, 20
<223> n = A,T,C or G

<400> 121
gttccttgac cccagtasnn catagcatag taagtag                                37

<210> 122
<211> 37
<212> DNA
<213> Artificial Sequence

<220>
<223> primer

<221> misc_feature
<222> 18, 19
<223> n = A,T,C or G

<400> 122
cttactatgc tatggacnns tggggtcaag gaacctc                                37

<210> 123
<211> 37
<212> DNA
<213> Artificial Sequence

<220>
<223> primer

<221> misc_feature
<222> 19, 20
<223> n = A,T,C or G

<400> 123
gaggttcctt gaccccasnn gtccatagca tagtaag                                37

<210> 124
<211> 37
<212> DNA
<213> Artificial Sequence

<220>
<223> primer

<221> misc_feature
<222> 18, 19
<223> n = A,T,C or G

<400> 124
actatgctat ggactacnns ggtcaaggaa cctctgt                                37

<210> 125
<211> 37
<212> DNA
<213> Artificial Sequence

<220>
<223> primer

<221> misc_feature
<222> 19, 20
<223> n = A,T,C or G

<400> 125
acagaggttc cttgaccsnn gtagtccata gcatagt                                37

<210> 126
<211> 37
<212> DNA
<213> Artificial Sequence

<220>
<223> primer

<221> misc_feature
<222> 18, 19
<223> n = A,T,C or G

<400> 126
cctccatctc ttgcaggnns agtcagagcc ttgtaca                                37

<210> 127
<211> 37
<212> DNA
<213> Artificial Sequence

<220>
<223> primer

<221> misc_feature
<222> 19, 20
<223> n = A,T,C or G

<400> 127
tgtacaaggc tctgactsnn cctgcaagag atggagg                                37

<210> 128
<211> 37
<212> DNA
<213> Artificial Sequence

<220>
<223> primer

<221> misc_feature
<222> 18, 19
<223> n = A,T,C or G

<400> 128

ccatctcttg cagggctnns cagagccttg tacacag 37

<210> 129
<211> 37
<212> DNA
<213> Artificial Sequence

<220>
<223> primer

<221> misc_feature
<222> 19, 20
<223> n = A,T,C or G

<400> 129
ctgtgtacaa ggctctgsnn agccctgcaa gagatgg 37

<210> 130
<211> 39
<212> DNA
<213> Artificial Sequence

<220>
<223> primer

<221> misc_feature
<222> 19, 20
<223> n = A,T,C or G

<400> 130
atctcttgca gggctagtnn sagccttgta cacagtaat 39

<210> 131
<211> 39
<212> DNA
<213> Artificial Sequence

<220>
<223> primer

<221> misc_feature
<222> 20, 21
<223> n = A,T,C or G

<400> 131
attactgtgt acaaggctsn nactagccct gcaagagat 39

<210> 132
<211> 37
<212> DNA
<213> Artificial Sequence

<220>
<223> primer

<221> misc_feature
<222> 18, 19
<223> n = A,T,C or G

<400> 132
cttgcagggc tagtcagnns cttgtacaca gtaatgg 37

<210> 133
<211> 37
<212> DNA
<213> Artificial Sequence

<220>
<223> primer

<221> misc_feature
<222> 19, 20
<223> n = A,T,C or G

<400> 133
ccattactgt gtacaagsnn ctgactagcc ctgcaag                                37

<210> 134
<211> 39
<212> DNA
<213> Artificial Sequence

<220>
<223> primer

<221> misc_feature
<222> 19, 20
<223> n = A,T,C or G

<400> 134
tgcagggcta gtcagagcnn sgtacacagt aatggaaac                              39

<210> 135
<211> 39
<212> DNA
<213> Artificial Sequence

<220>
<223> primer

<221> misc_feature
<222> 20, 21
<223> n = A,T,C or G

<400> 135
gtttccatta ctgtgtacsn ngctctgact agccctgca                              39

<210> 136
<211> 37
<212> DNA
<213> Artificial Sequence

<220>
<223> primer

<221> misc_feature
<222> 18, 19
<223> n = A,T,C or G

<400> 136
gggctagtca gagccttnns cacagtaatg gaaacac                                37

<210> 137
<211> 37
<212> DNA
<213> Artificial Sequence

<220>
<223> primer

<221> misc_feature
<222> 19, 20
<223> n = A,T,C or G

<400> 137
gtgtttccat tactgtgsnn aaggctctga ctagccc                                37

<210> 138
<211> 37
<212> DNA
<213> Artificial Sequence

<220>
<223> primer

<221> misc_feature
<222> 18, 19
<223> n = A,T,C or G

<400> 138
ctagtcagag ccttgtanns agtaatggaa acaccta                                37

<210> 139
<211> 37
<212> DNA
<213> Artificial Sequence

<220>
<223> primer

<221> misc_feature
<222> 19, 20
<223> n = A,T,C or G

<400> 139
taggtgtttc cattactsnn tacaaggctc tgactag                                37

<210> 140
<211> 41
<212> DNA
<213> Artificial Sequence

<220>
<223> primer

<221> misc_feature
<222> 20, 21
<223> n = A,T,C or G

<400> 140
tagtcagagc cttgtacacn nsaatggaaa cacctattta c                           41

<210> 141

<211> 41
<212> DNA
<213> Artificial Sequence

<220>
<223> primer

<221> misc_feature
<222> 21, 22
<223> n = A,T,C or G

<400> 141
gtaaataggt gtttccatts nngtgtacaa ggctctgact a                41

<210> 142
<211> 37
<212> DNA
<213> Artificial Sequence

<220>
<223> primer

<221> misc_feature
<222> 18, 19
<223> n = A,T,C or G

<400> 142
agagccttgt acacagtnns ggaaacacct atttaca                37

<210> 143
<211> 37
<212> DNA
<213> Artificial Sequence

<220>
<223> primer

<221> misc_feature
<222> 19, 20
<223> n = A,T,C or G

<400> 143
tgtaaatagg tgtttccsnn actgtgtaca aggctct                37

<210> 144
<211> 37
<212> DNA
<213> Artificial Sequence

<220>
<223> primer

<221> misc_feature
<222> 18, 19
<223> n = A,T,C or G

<400> 144
gccttgtaca cagtaatnns aacacctatt tacattg                37

<210> 145
<211> 37

<212> DNA
<213> Artificial Sequence

<220>
<223> primer

<221> misc_feature
<222> 19, 20
<223> n = A,T,C or G

<400> 145
caatgtaaat aggtgttsnn attactgtgt acaaggc                                37

<210> 146
<211> 37
<212> DNA
<213> Artificial Sequence

<220>
<223> primer

<221> misc_feature
<222> 18, 19
<223> n = A,T,C or G

<400> 146
ttgtacacag taatgganns acctatttac attggta                                37

<210> 147
<211> 37
<212> DNA
<213> Artificial Sequence

<220>
<223> primer

<221> misc_feature
<222> 19, 20
<223> n = A,T,C or G

<400> 147
taccaatgta aataggtsnn tccattactg tgtacaa                                37

<210> 148
<211> 36
<212> DNA
<213> Artificial Sequence

<220>
<223> primer

<221> misc_feature
<222> 18, 19
<223> n = A,T,C or G

<400> 148
tacacagtaa tggaaacnns tatttacatt ggtacc                                 36

<210> 149
<211> 36
<212> DNA

<213> Artificial Sequence

<220>
<223> primer

<221> misc_feature
<222> 18, 19
<223> n = A,T,C or G

<400> 149
ggtaccaatg taaatasnng tttccattac tgtgta                                36

<210> 150
<211> 37
<212> DNA
<213> Artificial Sequence

<220>
<223> primer

<221> misc_feature
<222> 18, 19
<223> n = A,T,C or G

<400> 150
acagtaatgg aaacaccnns ttacattggt acctgca                               37

<210> 151
<211> 37
<212> DNA
<213> Artificial Sequence

<220>
<223> primer

<221> misc_feature
<222> 19, 20
<223> n = A,T,C or G

<400> 151
tgcaggtacc aatgtaasnn ggtgtttcca ttactgt                               37

<210> 152
<211> 39
<212> DNA
<213> Artificial Sequence

<220>
<223> primer

<221> misc_feature
<222> 19, 20
<223> n = A,T,C or G

<400> 152
agtaatggaa acacctatnn scattggtac ctgcagaag                             39

<210> 153
<211> 39
<212> DNA
<213> Artificial Sequence

<220>
<223> primer

<221> misc_feature
<222> 20, 21
<223> n = A,T,C or G

<400> 153
cttctgcagg taccaatgsn nataggtgtt tccattact                                39

<210> 154
<211> 37
<212> DNA
<213> Artificial Sequence

<220>
<223> primer

<221> misc_feature
<222> 18, 19
<223> n = A,T,C or G

<400> 154
atggaaacac ctatttanns tggtacctgc agaagcc                                  37

<210> 155
<211> 37
<212> DNA
<213> Artificial Sequence

<220>
<223> primer

<221> misc_feature
<222> 19, 20
<223> n = A,T,C or G

<400> 155
ggcttctgca ggtaccasnn taaataggtg tttccat                                  37

<210> 156
<211> 37
<212> DNA
<213> Artificial Sequence

<220>
<223> primer

<221> misc_feature
<222> 18, 19
<223> n = A,T,C or G

<400> 156
ctccaaagct cctgatcnns agagtttcca accgatt                                  37

<210> 157
<211> 37
<212> DNA
<213> Artificial Sequence

<220>
<223> primer

<221> misc_feature
<222> 19, 20
<223> n = A,T,C or G

<400> 157
aatcggttgg aaactctsnn gatcaggagc tttggag                                37

<210> 158
<211> 37
<212> DNA
<213> Artificial Sequence

<220>
<223> primer

<221> misc_feature
<222> 18, 19
<223> n = A,T,C or G

<400> 158
caaagctcct gatctacnns gtttccaacc gattttc                                37

<210> 159
<211> 37
<212> DNA
<213> Artificial Sequence

<220>
<223> primer

<221> misc_feature
<222> 19, 20
<223> n = A,T,C or G

<400> 159
gaaaatcggt tggaaacsnn gtagatcagg agctttg                                37

<210> 160
<211> 37
<212> DNA
<213> Artificial Sequence

<220>
<223> primer

<221> misc_feature
<222> 18, 19
<223> n = A,T,C or G

<400> 160
   agctcctgat ctacaganns tccaaccgat tttctgg                                 37

<210> 161
<211> 37
<212> DNA
<213> Artificial Sequence

<220>

<223> primer

<221> misc_feature
<222> 19, 20
<223> n = A,T,C or G

<400> 161
ccagaaaatc ggttggasnn tctgtagatc aggagct                                37

<210> 162
<211> 37
<212> DNA
<213> Artificial Sequence

<220>
<223> primer

<221> misc_feature
<222> 18, 19
<223> n = A,T,C or G

<400> 162
tcctgatcta cagagttnns aaccgatttt ctggggt                                37

<210> 163
<211> 37
<212> DNA
<213> Artificial Sequence

<220>
<223> primer

<221> misc_feature
<222> 19, 20
<223> n = A,T,C or G

<400> 163
accccagaaa atcggttsnn aactctgtag atcagga                                37

<210> 164
<211> 37
<212> DNA
<213> Artificial Sequence

<220>
<223> primer

<221> misc_feature
<222> 18, 19
<223> n = A,T,C or G

<400> 164
tgatctacag agtttccnns cgattttctg gggtccc                                37

<210> 165
<211> 37
<212> DNA
<213> Artificial Sequence

<220>
<223> primer

<221> misc_feature
<222> 19, 20
<223> n = A,T,C or G

<400> 165
gggaccccag aaaatcgsnn ggaaactctg tagatca                                37

<210> 166
<211> 37
<212> DNA
<213> Artificial Sequence

<220>
<223> primer

<221> misc_feature
<222> 18, 19
<223> n = A,T,C or G

<400> 166
tctacagagt ttccaacnns ttttctgggg tcccaga                                37

<210> 167
<211> 37
<212> DNA
<213> Artificial Sequence

<220>
<223> primer

<221> misc_feature
<222> 19, 20
<223> n = A,T,C or G

<400> 167
tctgggaccc cagaaaasnn gttggaaact ctgtaga                                37

<210> 168
<211> 37
<212> DNA
<213> Artificial Sequence

<220>
<223> primer

<221> misc_feature
<222> 18, 19
<223> n = A,T,C or G

<400> 168
acagagtttc caaccganns tctggggtcc cagacag                                37

<210> 169
<211> 37
<212> DNA
<213> Artificial Sequence

<220>
<223> primer

<221> misc_feature
<222> 19, 20
<223> n = A,T,C or G

<400> 169
ctgtctggga ccccagasnn tcggttggaa actctgt                                37

<210> 170
<211> 37
<212> DNA
<213> Artificial Sequence

<220>
<223> primer

<221> misc_feature
<222> 18, 19
<223> n = A,T,C or G

<400> 170
gagtttccaa ccgatttnns ggggtcccag acaggtt                                37

<210> 171
<211> 37
<212> DNA
<213> Artificial Sequence

<220>
<223> primer

<221> misc_feature
<222> 19, 20
<223> n = A,T,C or G

<400> 171
aacctgtctg ggaccccsnn aaatcggttg gaaactc                                37

<210> 172
<211> 37
<212> DNA
<213> Artificial Sequence

<220>
<223> primer

<221> misc_feature
<222> 18, 19
<223> n = A,T,C or G

<400> 172
gagtttattt ctgctctnns agtacacatg ttcctcc                                37

<210> 173
<211> 37
<212> DNA
<213> Artificial Sequence

<220>
<223> primer

<221> misc_feature

<222> 19, 20
<223> n = A,T,C or G

<400> 173
ggaggaacat gtgtactsnn agagcagaaa taaactc                                37

<210> 174
<211> 43
<212> DNA
<213> Artificial Sequence

<220>
<223> primer

<221> misc_feature
<222> 20, 21
<223> n = A,T,C or G

<400> 174
gagtttattt ctgctctcaa nnsacacatg ttcctccgca ttt                         43

<210> 175
<211> 43
<212> DNA
<213> Artificial Sequence

<220>
<223> primer

<221> misc_feature
<222> 22, 23
<223> n = A,T,C or G

<400> 175
aaatgcggag gaacatgtgt snnttgagag cagaaataaa ctc                         43

<210> 176
<211> 39
<212> DNA
<213> Artificial Sequence

<220>
<223> primer

<221> misc_feature
<222> 19, 20
<223> n = A,T,C or G

<400> 176
tatttctgct ctcaaagtnn scatgttcct ccgcatttc                              39

<210> 177
<211> 39
<212> DNA
<213> Artificial Sequence

<220>
<223> primer

<221> misc_feature
<222> 20, 21

<223> n = A,T,C or G

<400> 177
gaaatgcgga ggaacatgsn nactttgaga gcagaaata                                39

<210> 178
<211> 37
<212> DNA
<213> Artificial Sequence

<220>
<223> primer

<221> misc_feature
<222> 18, 19
<223> n = A,T,C or G

<400> 178
tctgctctca aagtacanns gttcctccgc atttcgg                                  37

<210> 179
<211> 37
<212> DNA
<213> Artificial Sequence

<220>
<223> primer

<221> misc_feature
<222> 19, 20
<223> n = A,T,C or G

<400> 179
ccgaaatgcg gaggaacsnn tgtactttga gagcaga                                  37

<210> 180
<211> 37
<212> DNA
<213> Artificial Sequence

<220>
<223> primer

<221> misc_feature
<222> 18, 19
<223> n = A,T,C or G

<400> 180
gctctcaaag tacacatnns cctccgcatt tcggtgg                                  37

<210> 181
<211> 37
<212> DNA
<213> Artificial Sequence

<220>
<223> primer

<221> misc_feature
<222> 19, 20
<223> n = A,T,C or G

<400> 181
ccaccgaaat gcggaggsnn atgtgtactt tgagagc                                37

<210> 182
<211> 37
<212> DNA
<213> Artificial Sequence

<220>
<223> primer

<221> misc_feature
<222> 18, 19
<223> n = A,T,C or G

<400> 182
ctcaaagtac acatgttnns ccgcatttcg gtggagg                                37

<210> 183
<211> 37
<212> DNA
<213> Artificial Sequence

<220>
<223> primer

<221> misc_feature
<222> 19, 20
<223> n = A,T,C or G

<400> 183
cctccaccga aatgcggsnn aacatgtgta ctttgag                                37

<210> 184
<211> 39
<212> DNA
<213> Artificial Sequence

<220>
<223> primer

<221> misc_feature
<222> 19, 20
<223> n = A,T,C or G

<400> 184
caaagtacac atgttcctnn scatttcggt ggaggcacc                              39

<210> 185
<211> 39
<212> DNA
<213> Artificial Sequence

<220>
<223> primer

<221> misc_feature
<222> 20, 21
<223> n = A,T,C or G

<400> 185
ggtgcctcca ccgaaatgsn naggaacatg tgtactttg                                39

<210> 186
<211> 39
<212> DNA
<213> Artificial Sequence

<220>
<223> primer

<221> misc_feature
<222> 19, 20
<223> n = A,T,C or G

<400> 186
agtacacatg ttcctccgnn sttcggtgga ggcaccaag                                39

<210> 187
<211> 39
<212> DNA
<213> Artificial Sequence

<220>
<223> primer

<221> misc_feature
<222> 20, 21
<223> n = A,T,C or G

<400> 187
cttggtgcct ccaccgaasn ncggaggaac atgtgtact                                39

<210> 188
<211> 37
<212> DNA
<213> Artificial Sequence

<220>
<223> primer

<400> 188
cggccatggc ccaggtgcag ctgcagcagt ctggggc                                  37

<210> 189
<211> 37
<212> DNA
<213> Artificial Sequence

<220>
<223> primer

<400> 189
ctggggcaga acttgtgaaa tcagggacct cagtcaa                                  37

<210> 190
<211> 37
<212> DNA
<213> Artificial Sequence

<220>

<223> primer

<400> 190
gggcagaact tgtgaggccg gggacctcag tcaagtt                                37

<210> 191
<211> 37
<212> DNA
<213> Artificial Sequence

<220>
<223> primer

<400> 191
aacttgtgag gtcagggggc tcagtcaagt tgtcctg                                37

<210> 192
<211> 37
<212> DNA
<213> Artificial Sequence

<220>
<223> primer

<400> 192
gcacagcttc tggcttcacc attaaagact cctatat                                37

<210> 193
<211> 37
<212> DNA
<213> Artificial Sequence

<220>
<223> primer

<400> 193
cagcttctgg cttcaacttt aaagactcct atatgca                                37

<210> 194
<211> 37
<212> DNA
<213> Artificial Sequence

<220>
<223> primer

<400> 194
cttctggctt caacattagc gactcctata tgcactg                                37

<210> 195
<211> 37
<212> DNA
<213> Artificial Sequence

<220>
<223> primer

<400> 195
actcctatat gcactgggtg aggcaggggc ctgaaca                                37

<210> 196

<211> 37
<212> DNA
<213> Artificial Sequence

<220>
<223> primer

<400> 196
tgcactggtt gaggcaggcg cctgaacagg gcctgga                                37

<210> 197
<211> 37
<212> DNA
<213> Artificial Sequence

<220>
<223> primer

<400> 197
ggttgaggca ggggcctggc cagggcctgg agtggat                                37

<210> 198
<211> 37
<212> DNA
<213> Artificial Sequence

<220>
<223> primer

<400> 198
ccccgaagtt ccagggccgt gccactttta ctacaga                                37

<210> 199
<211> 37
<212> DNA
<213> Artificial Sequence

<220>
<223> primer

<400> 199
cgaagttcca gggcaagttc acttttacta cagacac                                37

<210> 200
<211> 37
<212> DNA
<213> Artificial Sequence

<220>
<223> primer

<400> 200
tccagggcaa ggccactatt actacagaca catcctc                                37

<210> 201
<211> 37
<212> DNA
<213> Artificial Sequence

<220>
<223> primer

<400> 201
gcaaggccac ttttactcgc gacacatcct ccaacac                                37

<210> 202
<211> 37
<212> DNA
<213> Artificial Sequence

<220>
<223> primer

<400> 202
ttactacaga cacatccaaa aacacagcct acctgca                                37

<210> 203
<211> 37
<212> DNA
<213> Artificial Sequence

<220>
<223> primer

<400> 203
ctgccgtcta ttattgtgcg gaggggactc cgactgg                                37

<210> 204
<211> 37
<212> DNA
<213> Artificial Sequence

<220>
<223> primer

<400> 204
ccgtctatta ttgtaatcgc gggactccga ctgggcc                                37

<210> 205
<211> 37
<212> DNA
<213> Artificial Sequence

<220>
<223> primer

<400> 205
ctggcggtgg cggatcacag aatgtgctca cccagtc                                37

<210> 206
<211> 37
<212> DNA
<213> Artificial Sequence

<220>
<223> primer

<400> 206
gcggtggcgg atcagaaagc gtgctcaccc agtctcc                                37

<210> 207
<211> 38

<212> DNA
<213> Artificial Sequence

<220>
<223> primer

<400> 207
gaaaatgtgc tcacccagcc gccagcaatc atgtctgc                                38

<210> 208
<211> 37
<212> DNA
<213> Artificial Sequence

<220>
<223> primer

<400> 208
tgctcaccca gtctccaagc atcatgtctg catctcc                                 37

<210> 209
<211> 37
<212> DNA
<213> Artificial Sequence

<220>
<223> primer

<400> 209
   cccagtctcc agcaatcgtg tctgcatctc cagggga                                  37

<210> 210
<211> 37
<212> DNA
<213> Artificial Sequence

<220>
<223> primer

<400> 210
tgtctgcatc tccagggcag aaggtcacca taacctg                                 37

<210> 211
<211> 37
<212> DNA
<213> Artificial Sequence

<220>
<223> primer

<400> 211
ctgcatctcc aggggagacc gtcaccataa cctgcag                                 37

<210> 212
<211> 37
<212> DNA
<213> Artificial Sequence

<220>
<223> primer

<400> 212
taagttacat gcactggtac cagcagaagc caggcac                                37

<210> 213
<211> 37
<212> DNA
<213> Artificial Sequence

<220>
<223> primer

<400> 213
gcacttctcc caaactcgtg atttatagca catccaa                                37

<210> 214
<211> 37
<212> DNA
<213> Artificial Sequence

<220>
<223> primer

<400> 214
tggcttctgg agtccctgat cgcttcagtg gcagtgg                                37

<210> 215
<211> 37
<212> DNA
<213> Artificial Sequence

<220>
<223> primer

<400> 215
ctcgcttcag tggcagtaaa tctgggacct cttactc                                37

<210> 216
<211> 37
<212> DNA
<213> Artificial Sequence

<220>
<223> primer

<400> 216
gtggatctgg gacctctgcg tctctcacaa tcagccg                                37

<210> 217
<211> 37
<212> DNA
<213> Artificial Sequence

<220>
<223> primer

<400> 217
ctctcacaat cagccgactg gaggctgaag atgctgc                                37

<210> 218
<211> 37
<212> DNA

<213> Artificial Sequence

<220>
<223> primer

<400> 218
gaatggaggc tgaagatgaa gccacttatt actgcca                                37

<210> 219
<211> 37
<212> DNA
<213> Artificial Sequence

<220>
<223> primer

<400> 219
aggctgaaga tgctgccgat tattactgcc agcaaag                                37

<210> 220
<211> 37
<212> DNA
<213> Artificial Sequence

<220>
<223> primer

<400> 220
acccactcac gttcggtggc ggcaccaagc tggagct                                37

<210> 221
<211> 37
<212> DNA
<213> Artificial Sequence

<220>
<223> primer

<400> 221
cttctggctt caacattsat gactcctata tgcactg                                37

<210> 222
<211> 37
<212> DNA
<213> Artificial Sequence

<220>
<223> primer

<400> 222
ctggcttcaa cattaaasat tcctatatgc actgggt                                37

<210> 223
<211> 37
<212> DNA
<213> Artificial Sequence

<220>
<223> primer

<400> 223

gcttcaacat taaagacsat tatatgcact gggtgag                                37

<210> 224
<211> 37
<212> DNA
<213> Artificial Sequence

<220>
<223> primer

<400> 224
tcaacattaa agactccsat atgcactggg tgaggca                                37

<210> 225
<211> 37
<212> DNA
<213> Artificial Sequence

<220>
<223> primer

<400> 225
ttaaagactc ctatatgsat tgggtgaggc aggggcc                                37

<210> 226
<211> 37
<212> DNA
<213> Artificial Sequence

<220>
<223> primer

<400> 226
gcctggagtg gattggasat attgatcctg agaatgg                                37

<210> 227
<211> 37
<212> DNA
<213> Artificial Sequence

<220>
<223> primer

<400> 227
agtggattgg atggattsat cctgagaatg gtgatac                                37

<210> 228
<211> 37
<212> DNA
<213> Artificial Sequence

<220>
<223> primer

<400> 228
ttggatggat tgatcctsat aatggtgata ctgaata                                37

<210> 229
<211> 37
<212> DNA
<213> Artificial Sequence

<220>
<223> primer

<400> 229
gatggattga tcctgagsat ggtgatactg aatatgc 37

<210> 230
<211> 37
<212> DNA
<213> Artificial Sequence

<220>
<223> primer

<400> 230
ttgatcctga gaatggtsat actgaatatg ccccgaa 37

<210> 231
<211> 37
<212> DNA
<213> Artificial Sequence

<220>
<223> primer

<400> 231
atcctgagaa tggtgatsat gaatatgccc cgaagtt 37

<210> 232
<211> 37
<212> DNA
<213> Artificial Sequence

<220>
<223> primer

<400> 232
ctgagaatgg tgatactsat tatgccccga agttcca 37

<210> 233
<211> 37
<212> DNA
<213> Artificial Sequence

<220>
<223> primer

<400> 233
gtgatactga atatgccsat aagttccagg gcaaggc 37

<210> 234
<211> 37
<212> DNA
<213> Artificial Sequence

<220>
<223> primer

<400> 234
atactgaata tgccccgsat ttccagggca aggccac 37

<210> 235
<211> 37
<212> DNA
<213> Artificial Sequence

<220>
<223> primer

<400> 235
aatatgcccc gaagttcsat ggcaaggcca cttttac                                37

<210> 236
<211> 37
<212> DNA
<213> Artificial Sequence

<220>
<223> primer

<400> 236
ccgtctatta ttgtaatsat gggactccga ctgggcc                                37

<210> 237
<211> 37
<212> DNA
<213> Artificial Sequence

<220>
<223> primer

<400> 237
tctattattg taatgagsat actccgactg ggccgta                                37

<210> 238
<211> 37
<212> DNA
<213> Artificial Sequence

<220>
<223> primer

<400> 238
attattgtaa tgaggggsat ccgactgggc cgtacta                                37

<210> 239
<211> 37
<212> DNA
<213> Artificial Sequence

<220>
<223> primer

<400> 239
attgtaatga ggggactsat actgggccgt actactt                                37

<210> 240
<211> 37
<212> DNA
<213> Artificial Sequence

<220>
<223> primer

<400> 240
gtaatgaggg gactccgsat gggccgtact actttga                                37

<210> 241
<211> 37
<212> DNA
<213> Artificial Sequence

<220>
<223> primer

<400> 241
atgaggggac tccgactsat ccgtactact ttgacta                                37

<210> 242
<211> 37
<212> DNA
<213> Artificial Sequence

<220>
<223> primer

<400> 242
aggggactcc gactgggsat tactactttg actactg                                37

<210> 243
<211> 37
<212> DNA
<213> Artificial Sequence

<220>
<223> primer

<400> 243
ctccgactgg gccgtacsat tttgactact ggggcca                                37

<210> 244
<211> 37
<212> DNA
<213> Artificial Sequence

<220>
<223> primer

<400> 244
taacctgcag tgccagcsat agtgtaagtt acatgca                                37

<210> 245
<211> 37
<212> DNA
<213> Artificial Sequence

<220>
<223> primer

<400> 245
cctgcagtgc cagctcasat gtaagttaca tgcactg                                37

<210> 246
<211> 37
<212> DNA
<213> Artificial Sequence

<220>
<223> primer

<400> 246
gcagtgccag ctcaagtsat agttacatgc actggtt                                37

<210> 247
<211> 37
<212> DNA
<213> Artificial Sequence

<220>
<223> primer

<400> 247
gtgccagctc aagtgtasat tacatgcact ggttcca                                37

<210> 248
<211> 37
<212> DNA
<213> Artificial Sequence

<220>
<223> primer

<400> 248
ccagctcaag tgtaagtsat atgcactggt tccagca                                37

<210> 249
<211> 37
<212> DNA
<213> Artificial Sequence

<220>
<223> primer

<400> 249
ctcccaaact cgtgattsat agcacatcca acctggc                                37

<210> 250
<211> 37
<212> DNA
<213> Artificial Sequence

<220>
<223> primer

<400> 250
ccaaactcgt gatttatsat acatccaacc tggcttc                                37

<210> 251
<211> 37
<212> DNA
<213> Artificial Sequence

<220>

<223> primer

<400> 251
aactcgtgat ttatagcsat tccaacctgg cttctgg                                37

<210> 252
<211> 37
<212> DNA
<213> Artificial Sequence

<220>
<223> primer

<400> 252
tcgtgattta tagcacasat aacctggctt ctggagt                                37

<210> 253
<211> 37
<212> DNA
<213> Artificial Sequence

<220>
<223> primer

<400> 253
tgatttatag cacatccsat ctggcttctg gagtccc                                37

<210> 254
<211> 37
<212> DNA
<213> Artificial Sequence

<220>
<223> primer

<400> 254
atagcacatc caacctgsat tctggagtcc ctgctcg                                37

<210> 255
<211> 37
<212> DNA
<213> Artificial Sequence

<220>
<223> primer

<400> 255
gcacatccaa cctggctsat ggagtccctg ctcgctt                                37

<210> 256
<211> 37
<212> DNA
<213> Artificial Sequence

<220>
<223> primer

<400> 256
cttattactg ccagcaasat tctagttacc cactcac                                37

<210> 257

<211> 36
<212> DNA
<213> Artificial Sequence

<220>
<223> primer

<400> 257
attactgcca gcaaagasat agttacccac tcacgt                                36

<210> 258
<211> 36
<212> DNA
<213> Artificial Sequence

<220>
<223> primer

<400> 258
actgccagca aagatctsat tacccactca cgttcg                                36

<210> 259
<211> 36
<212> DNA
<213> Artificial Sequence

<220>
<223> primer

<400> 259
gccagcaaag atctagtsat ccactcacgt tcggtg                                36

<210> 260
<211> 37
<212> DNA
<213> Artificial Sequence

<220>
<223> primer

<400> 260
aaagatctag ttacccasat acgttcggtg ctggcac                               37

<210> 261
<211> 17
<212> DNA
<213> Artificial Sequence

<220>
<223> primer

<400> 261
caggaaacag ctatgac                                                     17

<210> 262
<211> 22
<212> DNA
<213> Artificial Sequence

<220>
<223> primer

<400> 262
ggaccacggt caccgtctcc tc                                                22